# EUROPEAN PATENT SPECIFICATION

(11) **EP 4 103 160 B1**
(45) Date of publication and mention of the grant of the patent: **26.06.2024**
(21) Application number: 21706503.6
(22) Date of filing: 14.02.2021
(51) Int. Cl.: A61K 9/51, A61K 47/34, A61K 47/42, C07K 14/00

(54) **NANOPARTICLES ENCAPSULATING SMALL MOLECULES**
KLEINE MOLEKÜLE EINKAPSELNDE NANOPARTIKEL
NANOPARTICULES D'ENCAPSULATION DE PETITES MOLÉCULES

(30) Priority: 13.02.2020 EP 20157250
(43) Date of publication of application: 21.12.2022
(73) Proprietor: ETH Zurich, 8092 Zurich (CH)
(72) Inventor: HILVERT, Donald, 8006 Zürich (CH); EDWARDSON, Thomas, 8048 Zürich (CH)
(74) Representative: Latscha Schöllhorn Partner AG
(86) International application number: PCT/EP2021/053571
(87) International publication number: WO 2021/160873

(56) References cited:
- WO-A1-2015/054639
- WO-A1-2016/138525
- US-A1- 2018 318 218
- AZUMA YUSUKE ET AL: "Tailoring lumazine synthase assemblies for bionanotechnology", CHEMICAL SOCIETY REVIEWS,, vol. 47, no. 10, 21 May 2018 (2018-05-21), pages 3543-3557, XP002786639,
- SETH LILAVIVAT ET AL: "In Vivo Encapsulation of Nucleic Acids Using an Engineered Nonviral Protein Capsid", JOURNAL OF THE AMERICAM SOCIETY, vol. 134, no. 32, 15 August 2012 (2012-08-15), pages 13152-13155, XP055525136, US ISSN: 0002-7863, DOI: 10.1021/ja302743g
- EDWARDSON THOMAS G W ET AL: "Rational Engineering of a Designed Protein Cage for siRNA Delivery", JOURNAL OF THE AMERICAM SOCIETY, AMERICAN CHEMICAL SOCIETY, US, vol. 140, no. 33, 22 August 2018 (2018-08-22), pages 10439-10442, XP002786641, ISSN: 0002-7863 cited in the application

## Description

The present invention relates to the field of nanoparticles. Especially the invention relates to a lipoprotein cage comprising a protein cage and a surfactant composition for intracellular delivery of cargo.

### RELATED ART

Well-defined nanoparticles can be well suited for protective transport of molecules within biological systems.

Protein cages have recently emerged as an important platform for nanotechnology development. Of the naturally existing protein cages, viruses are among the most efficient nanomachines, achieving component replication and efficient self-assembly in complex biological milieu. An artificial system that can carry out the most basic steps of viral particle assembly in vivo has been designed based on patchwork cages formed from Aquifex aeolicus lumazine synthase and a circularly permuted variant with appended cationic peptides. These two-component protein containers self-assemble in vivo, capturing endogenous RNA molecules in a size-selective manner (Azuma et al., Modular Protein Cages for Size-Selective RNA Packaging in Vivo, J. Am. Chem. Soc. 2018, vol. 140, 566-569)

In a further approach, nanoparticulate phospholipid bilayer disks were assembled from phospholipid and a class of amphipathic helical proteins using a synthetic gene. The self-assembly process begins with a mixture of the phospholipid and protein in the presence of a detergent. Upon removal of detergent, particles form containing either saturated or unsaturated phospholipid (Bayburt et al., Self-Assembly of Discoidal Phospholipid Bilayer Nanoparticles with Membrane Scaffold Proteins Nano Letters 2002, vol. 2(8), pp. 853-85)

The advent of de novo-designed protein cages offers an alternative strategy for creating cargo transport vehicles. A designed nonfunctional protein cage was transformed into a nucleic acid delivery vehicle, which can encapsulate oligonucleotides in vitro with high binding affinity (Edwardson et al., Rational Engineering of a Designed Protein Cage for siRNA Delivery. J. Am. Chem. Soc. 2018, vol. 140, 10439-10442).

However, proteinaceous compartments are limited in the types of cargo that they carry. Incorporation of molecules other than proteins and nucleic acids requires developing an additional set of self-assembly rules. Development of protein cages by incorporation of other molecular species has the potential to expand applicability into new areas, one of which is the transport of small molecules as possible when using the present invention.

### SUMMARY OF THE INVENTION

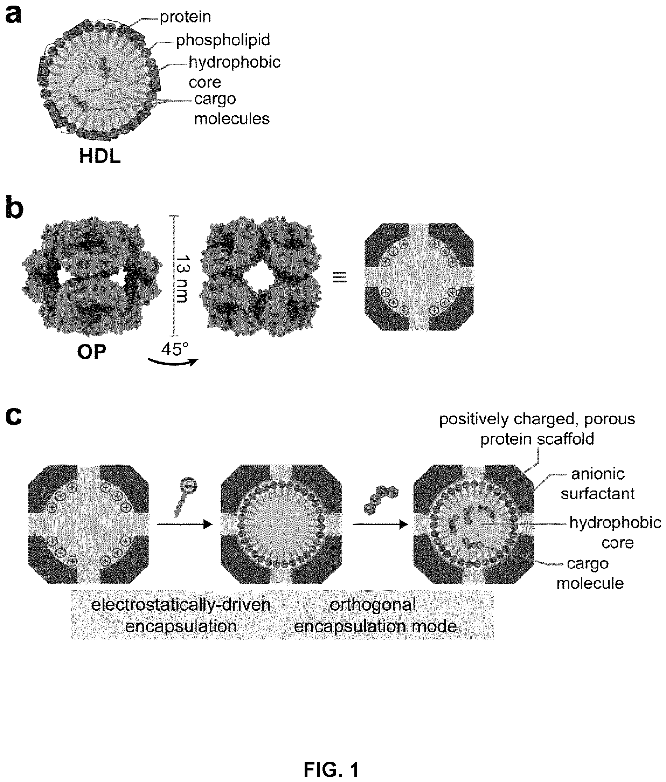

The present invention provides porous lipoprotein cages (also called herein lipoprotein scaffolds) capable of encapsulating poorly water-soluble molecule cargo. In the lipoprotein cages of the invention, hydrophobic compartmentalization is achieved by combining the engineered proteins that stabilize the cage and act as biorecognizable barcodes for the cargo to be delivered by the cage via the specific pores with amphiphiles that create a hydrophobic interior of the cage (Fig. 1a).

In a two-tier host-guest approach, a designed protein cage with a highly positively charged lumen is used to nucleate anionic surfactant molecules into micellar aggregates within its interior cavity at concentrations well below their critical aggregation concentration. Electrostatic attraction drives the encapsulation of anionic surfactants, which phase separate due to the high effective concentration to form micellar aggregates within the protein cage. The non-polar core of this stable protein-surfactant complex can then sequester small molecules through the hydrophobic effect.

The protein cage is highly stable and acts as a template to form the lipidic/micellar core within its inner cavity, meaning a previous formulation step is not required. Therefore, the amphiphiles do not need to form a stable particle on their own before addition of the protein.

Through their unique architecture, the resulting lipoprotein cages of the invention can recruit and sequester small, preferably small hydrophobic molecules through the protein-scaffolded hydrophobic core. The inventors showed that these lipoprotein cages are stable, monodisperse and protect their cargo from sequestration by serum proteins, thus enhancing the cellular uptake of poorly soluble fluorescent probes and cytotoxic drugs. These findings demonstrate the beneficial combination of electrostatically-driven and amphiphilic self-assembly within stable protein compartments using a protein and surfactant molecule.

Due to the generality of the hydrophobic effect, this system could be used to encapsulate all manner of small, preferably hydrophobic molecules, including hormones, hydrophobic peptides, luminescent metal complexes, therapeutic agents, and vitamins etc..

Thus, in a first aspect, the present invention provides for a lipoprotein cage for intracellular delivery of cargo, said lipoprotein cage comprises:
(i) a protein cage comprising at least one polypeptide comprising an amino acid sequence I consisting of: 
MX13QAIGILELX1SIAAGMELGDAMLKSAX14VX15LLVSKTISX2GKFLLML
GGDIX8AIX9X12AIX10TGTX11QAGX3LLVDSLVLAX16IHPSVLPAIX17GX18N
X19VX20X7X21QAVGIVETX4SVAACISAADX22AVX23GSX24VTLVRVHMAX5
GIGGKCYMVVAGDVSDVALAVTVASSSAGAYGX6LVYASLIPX25PHX26A
MWX27QMVX28GX29E (SEQ ID NO: 1),
   wherein any of X₁ to X₂₉ are independently of each other an amino acid, provided that at least 3 of X₁ to X₆ are independently of each other a positively charged amino acid, and wherein optionally up to 5 amino acids in positions other than denoted by X₁ to X₂₉ in SEQ ID NO: 1 are exchanged by any amino acid;
   wherein said protein cage possesses a positively charged interior; and
(ii) a surfactant composition comprising one or more amphiphiles, wherein the one or more amphiphiles are selected such that the net charge of the composition is negative; the one or more amphiphiles comprise a hydrophilic group and a hydrophobic group, wherein the hydrophobic group comprises at least one hydrocarbon moiety selected from the group consisting of C4-C30 alkyl, C4-C30 alkenyl, C4-C30 alkynyl, C4-C30 alkoxy or C5-C30 cycloalkyl;
   wherein said surfactant composition is encapsulated into the assembled protein cage.

In a further aspect, the invention relates to a complex comprising the lipoprotein cage of the invention and one or more cargo molecules.

In a further aspect, the invention relates to a method for manufacturing the lipoprotein cage of the invention comprising the steps of self-assembling a protein cage from at least one polypeptide comprising the amino acid sequence I, preferably from 24 polypeptides each comprising the amino acid sequence I, and encapsulating the surfactant composition of the invention into the protein cage, without disassembly of the protein cage.

In a further aspect, the invention provides a method for manufacturing the complex of the invention comprising the step of mixing the lipoprotein cage of the invention with one or more cargo molecules, wherein said cargo is encapsulated into the lipoprotein cage of the invention without disassembly of the lipoprotein cage.

In a further aspect, the invention provides a method for treating cells with the complex of the invention comprising the step of contacting said cell with the complex of the invention.

### DESCRIPTION OF FIGURES

**Figure 1**: Self-assembly of lipoprotein-mimetic cages, (a) Cartoon depiction of a high density lipoprotein (HDL) particle, showing the charged phospholipids, proteins and hydrophobic cargo molecules. (b) Surface representations of the OP protein cage viewed along the 2-fold (left) and 4-fold (middle) symmetry axes and cartoon cut away of positively charged interior (right), (c) Cartoon depiction of lipoprotein particle with positively charged porous protein scaffold, encapsulated anionic surfactants, driven by electrostatic attraction, which form a non-polar core on the stable protein-surfactant complex that can then sequester non-polar small molecules through the hydrophobic effect.

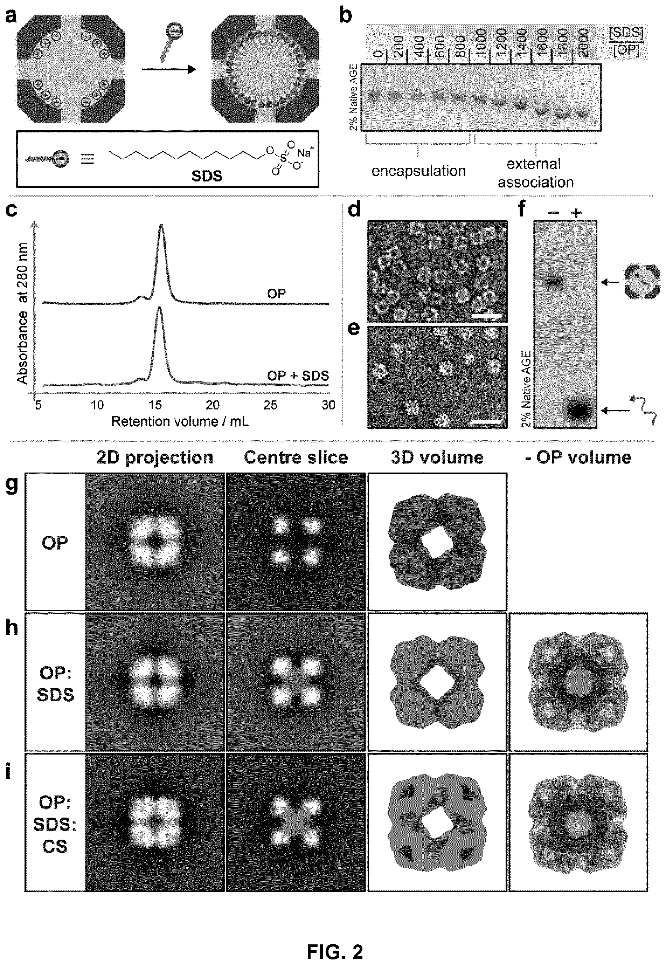

**Figure 2**: Capsid-templated micelle formation, (a) Cartoon depiction of protein cage-templated micellization of sodium dodecyl sulfate (SDS) molecules. (b) Native gel electrophoresis of the OP cage in the presence of increasing molar equivalents of SDS. (c) Size-exclusion chromatography of the OP cage before and after incubation with 800 equivalents of SDS. (d) Transmission electron micrograph of empty OP cages and (e) OP cages in the presence of SDS (800 equiv.), scale bars are 30 nm. (f) Encapsulation of Atto488-labelled ssDNA visualized by native gel electrophoresis. Without pre-incubation with SDS (-) the OP cage internalizes the ssDNA probe quantitatively. After internalization of the SDS molecules (+) the OP cage can no longer encapsulate the oligonucleotide probe. (g) 2-D projection, central slice, 3-D reconstructions and subtracted volumes, determined by cryo-EM, for empty OP capsid and (h) OP capsid with SDS, and (i) OP capsid with SDS:CS. Additional density from the surfactants can be seen in the center slice and in the density obtained by subtracting empty OP from OP:SDS (shown as solid surface, right column).

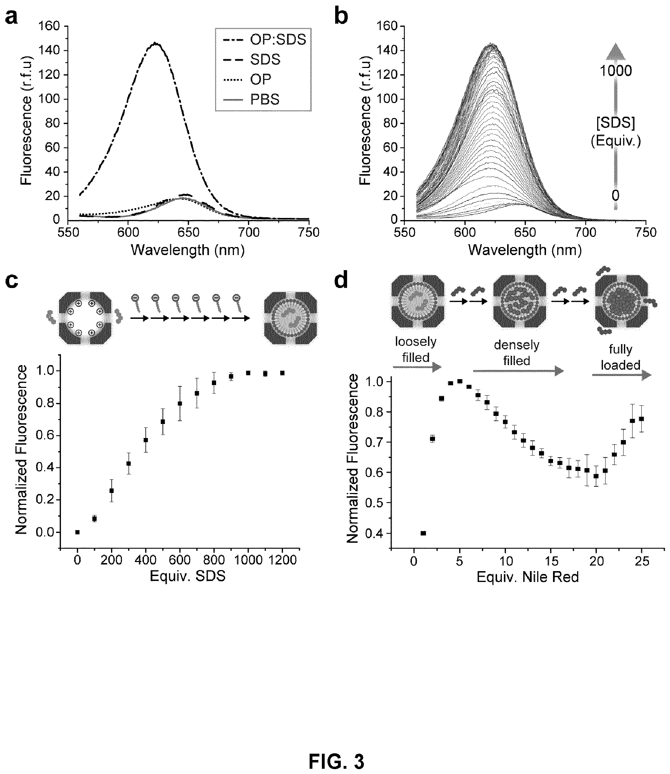

**Figure 3**: Hydrophobic core formation, cargo capacity and kinetics. (a) Steady state fluorescence emission spectra of Nile Red in the presence of OP, SDS and the OP: SDS complex, with reference to PBS buffer, (b) Steady state fluorescence emission spectra of Nile Red in the presence of OP with increasing molar equivalents of SDS. (c) Normalized fluorescence emission of Nile Red at 620 nm vs. molar equivalent of SDS with respect to OP cages, (d) Normalized fluorescence emission at 620 nm vs. molar equivalent of Nile Red added to OP:SDS complexes.

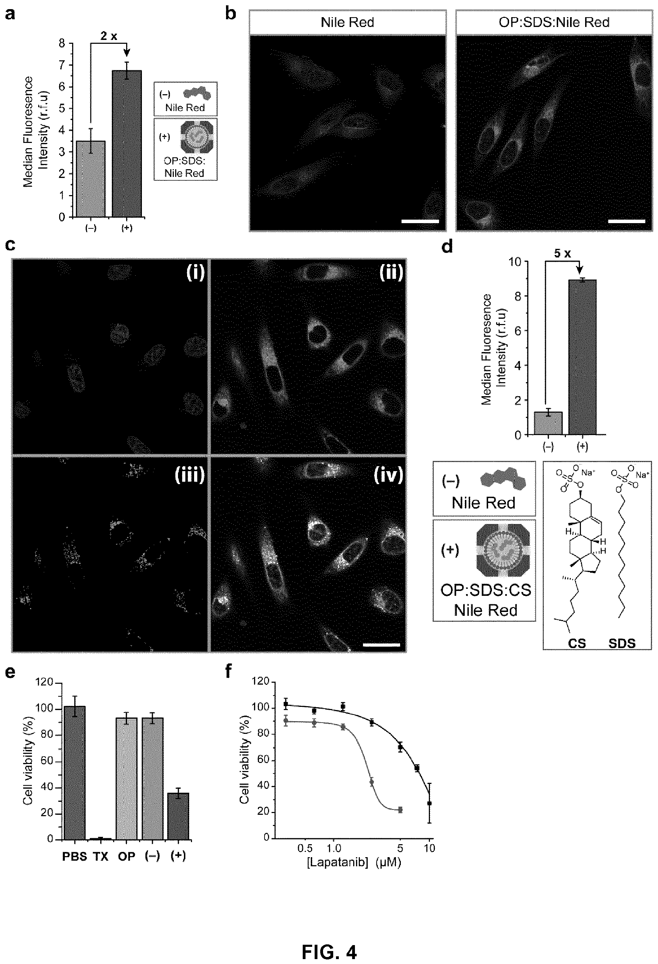

**Figure 4**: Measurement of biological activity, sodium dodecyl sulfate (SDS), cholesterol sulfate (CS). (a) Flow cytometry dataset for HeLa cells treated with either free Nile Red, or Nile Red packaged in OP:SDS complexes (right panel), with 5 Nile Red molecules per capsid (n=3). (b) Confocal fluorescence microscopy of HeLa cells treated with Nile Red as free molecules (left panel) or packaged in OP: SDS complexes (right panel). Hoechst 33342 (round nucleus shape), Nile Red (cytosol surrounding nucleus), scale bars are 30 µm. (c) Confocal fluorescence microscopy of HeLa cells treated with OPS38C:SDS:CS:Nile Red complexes (1:600:200:5) where OP-S38C cages are labelled with Atto425. Hoechst 33342 (round nucleus shape, i), Nile Red (ii), Atto425 (OP-S38C; iii), overlay of Hoechst 33342, Nile Red and Atto425 (iv), scale bar is 30 µm. (d) Flow cytometry comparison of HeLa cells treated with Nile Red (-) or Nile Red packaged in OP:SDS:CS complexes (+), where the ratio of OP:SDS:CS:Nile Red is 1:600:200:5. (e) Viability of HeLa cells after treatment with 2.5 µM of free lapatinib (-) or lapatinib encapsulated in OP:SDS:CS complexes (+). Control samples are PBS buffer, Triton X-100 (TX) and OP:SDS:CS complexes without encapsulated drug (OP). (f) Dose-response comparison of cell viability after treatment with free lapatinib (black), or lapatinib encapsulated in OP:SDS:CS complexes (grey).

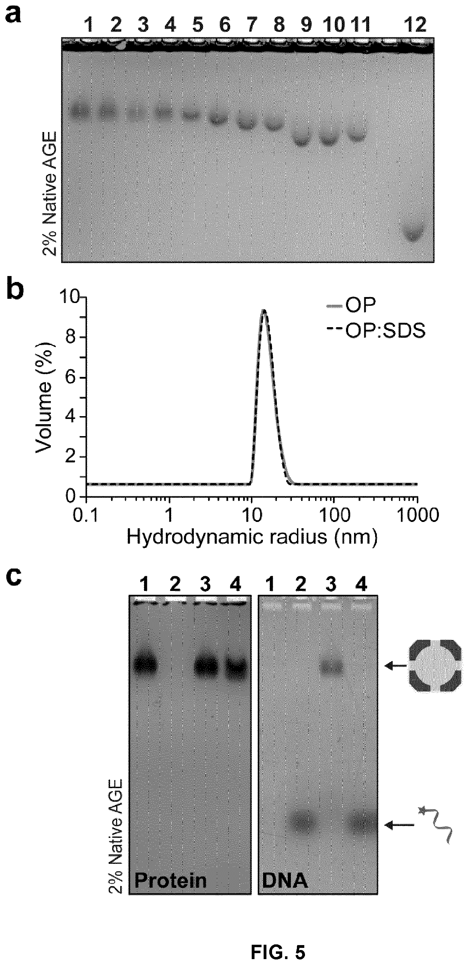

**Figure 5**: Additional characterization of OP:SDS complexes. (a) Duplicate of the native gel shown in Figure 2b, Lanes 1-11: increments of 200 equivalents of SDS added to OP cages, covering the range from 0 to 2000; Lane 12: fully denatured protein, which was obtained by treatment with 65mM SDS, equating to 130,000 equivalents. (b) Dynamic light scattering of empty OP cages and OP:SDS complexes shows negligible change in external particle diameter nor aggregation in solution. (c) Native agarose gel stained with coomassie blue for protein (left) and visualized by Atto488 fluorescence for labelled ssDNA probe (right). Lane 1 - OP; Lane 2 - Atto488-labelled DNA; Lane 2 - DNA + OP, Lane 3 - DNA + pre-formed OP:SDS complexes. While empty OP cages can internalize the DNA quantitatively, the SDS-filled cages cannot encapsulate the DNA probe.

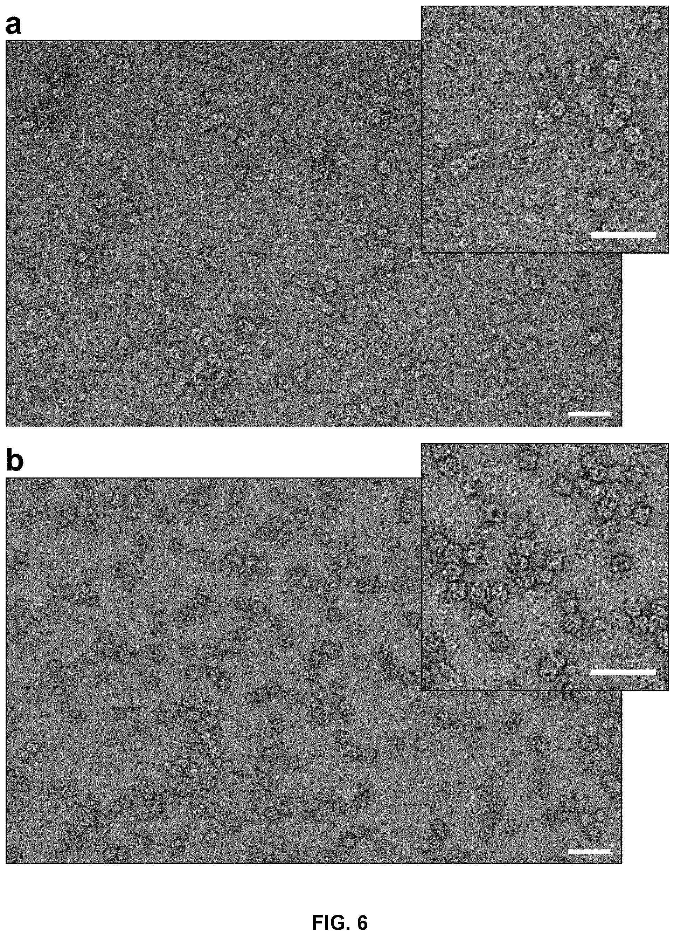

**Figure 6**: Additional TEM images of OP and OP:SDS complexes. Negatively stained transmission electron micrographs of (a) OP and (b) OP:SDS. Scale bars are 50 nm.

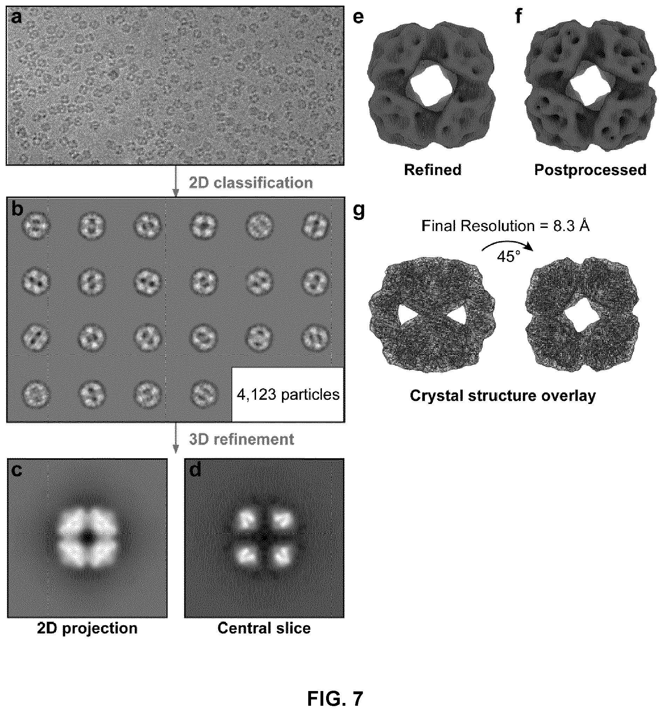

**Figure 7**: Cryo-EM of empty OP capsids. (a) Representative cryo-electron micrograph of OP particles in vitreous ice. (b) The best classes from three consecutive rounds of 2D classification (22 classes, 4,123 particles). These particles were refined into a 3D model with imposed octahedral symmetry. The reconstruction is shown as a projection (c), a slice through the center (d), a 3D representation (e), and after postprocessing (f). The 3D reconstruction (g) overlaps well with the reported crystal structure of OP (PDB-ID: 6FDB).

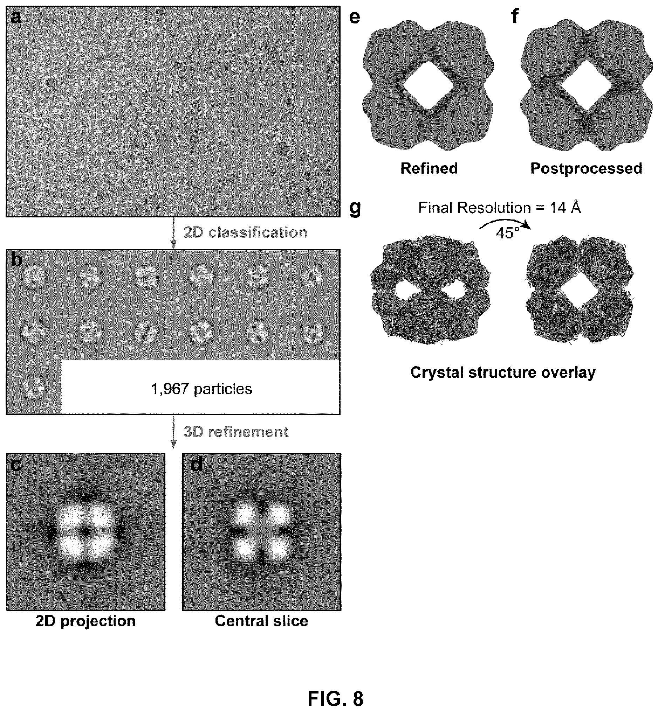

**Figure 8**: Cryo-EM of OP:SDS complexes. (a) Representative cryo-electron micrograph of OP:SDS particles in vitreous ice. (b) The best classes from three consecutive rounds of 2D classification (13 classes, 1,967 particles). These particles were refined into a 3D model with imposed octahedral symmetry. The reconstruction is shown as a projection (c), a slice through the center (d), a 3D representation (e), and after postprocessing (f). The 3D reconstruction (g) overlaps well with the reported crystal structure of OP (PDB-ID: 6FDB).

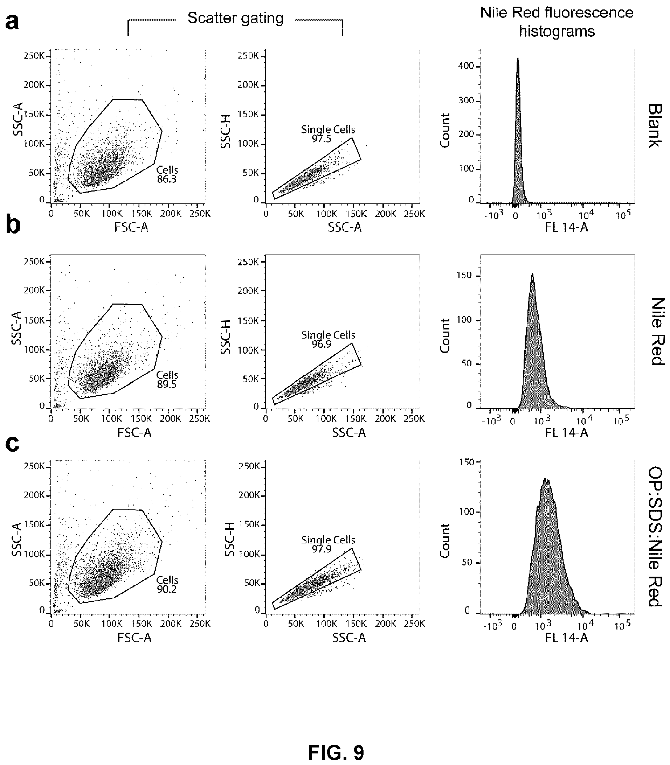

**Figure 9**: Representative flow cytometry dataset. All sample gating and resulting histograms of Nile Red fluorescence for the treatment of HeLa cells with (a) PBS buffer (blank), (b) Nile Red (500 nM), and (c) OP:SDS:Nile Red (1:800:5, at 500 nM Nile Red). Population gating based on the PBS control reveals that while 58% of cells are Nile Red positive when treated with the free dye, treatment with OP:SDS:Nile Red increases this fraction of cells to 90%. A minimum of 5,000 cells were analyzed for each sample replicate.

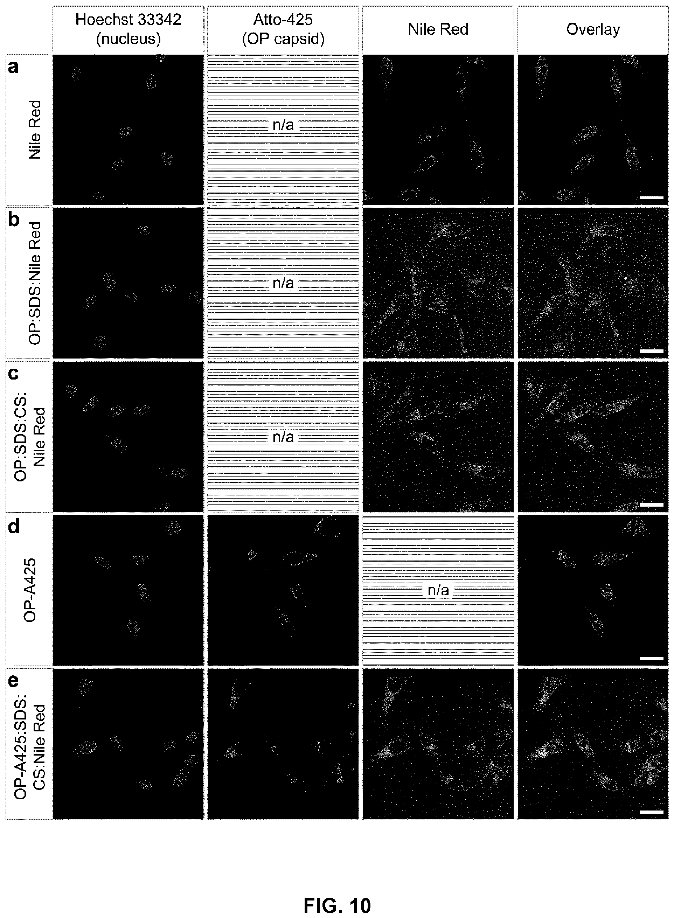

**Figure 10**: Additional confocal fluorescence micrographs. In each case cells were exposed to 500 nM Nile Red. For the surfactant-filled OP cages the molar ratios are OP:SDS:NR = 1:800:5, OP:SDS:CS:NR = 1:600:200:5, and OP-S38C-A425:SDS:CS:NR = 1:600:200:5. Atto425-labeled OP-S38C capsids have an average of 1.9 dyes per capsid. All scale bars are 30 µm. n/a, not applicable.

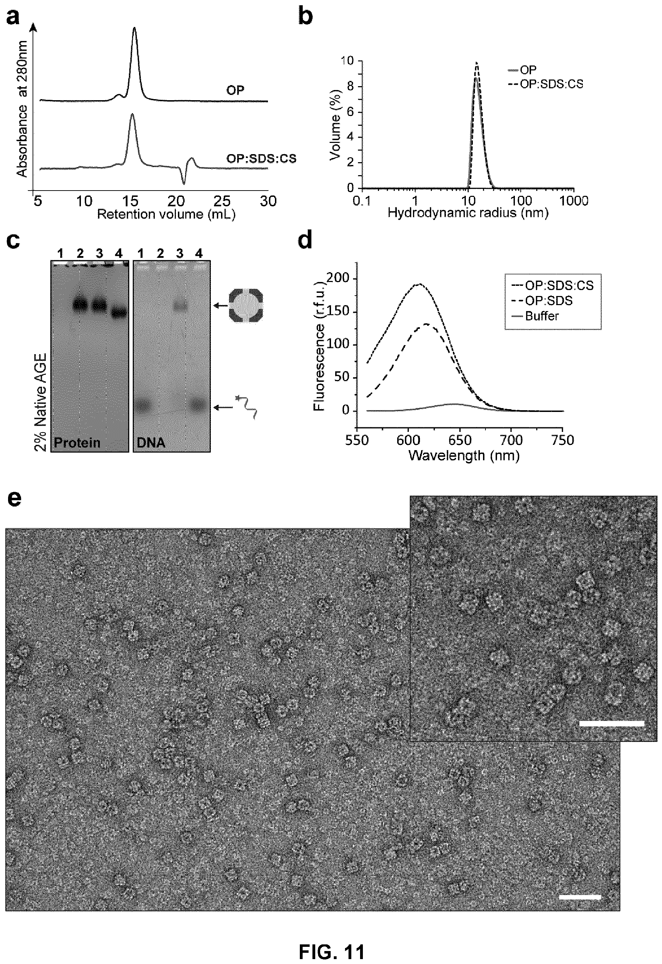

**Figure 11**: Characterization of OP:SDS:CS complexes. (a) Size-exclusion chromatograms of OP and OP:SDS:CS complexes. The negative and positive peaks at 21 mL are due to the elution of DMSO, which is not present in the running buffer, (b) Dynamic light scattering of empty OP cages and OP:SDS:CS complexes shows negligible change in external particle diameter nor aggregation in solution. (c) Native agarose gel stained with coomassie blue for protein (left) and visualized by Atto488 fluorescence for labelled ssDNA probe (right). Lane 1 - Atto488-labelled DNA only; Lane 2 - OP; Lane 3 - DNA + OP, Lane 4 - DNA + pre-formed OP:SDS:CS complexes. While empty OP cages can internalize the DNA quantitatively, the SDS:CS-filled cages cannot encapsulate the DNA probe. (d) Fluorescence spectra of Nile Red in buffer, and in the presence of OP:SDS cages and OP:SDS:CS cages. The increase in fluorescence and blueshift of the Nile Red emission maximum indicate that CS is incorporated into the protein-scaffolded micelle complex. (e) Negatively stained transmission electron microscopy of OP:SDS:CS complexes. Scale bars are 50 nm.

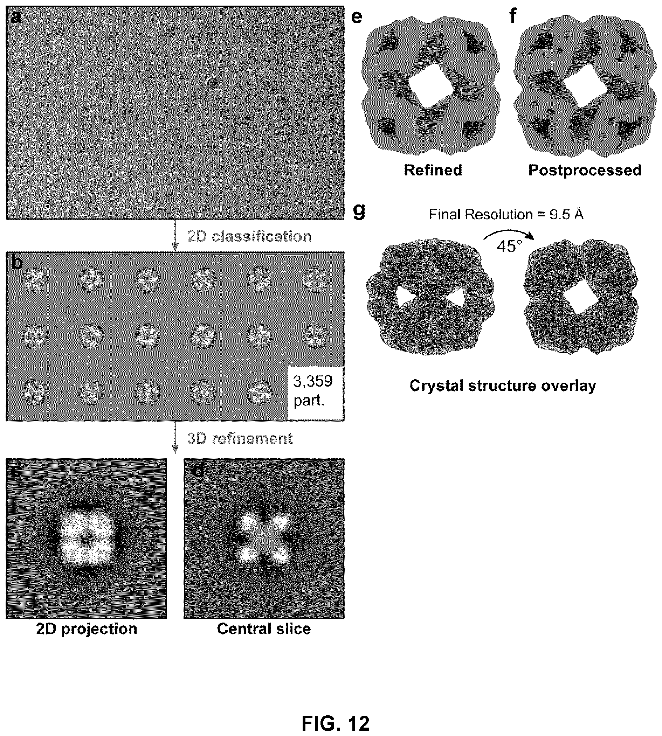

**Figure 12**: Cryo-EM of OP:SDS:CS complexes. (a) Representative cryo-electron micrograph of OP:SDS:CS particles in vitreous ice. (b) The best classes from three consecutive rounds of 2D classification (17 classes, 3,359 particles). These particles were refined into a 3D model with imposed octahedral symmetry. The reconstruction is shown as a projection (c), a slice through the center (d), a 3D representation (e), and after postprocessing (f). The 3D reconstruction (g) overlaps well with the reported crystal structure of OP (PDB-ID: 6FDB).

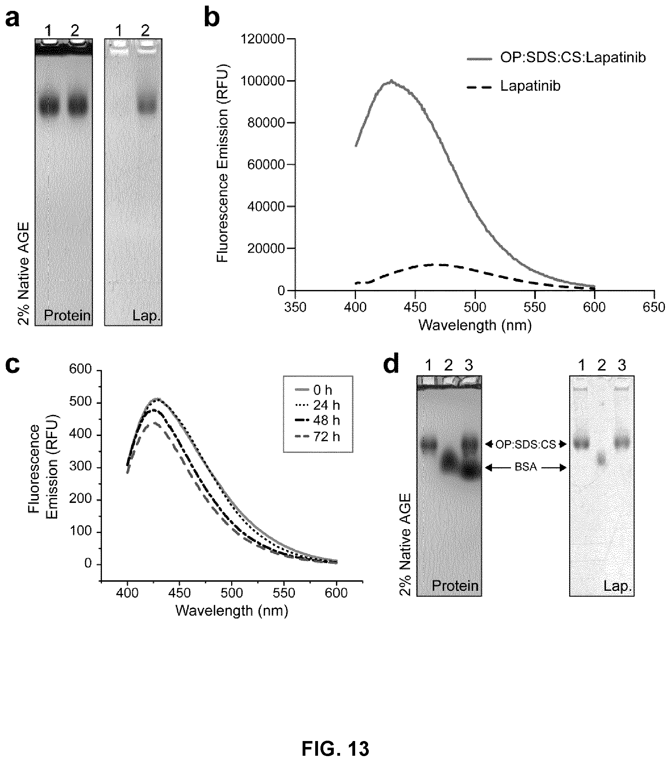

**Figure 13**: Lapatinib loading, (a) Native agarose gel of OP:SDS:CS complexes visualized with coomassie blue for protein (left) and by lapatinib fluorescence (right). Lane 1 - OP:SDS:CS (1:600:200); Lane 2 - OP:SDS:CS:lapatinib (1:600:200:10). (b) In the presence of OP:SDS:CS complexes lapatinib fluorescence increases significantly and the emission maximum is shifts to lower wavelengths, consistent with encapsulation in a non-polar environment. (c) Fluorescence spectra of lapatinib-loaded OP:SDS:CS complexes that have been dialyzed against bovine serum albumin (BSA) containing medium for 24, 48 or 72 hours compared to the undialyzed control. (d) Native agarose gel of OP:SDS:CS complexes and BSA visualized with coomassie blue for protein (left) and by lapatinib fluorescence (right). Lane 1 - OP:SDS:CS:lapatinib (1:600:200:10); Lane 2-BSA:lapatinib (1:1); Lane 3 - OP:SDS:CS:lapatinib + BSA. These data reveal that lapatinib is stably encapsulated by OP:SDS:CS complexes after incubation with equimolar concentrations of BSA, corroborating the results from the fluorescence experiment shown in (c).

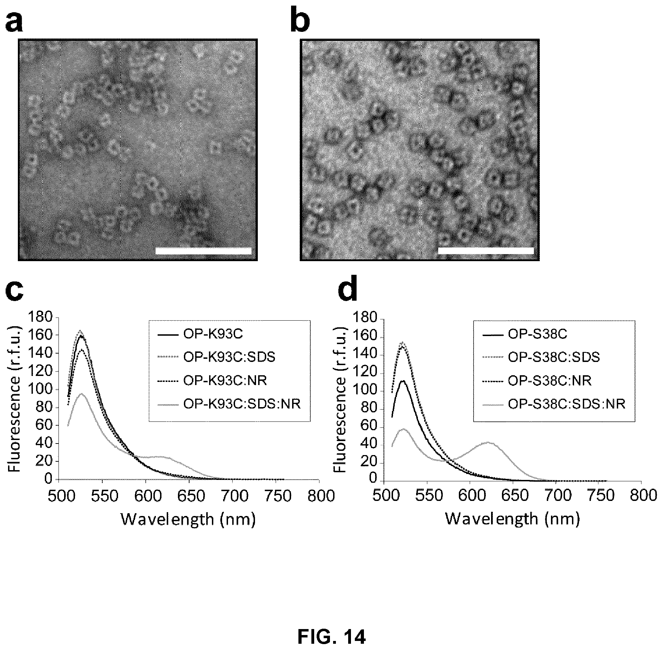

**Figure 14**: Formation of protein cage-micelle complexes in two chemically-modified protein cage variants, (a) Transmission electron micrograph of OP-K93C and (b) OP-S38C protein cages, scale bars are 100 nm. (c) Steady state fluorescence emission spectra of protein conjugated Atto495 and Nile Red in the presence of OP-K93C protein cages with and without SDS in PBS. In each case, the sample is excited at 490 nm, only in the presence of SDS is Förster resonance energy transfer (FRET) observed between the Atto495 and Nile Red fluorophores, consistent with internalization in protein-micelle complexes. (d) Steady state fluorescence emission spectra of protein conjugated Atto495 and Nile Red in the presence of OP-S38C protein cages with and without SDS in PBS. As the S38C mutation is on the lumenal surface of the protein cage, internalization of SDS causes a change in fluorescence of the Atto495 fluorophore conjugated at that position. Additionally, stronger FRET is observed between Atto495 and Nile Red within OPS38C:SDS complexes than in OP-K93C:SDS, consistent with the closer distance of the S38C position to the micelle core, compared to the exterior surface displayed K93C position. In each measurement, 800 equivalents of SDS and 4 equivalents of Nile Red were used, with respect to OP capsids (120 nM).

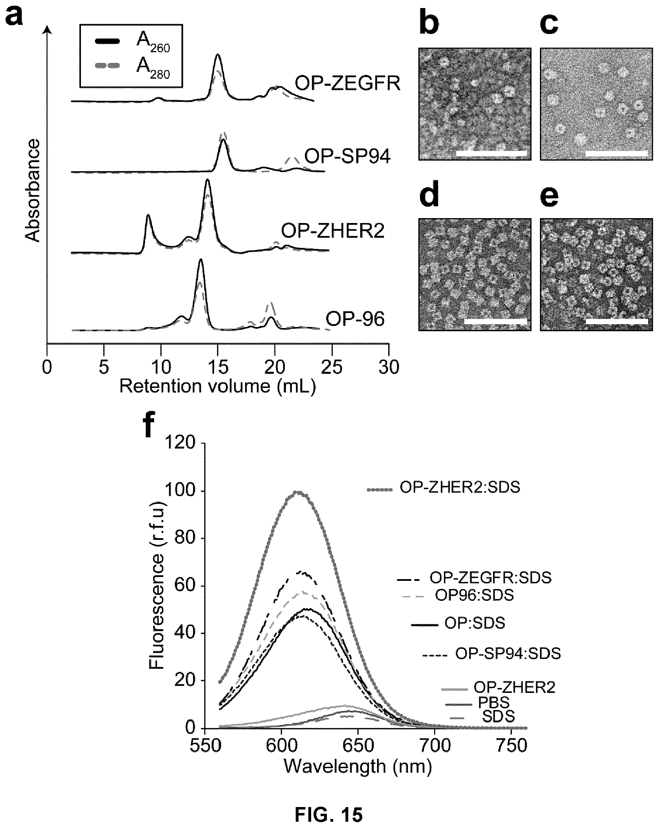

**Figure 15**: Formation of protein cage-micelle complexes with C-terminal peptide tags. (a) Size-exclusion chromatogram of protein cages with C-terminal modifications, the maj or peaks around 15 mL correspond to 24-mer cages with size difference consistent with the length of the peptide appendage. Additional minor peaks are impurities that are removed during this purification step. Transmission electron micrographs of (b) OP-ZEGFR, (c) OP-SP94, (d) OP-ZHER2 and (e) OP-96 protein cages, scale bars are 100 nm. (f) Steady-state fluorescence spectra of Nile Red in the presence of protein-micelle complexes at a 1:800:5 molar ratio of capsid:SDS:Nile Red, at a concentration of 120 nM capsid. All protein variants causes an increase in Nile Red fluorescence and a blueshift of the emission maximum, consistent with formation of protein-micelle complexes and encapsulation of Nile Red within the hydrophobic core. The original OP:SDS complex is also included for comparison. Control samples of PBS buffer, SDS alone and OP-ZHER2 capsids alone show negligible fluorescence increase.

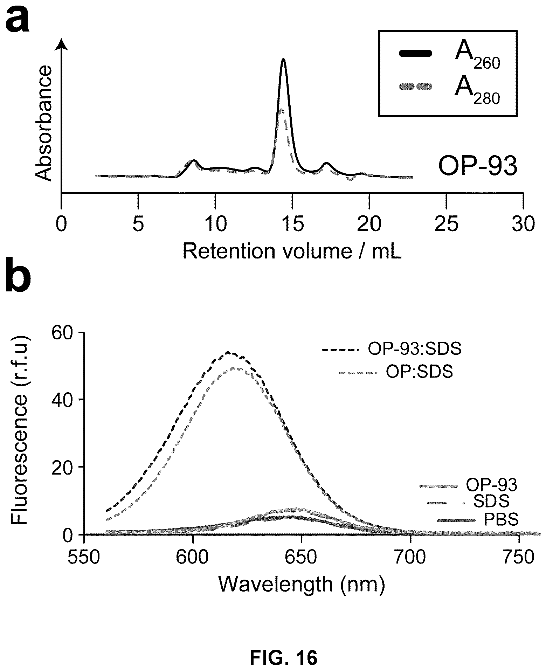

**Figure 16**: Formation of protein cage-micelle complexes with an N-terminally-modified protein. (a) Size-exclusion chromatogram of the protein cage formed by OP-93, the major peak at 14 mL is consistent with the formation of a 24-mer cage structure similar in size to OP. Additional minor peaks are impurities that are removed during this purification step. (b) Steady-state fluorescence spectra of Nile Red in the presence of protein-micelle complexes at a 1:800:5 molar ratio of capsid:SDS:Nile Red, at a concentration of 120 nM capsid. The OP-93 protein variant causes an increase in Nile Red fluorescence and a blueshift of the emission maximum, consistent with formation of protein-micelle complexes and encapsulation of Nile Red within the hydrophobic core. The original OP:SDS complex is also included for comparison. Control samples of PBS buffer, SDS alone and OP-93 capsids alone show negligible fluorescence increase.

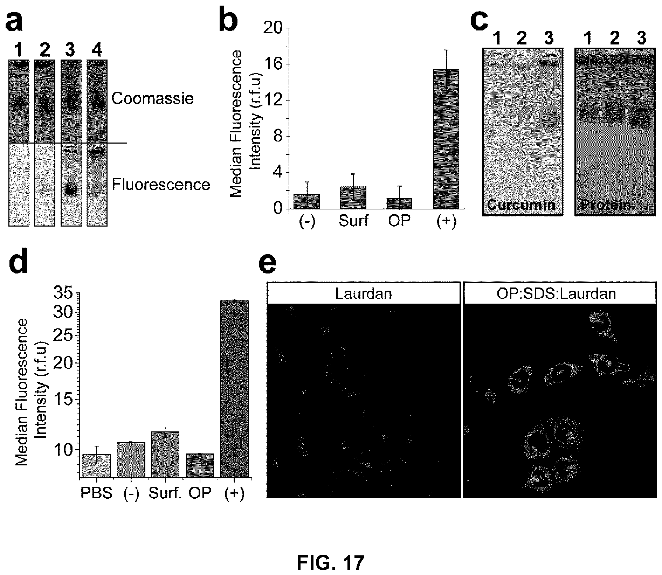

**Figure 17**: Altering surfactant composition and small molecule cargo, (a) Native agarose gel electrophoresis showing encapsulation of three different drug molecules in protein-micelle complexes using a surfactant mixture of SDS:SDBS (1:1), with a total of 800 equivalents of surfactant per capsid. Lane 1 - OP, Lane 2 - OP:SDS:SDBS:curcumin, Lane 3 - OP:SDS:SDBS:lapatinib, Lane 4 - OP:SDS:SDBS:daunorubicin. The upper and lower panels are the same gel lanes visualized by UV transillumination or stained with coomassie blue for protein visualization. (b) Flow cytometry dataset for HeLa cells treated with either free curcumin (-), or curcumin packaged in OP:SDS:SDBS complexes (+). Surf and OP refer to cells treated with the same concentration of curcumin in the presence of the surfactant mixture or empty OP cages, respectively. The ratio of OP:SDS:SDBS:curcumin is 1:400:400:8. Error bars show the standard deviation of triplicate measurements. (c) Native agarose gel electrophoresis showing increasing
concentrations (0.7, 1.4 and 2.1 uM) of OP:SDS:CS:curcumin complexes with 23 equiv. of curcumin per capsid. (d) Flow cytometry dataset for HeLa cells treated with either free curcumin (-), or curcumin packaged in OP:SDS:CS complexes (+). PBS refers to untreated cells and Surf and OP refer to cells treated with the same concentration of curcumin in the presence of the surfactant mixture or empty OP cages, respectively. Eight equivalents of curcumin were used per cage and error bars show the standard deviation of triplicate measurements. (e) Confocal microscopy of HeLa cells treated with free laurdan (left) or laurdan packaged in OP: Surf complexes (right), frames are 184 µm².

### DETAILED DESCRIPTION OF THE INVENTION

Unless defined otherwise, all technical and scientific terms used herein have the same meanings as commonly understood by one of ordinary skill in the art to which this invention belongs.

Throughout this specification and the claims which follow, unless the context requires otherwise, the word "comprise" or the word "include", and variations such as "comprises/includes" and "comprising/including", are to be understood to imply the inclusion of an element, stated integer, step or a group thereof but not the exclusion of any other element, stated integer, step or a group thereof.

As used in this specification and the appended claims, the singular forms "a", "an", and "the" include plural referents, unless the content clearly dictates otherwise.

The term "about" or "approximately" when used in connection with a numerical value is meant to encompass numerical values within a range having a lower limit that is 0-10% smaller than the indicated numerical value and having an upper limit that is 0-10% larger than the indicated numerical value. The term "about" or "approximately" means preferably ±10%, more preferably ±5%, again more preferably ±3% or most preferably ±0% (referring to the given numeric value, respectively). In each of the invention embodiments, "about" can be deleted. All ranges of values disclosed herein should refer to and include any and all values falling within said range including the values defining the range.

In a first aspect, the invention relates to a lipoprotein cage for intracellular delivery of cargo, said lipoprotein cage comprises:
(i) a protein cage comprising at least one polypeptide comprising an amino acid sequence I consisting of: MX13QAIGILELX1SIAAGMELGDAMLKSAX14VX15LLVSKTISX2GKFLLML 
GGDIX8AIX9X12AIX10TGTX11QAGX3LLVDSLVLAX16IHPSVLPAIX17GX18N
X19VX20X7X21QAVGIVETX4SVAACISAADX22AVX23GSX24VTLVRVHMAX5
GIGGKCYMVVAGDVSDVALAVTVASSSAGAYGX6LVYASLIPX25PHX26A
MWX27QMVX28GX29E (SEQ ID NO: 1),
   wherein any of X₁ to X₂₉ are independently of each other an amino acid, provided that at least 3 of X₁ to X₆ are independently of each other a positively charged amino acid, and wherein optionally up to 5 amino acids in positions other than denoted by X₁ to X₂₉ in SEQ ID NO: 1 are exchanged by any amino acid;
   wherein said protein cage possesses a positively charged interior;
(ii) a surfactant composition comprising one or more amphiphiles, wherein the one or more amphiphiles are selected such that the net charge of the composition is negative;
the one or more amphiphiles comprise a hydrophilic group and a hydrophobic group, and the hydrophobic group comprises at least one hydrocarbon moiety selected from the group consisting of C4-C30 alkyl, C4-C30 alkenyl, C4-C30 alkynyl, C4-C30 alkoxy or C5-C30 cycloalkyl, wherein said surfactant composition is encapsulated into the assembled protein cage.

As used herein, the terms protein cage and lipoprotein cage relate to cage-like nanoparticles. The protein cage and lipoprotein cage of the invention are preferably in the nanometer size range (i.e. from about 1 nm to about 1000 nanometers). In a further preferred embodiment, said protein or lipoprotein cage has an external diameter up to about 50 nm. In a very preferred embodiment, said lipoprotein cage has an external diameter of about 13 nm.

Said polypeptide of the invention is selected such that it is capable of forming a protein cage by self-assembly of said at least one polypeptide of the invention. In a preferred embodiment, the protein cage of the invention comprises exactly 24 polypeptides of the invention (i.e. said at least one polypeptide is defined as exactly 24 polypeptides of the invention). In another preferred embodiment, the protein cage of the invention has an octahedral geometry (octahedral point group symmetry). In a preferred embodiment of the invention, the protein cage has a quaternary structure of multiple subunits, preferably of 8 subunits, each subunit comprises 3 polypeptides of the invention.

In a preferred embodiment said protein cage comprises (i) an exterior protein scaffold and (ii) a central cavity (also mentioned herein as cavity, internal cavity, interior or lumen). Preferably, said exterior scaffold surrounds, i.e. is assembled around said central cavity.

The term "negatively charged" or "positively charged" as used herein includes and preferably refers to a molecule that has a negatively or positively charged group. The term "anion" as used herein refers to negatively charged ions. More preferably, said anion or negatively charged molecule has a negatively charged group at neutral or physiological pH.

The protein cage of the invention has a positively charged cavity. In a preferred embodiment, the protein cage is positively charged on the cavity surface. Said positive charges stem from multiple positively charged amino acids, preferably arginines or lysines. Thus, the protein cage of the invention has a very strong affinity to encapsulate negatively charged molecules. In a preferred embodiment, said cavity of the protein cage has a diameter from about 6.5 nm to about 8 nm, preferably of about 8 nm.

In a preferred embodiment, said protein cage has a porous structure, i.e. said protein cage includes pores. Preferably, the exterior scaffold of the protein cage includes pores that are connected to the cavity of said protein cage. A pore is defined herein as an opening or gap in the protein cage or in the exterior scaffold of the protein cage.

In a preferred embodiment, said protein or lipoprotein cage comprises six pores. Preferably, said exterior scaffold includes six pores, which are connected to the cavity of the protein or lipoprotein cage. In another preferred embodiment, said pores have a diameter from about 3 nm to about 4 nm. In another preferred embodiment, said protein or lipoprotein cage includes 6 pores having a diameter of 3-4 nm

In another further preferred embodiment, the protein cage of the invention includes 6 pores having a diameter of about 3-4 nm, the internal cavity of said protein cage has a diameter of about 8 nm, and the external diameter of said protein cage is about 13 nm.

Loading (or encapsulation) and unloading (or release) of cargo into the lipoprotein or of the surfactant composition into the protein cage works via the pores of the protein cage and the surfactant composition affects the loading and unloading of cargo. The terms loading or encapsulation relates to any uptake of cargo, composition or amphiphile into the lipoprotein or protein cage. The term unloading (or release) relates to liberation or displacement/replacement of the cargo, either in part or completely, preferably completely.

Said surfactant composition of the invention is encapsulated into the assembled protein cage, i.e. without disassembly of the protein cage. In a preferred embodiment, the assembled protein cage of the invention is loadable with the surfactant composition, without disassembly of the protein cage. In a preferred embodiment of the invention, electrostatic attraction drives the encapsulation of the surfactant composition. Preferably in the lipoprotein cage of the invention, said surfactant composition phase separates due to its high effective concentration within the lipoprotein cage. Preferably said surfactant composition is capable of forming a micellar aggregate within the protein cage.

More preferably said surfactant composition according to the invention creates a hydrophobic core within the protein cage. In a preferred embodiment, the lipoprotein cage of the invention is loadable and unloadable with cargo without disassembly of the lipoprotein cage. Further preferably, said lipoprotein cage of the invention, especially said non-polar core of said lipoprotein cage of the invention is capable of sequestering cargo molecules loaded into the lipoprotein cage of the invention. Preferably, said cargo is encapsulated within the lipoprotein cage by non-covalent interactions. Said non-covalent interactions are preferably hydrophobic interactions (hydrophobic effect). In a preferred embodiment, the lipoprotein cage of the invention is capable of delivering cargo intracellularly. Preferably, said cargo is delivered intracellularly, without disassembly of the lipoprotein cage. In a preferred embodiment, the lipoprotein cage of the invention is capable of encapsulating cargo, without disassembly of the lipoprotein cage, and to release said encapsulated cargo, preferably intracellularly, without disassembly of the lipoprotein cage. This is possible due to the porous structure of the lipoprotein cage. In a further preferred embodiment, the lipoprotein cage of the invention is loadable with cargo extracellularly and unloadable from said cargo intracellularly, without disassembly of the lipoprotein cage.

In a preferred embodiment, the lipoprotein cage of the invention is capable of encapsulating cargo extracellularly, to enter cells with said encapsulated cargo and to release said encapsulated cargo into the cells, without disassembly of the lipoprotein cage. In a preferred embodiment, the lipoprotein cage of the invention is capable of encapsulating cargo, to enter cells with said encapsulated cargo, and to release said encapsulated cargo into the cells, more preferably into the cytoplasm of a cell, each step without disassembly of the lipoprotein cage. In another preferred embodiment, the lipoprotein cage of the invention is capable of encapsulating cargo, to enter cells with said encapsulated cargo, and to release encapsulated hydrophobic cargo into the cells, wherein said released cargo escapes to the cytoplasm of the cell, each step without disassembly of the lipoprotein cage.

Preferably, said cargo is hydrophobic cargo, more preferably non-polar cargo.

Preferably, said cargo is small cargo. Small cargo has preferably a size of 1000 Da or below. In a preferred embodiment, a size of 1000 Da or below means that said cargo has a size of 1000 Da or lower, preferably 800 Da or lower, more preferably 600 Da or lower, again more preferably 500 Da or lower, again more preferably 400 Da or lower, again more preferably 300 Da or lower, again more preferably 200 Da or lower, again more preferably 100 Da or lower.

In another embodiment, said cargo is small hydrophobic cargo having a size of 1000 Da or below, again more preferably small non-polar cargo having a size of 1000 Da or below.

Preferably, said cargo has a low solubility in aqueous media. Preferably, said small cargo having a size of 1000 Da or below and has a low solubility in aqueous media. More preferably said cargo of low solubility is included in Class II or Class IV of the Biopharmaceutics Classification System (BCS). Again more preferably, said low solubility cargo has a lower solubility than a highly soluble cargo for which the highest strength dose is soluble in 250 mL or less of aqueous media over the pH range of 1.0-7.5, more preferably over the pH range of 1.0-6.8, at 37 ± 1°C. Preferred methods for determining solubility are the USP Dissolution Apparatus, shake-flask method or acid or base titration methods.

Preferably said cargo is an active agent, preferably a therapeutic or diagnostic agent. More preferably said cargo is selected from the group consisting of a chemotherapeutic agent, such doxorubicin or paclitaxel, an antifungal agent, such as bifonazole or amphotericin B, an antiviral agent such as indinavir or ritonavir, and an antibiotic.

The surfactant composition of the lipoprotein comprises one or more amphiphiles selected such that the net charge of the composition is negative. The net charge of a composition is the overall charge contributed by all compounds included in the composition. Preferably, the net charge of the composition is negative at a physiological pH.

Based on the positive charge of the protein cage assembled according to the examples and the number of amphiphiles encapsulated therein, the inventors found that at least 20 mol% of the compounds of the surfactant composition having at least one negative charge is a value sufficient for cargo encapsulation, especially for small cargo molecules that have a size of 1000 Da or below, or hydrophobic cargo molecules, or poorly low water soluble cargo molecules included in Class II or Class IV of BCS.

Thus, in a further preferred embodiment, at least 20 mol% of the compounds included in the surfactant composition have at least one negative charge. In a further preferred embodiment, at least 20 mol% of the amphiphiles included in the surfactant composition have at least one negative charge. In case the compound has more than one negative charge, i.e. an amount of N negative charges, said value of 20 mol% can be divide by N.

In a further preferred embodiment, at least 20 mol%, preferably at least 30 mol%, more preferably at least 40 mol%, again more preferably at least 50 mol%, again more preferably at least 60 mol%, again more preferably at least 70 mol%, again more preferably at least 80 mol%, again more preferably at least 90 mol%, again more preferably at least 100 mol%, of the compounds or amphiphiles included in the surfactant composition have at least one negative charge.

Amphiphile or amphiphilic compounds (herein also called surfactants) are defined herein as organic compounds that comprise at least one hydrophobic group and at least one hydrophilic group. In a preferred embodiment, said amphiphile is a diblock compound comprising a hydrophilic "head" group, and a hydrophobic "tail" region. In a preferred embodiment, the amphiphile consists of at least one hydrophobic and at least one hydrophilic group. In a preferred embodiment, said amphiphile is a diblock compound comprising, preferably consisting of a hydrophilic "head" group, and a hydrophobic "tail" region.

The hydrophobic group comprises at least one hydrocarbon moiety. In a preferred embodiment, the hydrophobic group consists of at least one hydrocarbon moiety. The term "hydrocarbon moiety", as used herein, encompasses compounds that consist only of hydrogen and carbon, joined by covalent bonds. The term encompasses open chain (aliphatic) hydrocarbons, including straight (unbranched) chain and branched hydrocarbons, and saturated as well as mono- and polyunsaturated hydrocarbons. The term also encompasses hydrocarbons containing one or more cyclic or aromatic ring.

The at least one hydrocarbon moiety is selected from the group consisting of linear or branched C4-C30 alkyl, C4-C30 alkenyl, C4-C30 alkynyl, C4-C30 alkoxy and C5-C30 cycloalkyl. In a preferred embodiment, said hydrocarbon moiety is selected from the group consisting of linear C4-C30 alkyl, C4-C30 alkenyl, C4-C30 alkynyl, C4-C30 alkoxy and C5-C30 cycloalkyl. In a preferred embodiment, said hydrocarbon moiety is selected from the group consisting of linear or branched alkyl, alkenyl, alkynyl, alkoxy, or cycloalkyl of C8 to C20, preferably C10 to 18, more preferably C12 to C18, most preferably C12, C14, C16, C17 or C18. In a preferred embodiment, said hydrocarbon moiety is selected from the group consisting of linear alkyl, linear alkenyl or cycloalkyl of C8 to C20, preferably C10 to 18, more preferably C12 to C18, most preferably C12, C14, C16, C17 or C18. In a preferred embodiment, said hydrocarbon moiety further comprises an aryl moiety in addition to the alkyl, alkenyl, alkynyl, alkoxy and cycloalkyl moiety.

In a preferred embodiment, said hydrocarbon moiety is selected from the group consisting of branched or linear alkyl, branched or linear alkenyl or cycloalkyl of C5-C30, preferably C8 to C20, more preferably C10 to 18, even more preferably C12 to C18, most preferably C12, C14, C16, C17 or C18. In a preferred embodiment, said anionic moiety is a sulfate moiety, and said hydrocarbon moiety is a linear alkyl, linear alkyl-ether or cycloalkyl residue, wherein the linear alkyl, linear alkyl-ether or cycloalkyl residue is C5-C30, preferably C8 to C20, more preferably C10 to 18, even more preferably C12 to C18, most preferably C12, C14, C16, C17 or C18.

The term "alkyl" or "alkyl residue", as used herein, refers to a straight or branched hydrocarbon chain radical consisting solely of carbon and hydrogen atoms, containing no unsaturation, having from 4-30 carbon atoms (e.g., C4-C30 alkyl), and which may be or typically is attached to the rest of the molecule by a single bond. Whenever it appears herein, a numerical range such as "4-30" refers to each integer in the given range. For example, "C4-C30" means that the alkyl group may consist of 4 carbon atom, 5 carbon atoms, 6 carbon atoms, etc., up to and including 30 carbon atoms, although the definition is also intended to cover the occurrence of the term "alkyl" where no numerical range is specifically designated. Typical alkyl groups include alkylether, methyl, ethyl, n-propyl, 1-methylethyl (interchangeably used with iso-propyl; interchangeably abbreviated herein as iPr or Pri), n-butyl, isobutyl, sec-butyl, isobutyl, tertiary butyl (interchangeably used with 1,1-dimethylethyl or tert-butyl), n-pentyl, isopentyl, neopentyl, hexyl, septyl, octyl, nonyl and decyl. Unless stated otherwise specifically in the specification, an alkyl group is optionally substituted by one or more of substituents which are independently alkenyl, alkoxy, carboxylic group (-COOH), heteroalkyl, heteroalkenyl, hydroxyl, phosphate group (-OP(O)(OH)O-), phosphonate group (-OP(O)O-), phenyl group (-C₆H₄) optionally substituted with a halogen, preferably iodine, or a carboxylic group. Preferably, the term "alkyl", as used herein, refers to an unsubstituted alkyl as defined herein.

The term "alkenyl" or "alkenyl residue", as used herein, refers to a straight or branched hydrocarbon chain radical group consisting solely of carbon and hydrogen atoms, containing at least one double bond, and having from 4 to 30 carbon atoms (i.e., C4-C30 alkenyl), which may be or typically is attached to the rest of the molecule by a single bond. Whenever it appears herein, a numerical range such as "4-30" refers to each integer in the given range - e.g., "C4-C30" means that the alkenyl group may consist of 4 carbon atoms, 5 carbon atoms, etc., up to and including 30 carbon atoms. Typical alkenyl groups include ethenyl (i.e., vinyl), prop-1-enyl (i.e., allyl), but-1-enyl, pent-1-enyl and penta-1,4-dienyl, alkenylether. Each double bond can be of either the (E)- or (Z)-configuration. Alkenyl, thus, may include, if applicable, either each of said double bond in its (E)-configuration, in its (Z)-configuration and mixtures thereof in any ratio. Unless stated otherwise specifically in the specification, an alkenyl group is optionally substituted by one or more of substituents which are independently alkenyl, alkoxy, carboxylic group (-COOH), heteroalkyl, heteroalkenyl, hydroxyl, phosphate group (-OP(O)(OH)O-), phosphonate group (-OP(O)O-), phenyl group (-C₆H₄) optionally substituted with a halogen, preferably iodine, or a carboxylic group. Preferably, the term "alkenyl", as used herein, refers to an unsubstituted alkenyl as defined herein.

The term "alkynyl" or "alkynyl residue", as used herein, refers to a straight or branched hydrocarbon chain radical group consisting solely of carbon and hydrogen atoms, containing at least one triple bond, having from two to ten carbon atoms (i.e., C4-C30 alkynyl). Whenever it appears herein, a numerical range such as "4-30" refers to each integer in the given range - e.g., "C4-C30" means that the alkynyl group may consist of 4 carbon atoms, 5 carbon atoms, etc., up to and including 30 carbon atoms. Typical alkynyl groups include ethynyl, propynyl, butynyl, pentynyl and hexynyl. Unless stated otherwise specifically in the specification, an alkynyl group is optionally substituted by one or more of substituents which are independently alkenyl, carboxylic group (-COOH), heteroalkyl, heteroalkenyl, phosphate group (-OP(O)(OH)O-), phosphonate group (-OP(O)O-), phenyl group (-C₆H₄) optionally substituted with a halogen, preferably iodine, or a carboxylic group. Preferably, the term "alkynyl", as used herein, refers to an unsubstituted alkynyl as defined herein.

The term "alkoxy" or "alkoxy residue", as used herein, refers to the group -O-alkyl, including from 4 to 30 carbon atoms of a straight, branched configuration and combinations thereof attached to the parent structure through an oxygen. Examples include methoxy, ethoxy, propoxy, isopropoxy, cyclopropyloxy and cyclohexyloxy. The term "alkoxy" includes substituted alkoxy which refers to alkoxy wherein the alkyl constituent is substituted (i.e., -O-(substituted alkyl)). Unless stated otherwise specifically in the specification, the alkyl moiety of an alkoxy group is optionally substituted by one or more of substituents which are independently alkenyl, carboxylic group (-COOH), heteroalkyl, heteroalkenyl, phosphate group (-OP(O)(OH)O-), phosphonate group (-OP(O)O-), phenyl group (-C6H4) optionally substituted with a halogen, preferably iodine, or a carboxylic group.

The term "aryl" or "aryl residue", as used herein, refers to an aromatic radical with six to ten ring atoms (e.g., C6-C10 aromatic or C6-C10 aryl) which has at least one ring having a conjugated pi electron system which is carbocyclic (e.g., phenyl, fluorenyl, and naphthyl). Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by adding "-idene" to the name of the corresponding univalent radical, e.g., a naphthyl group with two points of attachment is termed naphthylidene. The term includes monocyclic or fused-ring polycyclic (i.e., rings which share adjacent pairs of ring atoms) groups.

The term "cycloalkyl" or "cycloalkyl residue", as used herein, refers to a monocyclic or polycyclic radical that contains only carbon and hydrogen, and may be saturated, or partially unsaturated. Cycloalkyl groups include groups having from 5 to 30 ring carbon atoms (i.e. C5-30 cycloalkyl). Whenever it appears herein, a numerical range such as "5 to 30" refers to each integer in the given range, e.g., "C5-30 cycloalkyl" means that the cycloalkyl group may consist of 5 carbon atoms, etc., up to and including 30 carbon atoms. Illustrative examples of cycloalkyl groups include the following moieties: cyclopropyl, cyclobutyl, cyclopentyl, cyclopentenyl, cyclohexyl, cyclohexenyl, cycloheptyl, cyclooctyl, cyclononyl, cyclodecyl, norbornyl, and the like. The term "cycloalkyl" also relates to a monocyclic or polycyclic radical that contains further hydrocarbon moieties, such as linear or branched alkyl, alkenyl, alkynyl, alkoxy or aryl. The most preferred cycloalkyl is a saturated C17 polycyclic cycloalkyl.

The term "amphiphile" as used herein comprises in a preferred embodiment amphiphilic compounds selected from the group consisting of phospholipids, sphingolipids, glycerolipids, saccharolipids, fatty acids, fatty acid esters, steroids, sterols, steroid esters, polyketides, amphiphilic block copolymers, peptides, amphiphiles comprising peptides or oligonucleotides, peptide nucleic acids, carboxylates, sulfates, sulfonates, boronates, phosphonates, and phosphates.

In a preferred embodiment, the surfactant composition comprises a mixture of different amphiphiles.

In a preferred embodiment, the hydrophilic group of said amphiphile is an anionic hydrophilic group, i.e. comprising at least one anion. Preferably, said anionic hydrophilic group is such that said amphiphile has a negative molecular net charge (herein mentioned as anionic amphiphile).

In a preferred embodiment, the surfactant composition comprises at least one anionic amphiphile, i.e. an amphiphile having a negative molecular net charge. In another preferred embodiment, the surfactant composition comprises at least one anionic amphiphile having a negative molecular net charge and a cationic, uncharged and/or zwitterionic amphiphile. In a preferred embodiment, all amphiphiles included in the surfactant composition are anionic amphiphiles.

In another preferred embodiment, said surfactant composition comprises a steroid. Preferably, said steroid is an anionic steroid.

Preferably, said anionic amphiphile comprises an anionic moiety and a positively charged counterion, such as an ammonium, alkali or alkaline earth metal ion. More preferably, said positively charged counterion is a sodium, potassium, or ammonium ion, again more preferably a sodium ion.

In a preferred embodiment, said hydrophilic group of said amphiphile is selected from the group consisting of a carboxylate, sulfate, sulfonate, boronate, phosphonate, phosphate, peptide, nucleic acid, amino acid moiety or peptide nucleic acid. In a preferred embodiment, said hydrophilic group of said anionic amphiphile is selected from the group consisting of anions of carboxylate, sulfate, sulfonate, boronate, phosphonate, phosphate, peptide, nucleic acid, amino acid moiety or peptide nucleic acid.

In a preferred embodiment, said amino acid moiety comprises, preferably consists of one or two covalently coupled amino acids. In a preferred embodiment, said peptide moiety comprises, preferably consists of more than two amino acids. Said amino acids of the amino acid or peptide moiety of the hydrophilic group comprises hydrophilic amino acids, such as negatively charged Asp and Glu, or positively charged Lys, His and Arg, preferably negatively charged Asp and Glu.

In a preferred embodiment, said nucleic acid moiety comprises, preferably consists of DNA, RNA including analogs thereof, in the size of 5-500 base pairs, preferably 5-300 base pairs, more preferably from 5-200 base pairs, or again more preferably 5-100 base pairs, again more preferably 5-50 base pairs. Analogs of DNA or RNA are structurally similar to the native nucleic acid, but differ from the native nucleic acid (e.g., through chemical modification) at one or more of the nucleic acid backbone (e.g., phosphate in native nucleic acids), nucleic acid sugar (e.g., deoxyribose for native DNA and ribose in native RNA), and nucleic acid base (e.g., adenosine, cytosine, guanine, thymidine, or purine in native nucleic acids.) Nucleic acid analogs and mimics commonly result from modifications of native nucleic acids at the nucleobase (e.g., modified base), the sugar (e.g., fluorinated or deoxy sugars), and/or the phosphodiester backbone (e.g., peptide or thioester backbones). Nucleic acid analogs and mimics are known to those of skill in the art and include, for example, locked nucleic acids (LNAs), peptide nucleic acids (PNAs), and morpholinos.

In a more preferred embodiment, said hydrophilic group is selected from the group consisting of a carboxylate, sulfate, sulfonate, boronate, phosphonate and phosphate moiety. In an again more preferred embodiment, said hydrophilic group is selected from the group consisting of a carboxylate, sulfate, sulfonate and phosphate moiety. Again more preferably said hydrophilic group is a sulfate or phosphate moiety. Again more preferably, said hydrophilic group is a sulfate moiety.

In another preferred embodiment, said hydrophilic group comprises an anionic moiety. In another preferred embodiment, said hydrophilic group comprises an anionic moiety selected from the group consisting of a carboxylate, sulfate, sulfonate, phosphonate, boronate, phosphate and amino acid moiety. In another preferred embodiment, said hydrophilic group consists of an anionic moiety is selected from the group consisting of a carboxylate, sulfate, sulfonate, phosphonate, boronate, phosphate and amino acid moiety. Preferably, said anionic moiety is a sulfate.

In another preferred embodiment, at least one of said one or more amphiphiles included in the surfactant composition consists of (i) a hydrophilic group selected from the group consisting of a carboxylate, sulfate, sulfonate, boronate, phosphonate, phosphate moiety and amino acid, and (ii) a hydrophobic group comprising at least one hydrocarbon moiety selected from the group consisting of linear or branched C4-C30 alkyl, C4-C30 alkenyl, C4-C30 alkynyl, C4-C30 alkoxy or C5-C30 cycloalkyl.

The term "carboxy moiety" or "carboxylate moiety" as used herein refers preferably to groups of R-CO₂⁻. The term "sulfate moiety" as used herein refers to groups of R-SO₄⁻. The term "sulfonate moiety" as used herein refers preferably to groups of R-SO₃⁻. The term "phosphonate moiety" as used herein refers preferably to groups of R-PO₃⁻-R or R-PO₃²⁻. The term "phosphonate moiety" as used herein refers preferably to groups of R-PO₄⁻-R, R-PO₄²⁻. The term "boronate moiety" as used herein refers preferably to groups of R-BO₂²⁻,

In another preferred embodiment, said amphiphile is defined as a salt of R-CO₂⁻, R-SO₄⁻, R-SO₃⁻, R-PO₄⁻-R, R-PO₄²⁻, R-PO₃⁻-R, R-PO₃²⁻, R-BO₂²⁻, wherein R consists of a hydrocarbon moiety selected from the group consisting of linear or branched C4-C30 alkyl, C4-C30 alkenyl, C4-C30 alkynyl, C4-C30 alkoxy or C5-C30 cycloalkyl. In a more preferred embodiment, said amphiphile is defined as a salt of R-CO₂⁻, R-SO₄⁻, R-SO₃⁻, R-PO₄⁻-, R-PO₄²⁻, R-PO₃⁻-R, R-PO₃²⁻, R-BO₂²⁻, wherein R consists of a hydrocarbon moiety selected from the group consisting of linear C4-C30 alkyl, C4-C30 alkenyl, C4-C30 alkynyl, C4-C30 alkoxy or C5-C30 cycloalkyl.

In a preferred embodiment, said hydrophilic group of at least one of said one or more amphiphiles included in the surfactant composition is a sulfate moiety. In a preferred embodiment, said at least one of said one or more amphiphiles included in the surfactant composition is a salt of a sulfate.

In a preferred embodiment, said hydrophilic group of said amphiphile comprises a sulfate moiety, and the hydrocarbon moiety of the hydrophobic group is selected from the group consisting of linear or branched alkyl or alkyl-ether residue, wherein the alkyl or alkyl-ether is C6 to C30, preferably C8 to C20, more preferably C10 to 18, even more preferably C12 to C18, most preferably C12, C14, C16 or C18 or a mixture thereof. In a preferred embodiment, said hydrophilic group comprises a sulfate moiety, and said hydrocarbon moiety is selected from the group consisting of linear or branched alkyl, alkyl-ether or alkenyl residue, wherein the alkyl or alkyl-ether residue is C6 to C30, preferably C8 to C20, more preferably C10 to 18, even more preferably C12 to C18, most preferably C12, C14, C16 or C18 or a mixture thereof. In a preferred embodiment, said hydrophilic group consists of a sulfate moiety, and said hydrocarbon moiety is selected from the group consisting of linear alkyl, alkyl-ether or alkenyl residue, wherein the linear alkyl or alkyl-ether residue is C6 to C30, preferably C8 to C20, more preferably C10 to 18, even more preferably C12 to C18, most preferably C12, C14, C16 or C18 or a mixture thereof.

In another preferred embodiment, said amphiphile is a salt of a primary or secondary alkyl sulfate (fatty alcohol) or alkyl-ether sulfate (fatty alcohol ether).

In another preferred embodiment, said amphiphile is a salt of a primary or secondary alkyl sulfate (fatty alcohol) or alkyl-ether sulfate (fatty alcohol ether), said sulfate comprises a sulfate moiety (as a hydrophilic group) and a linear alkyl residue, wherein the linear alkyl residue is C6 to C30, preferably C8 to C20, more preferably C10 to 18, even more preferably C12 to C18, most preferably C12, C14, C16 or C18 or a mixture thereof. In another preferred embodiment, said amphiphile is a salt of a primary or secondary alkyl sulfate (fatty alcohol) or alkyl-ether sulfate (fatty alcohol ether), said hydrophilic group of said amphiphile consists of a sulfate moiety, and said hydrocarbon moiety is a linear alkyl residue, wherein the linear alkyl residue is C6 to C30, preferably C8 to C20, more preferably C10 to 18, even more preferably C12 to C18, most preferably C12, C14, C16 or C18 or a mixture thereof. The fatty alcohols and fatty alcohol ethers can be synthetic or they can be derived from natural fats. Primary alkyl sulfate amphiphiles are defined herein as those compounds, which have the sulfate moiety at the terminal of the carbon chain. Secondary alkyl sulfate amphiphiles are defined herein as those compounds, which have the sulfate moiety distributed randomly along the carbon chain.

In a preferred embodiment, at least one of said one or more amphiphiles included in the surfactant composition is a salt of a primary or secondary alkyl sulfate (fatty alcohol comprising a sulfate moiety (as hydrophilic moiety) and a linear alkyl residue, wherein the linear alkyl residue is C6 to C30, preferably C8 to C20, more preferably C10 to 18, even more preferably C12 to C18, again even more preferably C12, C14, C16 or C18 or a mixture thereof. In another preferred embodiment, said amphiphile is a salt of a primary alkyl sulfate (fatty alcohol) comprising a sulfate moiety and a linear alkyl residue, wherein the linear alkyl residue is C6 to C30, preferably C8 to C20, more preferably C10 to 18, even more preferably C12 to C18, again even more preferably C12, C14, C16 or C18 or a mixture thereof. In another preferred embodiment, said amphiphile is a salt of a primary alkyl sulfate (fatty alcohol), said sulfate comprises a sulfate moiety and a linear alkyl residue, wherein the linear alkyl residue is C6 to C20, preferably C8 to C16, more preferably C10 to 12, even more preferably C12.

In a preferred embodiment, at least one of said one or more amphiphiles included in the surfactant composition is a salt of a primary or secondary alkyl sulfate (fatty alcohol), said sulfate comprises a sulfate moiety (as hydrophilic moiety), and a linear alkyl residue, wherein the linear alkyl residue is C6 to C30, preferably C8 to C20, more preferably C10 to 18, even more preferably C12 to C18, again even more preferably C12, C14, C16 or C18; and a salt of a steroid comprising an anionic moiety selected from the group consisting of sulfonate, sulfate, carboxylate, phosphonate, boronate, phosphate ester or amino acids. More preferably said steroid comprises an anionic moiety selected from the group consisting of a sulfonate, sulfate, carboxylate, phosphonate, boronate or phosphate moiety, and a steroid moiety. Again more preferably said steroid comprises an anionic moiety selected from the group consisting of a sulfonate, sulfate, carboxylate, phosphonate, or phosphate moiety, and a sterol, preferably a cholesterol moiety.

Preferably, said amphiphile is a salt of a sulfate of formula H₃C-(CH₂)ₙ-CH₂-O-SO₃⁻ or H₃C-(CH₂)ₙ-CH₂-(O-CH₂CH₂)ₘ-O-SO₃⁻, wherein m is a value from 1 to 4 and n is a value from 4 to 20, preferably 6 to 20, more preferably 8 to 18, even more preferably from 10 to 16, again even more preferably from 10 to 14, again even more preferably 10-12, most preferably 10. In another preferred embodiment m is 2 or 3 and n is a value from 4 to 20, preferably 6 to 20, more preferably 8 to 18, even more preferably from 10 to 16, again even more preferably from 10 to 14, again even more preferably 10-12, most preferably 10. In another preferred embodiment m is 2 and n is a value from 4 to 20, preferably 6 to 20, more preferably 8 to 18, even more preferably from 10 to 16, again even more preferably from 10 to 14, again even more preferably 10-12, most preferably 10.

Preferably, said amphiphile is a salt of a sulfate of formula H₃C-(CH₂)ₙ-CH₂-O-SO₃⁻, wherein n is a value from 4 to 20, preferably 6 to 20, more preferably 8 to 18, even more preferably from 10 to 16, again even more preferably from 10 to 14, again even more preferably 10-12, most preferably 10.

In another preferred embodiment, said amphiphile is a salt of a sulfate of formula H₃C-(CH₂)ₙ-CH₂-O-SO₃⁻, wherein n is a value from 4 to 20, preferably 6 to 20, more preferably 8 to 18, even more preferably from 10 to 16, again even more preferably from 10 to 14, again even more preferably 10-12, most preferably 10; and a steroid sulfonate, sulfate or carboxylate, phosphonate, boronate, phosphate ester of a steroid. More preferably said anionic steroid for these amphiphiles is a sulfonate, sulfate, carboxylate, phosphonate, boronate or phosphate ester of cholesterol.

In another preferred embodiment, said amphiphile is selected from the group consisting of salt of lauryl sulfate, laureth sulfate, pareth sulfate, myreth sulfate, n-octyl sulfate, 8-hexadecylsulfate, and tetradecyl sulfate. In another preferred embodiment, said amphiphile is selected from the group consisting of sodium lauryl sulfate, ammonium lauryl sulfate, potassium lauryl sulfate, sodium laureth sulfate, ammonium laureth sulfate, sodium pareth sulfate, sodium myreth sulfate, sodium n-octyl sulfate, sodium 8-hexadecylsulfate, and sodium tetradecyl sulfate. In another preferred embodiment, said amphiphile is a salt of lauryl sulfate or laureth sulfate.

Preferably, said salt of a sulfate is selected from an ammonium, alkali or alkaline earth salts, more preferably from a sodium, potassium, or ammonium salt, again more preferably a sodium salt.

In a preferred embodiment, said amphiphile is selected from the group consisting of sodium lauryl sulfate (sodium dodecyl sulfate, SLS, or SDS, herein typically referred to as SDS), ammonium lauryl sulfate, potassium lauryl sulfate, sodium laureth sulfate (sodium lauryl ether sulfate or SLES), ammonium laureth sulfate, sodium pareth sulfate, sodium myreth sulfate, sodium n-octyl sulfate, sodium 8-hexadecylsulfate, sodium dodecylbenzenesulfonate (SDBS) and sodium tetradecyl sulfate.

In another preferred embodiment, said surfactant composition comprises an amphiphile selected from the group consisting of SDS, ammonium lauryl sulfate, potassium lauryl sulfate, SLES, ammonium laureth sulfate, sodium pareth sulfate, sodium myreth sulfate, sodium n-octyl sulfate, sodium 8-hexadecylsulfate, and sodium tetradecyl sulfate combined with a steroid comprising an anionic moiety selected from the group consisting of a sulfonate, sulfate, carboxylate, phosphonate, boronate, and phosphate ester. More preferably said steroid is a sulfonate, sulfate, carboxylate, phosphonate, boronate or phosphate ester of cholesterol.

In a preferred embodiment, said amphiphile is an ammonium, alkali or alkaline earth salt of lauryl sulfate or laureth sulfate. In a more preferred embodiment, said amphiphile is a sodium, potassium, or ammonium salt of lauryl sulfate or laureth sulfate. In another preferred embodiment, said amphiphile is a sodium salt of lauryl sulfate or laureth sulfate.

In a preferred embodiment, said amphiphile is an ammonium, alkali or alkaline earth salt of lauryl sulfate (sodium dodecyl sulfate, SLS, or SDS). In a more preferred embodiment, said amphiphile is a sodium, potassium, or ammonium salt of lauryl sulfate. Most preferably, said amphiphile is sodium lauryl sulfate.

In a preferred embodiment, said hydrophilic group of at least one of said one or more amphiphiles included in the surfactant composition is a sulfonate, boronate or phosphonate moiety. In a preferred embodiment, said at least one of said one or more amphiphiles included in the surfactant composition is a salt of a sulfonate, boronate or phosphonate.

In a preferred embodiment, said hydrophilic group of at least one of said one or more amphiphiles included in the surfactant composition is a sulfonate, boronate or phosphonate moiety; and said hydrocarbon moiety of said hydrophobic group is selected from the group consisting of alkyl, alkyl-benzene, benzene-alky, alky-ester, alkenyl, alkyl-succinate, alkyl-acetate and alkyl-tauride, wherein alkyl or alkenyl is linear or branched, preferably linear C6 to C30. Said hydrophilic group of at least one of said one or more amphiphiles included in the surfactant composition is a sulfonate, boronate or phosphonate moiety; and said hydrocarbon moiety is selected from the group consisting of alkyl, alkyl-benzene, benzene-alky, alky-ester, alkenyl, alkyl-succinate, alkyl-acetate and alkyl-tauride, wherein alkyl or alkenyl is linear or branched, preferably linear C8 to C20. said hydrophilic group of at least one of said one or more amphiphiles included in the surfactant composition is a sulfonate, boronate or phosphonate moiety and said hydrocarbon moiety is selected from the group consisting of alkyl, alkyl-benzene, benzene-alky, alky-ester, alkenyl, alkyl-succinate, alkyl-acetate and alkyl-tauride, wherein alkyl or alkenyl is linear or branched, preferably linear C10 to 18. said hydrophilic group of at least one of said one or more amphiphiles included in the surfactant composition is a sulfonate, boronate or phosphonate moiety and said hydrocarbon moiety is selected from the group consisting of alkyl, alkyl-benzene, benzene-alky, alky-ester, alkenyl, alkyl-succinate, alkyl-acetate and alkyl-tauride, wherein alkyl or alkenyl is linear or branched, preferably linear C12 to C18 or a mixture thereof. said hydrophilic group of at least one of said one or more amphiphiles included in the surfactant composition is a sulfonate, boronate or phosphonate moiety and said hydrocarbon moiety is selected from the group consisting of alkyl, alkyl-benzene, benzene-alky, alky-ester, alkenyl, alkyl-succinate, alkyl-acetate and alkyl-tauride, wherein alkyl or alkenyl is linear or branched, preferably linear C12, C14, C16 or C18 or a mixture thereof. said hydrophilic group of at least one of said one or more amphiphiles included in the surfactant composition is a sulfonate, boronate or phosphonate moiety and said hydrocarbon moiety is selected from the group consisting of alkyl, alkyl-benzene, benzene-alky, alky-ester, alkenyl, alkyl-succinate, alkyl-acetate and alkyl-tauride, wherein alkyl or alkenyl is linear or branched, preferably linear C12 or C14 or a mixture thereof.

In a preferred embodiment, at least one of said one or more amphiphiles included in the surfactant composition is a salt of a sulfonate, wherein said sulfonate is selected from the group consisting of primary alkylsulfonate, secondary alkyl sulfonate, sulfonated alkylester, alkylestersuphonates, alpha olefin sulfonate, arylalkylsulfonates, alkylarylsulfonates, alkylbenzenesulfonates (ABS), benzenealkylsulfonates, alkylsulfoacetates, alkylsulfosuccinates, alkyltaurides, sulfolipids and sulfoglycolipids. In another preferred embodiment, said amphiphile is a salt of a sulfonate selected from the group consisting of primary alkylsulfonate, secondary alkyl sulfonate, sulfonated alkyl ester, alkylestersuphonates, arylalkylsulfonates, alkylarylsulfonates, alkylbenzenesulfonates, benzenealkylsulfonates, alkylsulfoacetates, alkylsulfosuccinates, and alkyltaurides. In a more preferred embodiment, said amphiphile is selected from the group consisting of primary alkylsulfonate, secondary alkyl sulfonate, sulfonated alkyl ester, alkylestersuphonates, alpha olefin sulfonate, alkylbenzenesulfonates, benzenealkylsulfonates, alkylsulfoacetates, alkylsulfosuccinates, and alkyltaurides. In an even more preferred embodiment, said amphiphile is a salt of a primary alkylsulfonate or secondary alkyl sulfonate

In a preferred embodiment, said amphiphile is a salt of a sulfonate comprising a linear or branched, preferably linear alkyl or alkenyl residue, said salt of the sulfonate is selected from the group consisting of primary alkylsulfonate, secondary alkyl sulfonate, sulfonated alkylester, alkylestersulfonates, alpha olefin (alkenyl) sulfonate, alkylbenzenesulfonates (ABS), benzenealkylsulfonates, alkylsulfoacetates, alkylsulfosuccinates, and alkyltaurides, wherein the alkyl residue is C6 to C30, preferably C8 to C20, more preferably C10 to 18, even more preferably C12 to C18, most preferably C12, C14, C16 or C18 or a mixture thereof. In another preferred embodiment, said amphiphile is a salt of a primary or secondary alkyl sulfonate comprising a linear or branched alkyl residue of C6 to C30, preferably C8 to C20, more preferably C10 to 18, even more preferably C12 to C18, most preferably C12, C14, C16 or C18 or a mixture thereof. In another preferred embodiment, said amphiphile is a salt of a primary alkylsulfonate or secondary alkyl sulfonate comprising a linear alkyl residue of C6 to C30, preferably C8 to C20, more preferably C10 to 18, even more preferably C12 to C18, most preferably C12, C14, C16 or C18 or a mixture thereof.

Preferably, said salt of a sulfate, sulfonate, boronate or phosphonate is selected from an ammonium, alkali or alkaline earth salts, more preferably from a sodium, potassium, or ammonium salt, again more preferably a sodium salt.

In a preferred embodiment, at least one of said one or more amphiphiles included in the surfactant composition is a salt of a primary or secondary alkylsulfate, or a salt of a primary or secondary alkyl sulfonate, wherein the alkyl residue of said alkylsulfate or alkylsulfonate is linear C6 to C30, or salt of a sulfate of formula H₃C-(CH₂)ₙ-CH₂-(O-CH₂CH₂)ₘ-O-SO₃⁻, wherein m is a value from 1 to 4 and n is a value from 4 to 20. Preferably, said amphiphile is a salt of a primary or secondary alkylsulfate or a salt of a primary or secondary alkyl sulfonate, wherein the alkyl residue of said alkylsulfate or alkylsulfonate is linear C8 to C20, or a salt of a sulfate of formula H₃C-(CH₂)ₙ-CH₂-(O-CH₂CH₂)ₘ-O-SO₃⁻, wherein m is a value from 1 to 4 and n is a value from 6 to 18. Preferably, said amphiphile is a salt of a primary or secondary alkylsulfate or a salt of a primary or secondary alkyl sulfonate, wherein the alkyl residue of said alkylsulfate or alkylsulfonate is linear C10 to C18, or a salt of a sulfate of formula H₃C-(CH₂)ₙ-CH₂-(O-CH₂CH₂)ₘ-O-SO₃⁻, wherein m is a value from 2 or 3 and n is a value from 8 to 16. Preferably, said amphiphile is a salt of a primary or secondary alkylsulfate or a salt of a primary or secondary alkyl sulfonate, wherein the alkylsulfate or alkyl residue of said alkylsulfonate is linear C10 to C16, or a salt of a sulfate of formula H₃C-(CH₂)ₙ-CH₂-(O-CH₂CH₂)ₘ-O-SO₃⁻, wherein m is a value from 2 or 3 and n is a value from 8 to 14. Preferably, said amphiphile is a salt of a primary or secondary alkylsulfate or a salt of a primary or secondary alkyl sulfonate, wherein the alkyl residue of said alkylsulfate or alkylsulfonate is linear C10 to C14, or a salt of a sulfate of formula H₃C-(CH₂)ₙ-CH₂-(O-CH₂CH₂)ₘ-O-SO₃⁻, wherein m is a value from 2 or 3 and n is a value from 8 to 12. Preferably, said amphiphile is a salt of a primary or secondary alkylsulfate or a salt of a primary or secondary alkyl sulfonate, wherein the alkyl residue of said alkylsulfate or alkylsulfonate is linear C12, or a salt of a sulfate of formula H₃C-(CH₂)ₙ-CH₂-(O-CH₂CH₂)ₘ-O-SO₃⁻, wherein m is a value from 2 or 3 and n is 10. Preferably, said salts of said sulfates or sulfonates are selected from an ammonium, alkali or alkaline earth salts, more preferably from a sodium, potassium, or ammonium salt, again more preferably a sodium salt.

In a preferred embodiment, said hydrophilic group of at least one of said one or more amphiphiles included in the surfactant composition is a carboxyl moiety. In a preferred embodiment, at least one of said one or more amphiphiles included in the surfactant composition is a salt of a carboxylate.

In a preferred embodiment, said hydrophilic group is a carboxy moiety, and said hydrocarbon moiety of said hydrophobic group is selected from the group consisting of alkyl, alkynyl, alkenyl or fatty acid residue, preferably a linear alkyl, alkenyl or fatty acid residue. In a preferred embodiment, said hydrophilic group is a carboxy moiety, and said hydrocarbon moiety of said hydrophobic group is selected from the group consisting of linear alkyl, alkynyl, alkenyl or fatty acid residue, wherein the alkyl or alkenyl residue is C6 to C30, preferably C8 to C20, more preferably C10 to 18, even more preferably C12 to C18, most preferably C12, C14, C16 or C18 or a mixture thereof.

In a preferred embodiment, said hydrophilic group is a carboxy moiety, and said hydrocarbon moiety of said hydrophobic group is a linear alkyl residue, wherein the linear alkyl residue is C6 to C30, preferably C8 to C20, more preferably C10 to 18, even more preferably C12 to C18, most preferably C12, C14, C16 or C18 or a mixture thereof.

In a preferred embodiment, said hydrophilic group is a carboxy moiety, and said hydrocarbon moiety of said hydrophobic group is a linear or branched moiety selected from alkyl, alkynyl, alkenyl or fatty acid, alkyl ester, alkyl ether, alkyl polyglycol or alkysarcosinate, wherein alkyl or alkenyl is C6 to C30, preferably C8 to C20, more preferably C10 to 18, even more preferably C12 to C18, most preferably C12, C14, C16 or C18 or a mixture thereof. In a preferred embodiment, said hydrophilic group is a carboxy moiety, and said hydrocarbon moiety of said hydrophobic group is a linear alkyl residue, wherein the linear alkyl residue is C6 to C30, preferably C8 to C20, more preferably C10 to 18, even more preferably C12 to C18, most preferably C12, C14, C16 or C18 or a mixture thereof.

In a preferred embodiment, at least one of said one or more amphiphiles included in the surfactant composition is a amphiphile selected from the group consisting of primary or secondary alkylsulfate, primary or secondary alkylsulfonate, alkylsarcosinate or alkylcarboxylate, wherein the alkyl residue of said primary or secondary alkylsulfate, alkylsulfonate, alkylsarcosinate or alkylcarboxylate is linear or branched, preferably linear C6 to C30; or a salt of a sulfate of formula H₃C-(CH₂)ₙ-CH₂-(O-CH₂CH₂)ₘ-O-SO₃⁻, wherein m is a value from 1 to 4 and n is a value from 4 to 20. More preferably, said amphiphile is a salt selected from the group consisting of primary or secondary alkylsulfate, primary or secondary alkylsulfonate, alkylsarcosinate or alkylcarboxylate, wherein the alkyl residue of said primary or secondary alkylsulfate, alkylsulfonate, alkylsarcosinate or alkylcarboxylate is linear or branched, preferably linear C8 to C20; or a salt of a sulfate of formula H₃C-(CH₂)ₙ-CH₂-(O-CH₂CH₂)ₘ-O-SO₃⁻, wherein m is 2 or 3 and n is a value from 6 to 18. More preferably, said amphiphile is a salt selected from the group consisting of primary or secondary alkylsulfate, primary or secondary alkylsulfonate, alkylsarcosinate or alkylcarboxylate, wherein the alkyl residue of said primary or secondary alkylsulfate, alkylsulfonate, alkylsarcosinate or alkylcarboxylate is linear or branched, preferably linear C10 to C18; or a salt of a sulfate of formula H₃C-(CH₂)ₙ-CH₂-(O-CH₂CH₂)ₘ-O-SO₃⁻, wherein m is 2 or 3 and n is a value from 8 to 16. More preferably, said amphiphile is a salt selected from the group consisting of primary or secondary alkylsulfate, primary or secondary alkylsulfonate, alkylsarcosinate or alkylcarboxylate, wherein the alkyl residue of said primary or secondary alkylsulfate, alkyl sulfonate, alkylsarcosinate or alkylcarboxylate is linear or branched, preferably linear C12 to C18; or a salt of a sulfate of formula H₃C-(CH₂)ₙ-CH₂-(O-CH₂CH₂)ₘ-O-SO₃⁻, wherein m is 2 or 3 and n is a value from 10 to 16. More preferably, said amphiphile is a salt selected from the group consisting of primary or secondary alkylsulfate, primary or secondary alkylsulfonate, alkylsarcosinate or alkylcarboxylate, wherein the alkyl residue of said primary or secondary alkylsulfate, alkyl sulfonate, alkylsarcosinate or alkylcarboxylate is linear or branched, preferably linear C12 to C16; or a salt of a sulfate of formula H₃C-(CH₂)ₙ-CH₂-(O-CH₂CH₂)ₘ-O-SO₃⁻, wherein m is 2 or 3 and n is a value from 10 to 14.

In a preferred embodiment, at least one of said one or more amphiphiles included in the surfactant composition is an amphiphilic lipid. The term "amphiphilic lipid" as used herein refers to amphiphilic compounds which contain both hydrophilic moieties and hydrophobic moieties comprising hydrocarbons selected from oils, fats (such as fatty acids, glycerides), sterols, steroids, and derivative forms of these compounds. Suitable amphiphilic lipids include moieties derived from fatty acids and their derivatives, hydrocarbons and their derivatives, and sterols, such as cholesterol. In a preferred embodiment, said amphiphilic lipid is selected from the group consisting of phospholipids, sphingolipids, glycerolipids, and saccharolipids.

In a preferred embodiment, said hydrophilic group of at least one of said one or more amphiphiles included in the surfactant composition is a phosphate moiety. In a preferred embodiment, at least one of said one or more amphiphiles included in the surfactant composition is a phosphate salt.

In a preferred embodiment, said hydrophilic group is a phosphate moiety, and said hydrocarbon moiety of said hydrophobic group is selected from the group consisting of fatty acid, fatty acid ester, alkyl, alkenyl, glyceride moiety. Preferably, said fatty acid, fatty acid ester, alkyl or alkenyl is linear. Preferably, said hydrophobic group is selected from the group consisting of saturated fatty acid, fatty acid ester, linear alkyl or linear alkenyl.

The term "fatty acid", as used herein, refers to a hydrocarbon chain that terminates with a carboxylic acid group, wherein said hydrocarbon chain is typically and preferably either an alkyl or alkenyl of typically 3 to 32 carbons long, and that are, thus, saturated or unsaturated, and that are optionally substituted by one or more, preferably one, carboxylic group (-COOH), one or more, preferably one, C1-32 alkyl, one or more, preferably one, phosphate group (HOP(O)(OH)O-), one or more, preferably one, phosphonate group (HOP(O)O-), one or more, preferably one, thiophosphate group (HOP(O)(SH)O-), one or more, preferably one, dithiophosphate group (HOP(S)(SH)O-), one or more, preferably one, diphosphate group (HOP(O)(OH)-O-P(O)(OH)-O-), one or more, preferably one, triphosphate group (HO-P(O)(OH)-O-P(O)(OH)-O-P(O)(OH)-O-), one or more phenyl group (-C₆H₅), one or more phenyl group substituted with a halogen, preferably iodine, or a carboxylic group. If a fatty acid contains one or more double bonds, and is thus unsaturated, there is the possibility of either a cis or trans geometric isomerism. The term "fatty acid moiety", as used herein, refers to a moiety derived from a fatty acid, as defined herein, wherein one carboxylic group (-COOH) of said fatty acid becomes and is a -C(O)- group of said fatty acid moiety, which -C(O)- group is linked to said oligonucleotide either directly or via spacer in accordance with the present invention. The term "fatty acid" includes fatty diacids which refer to fatty acids as defined herein but with an additional carboxylic acid group in the omega position. In a preferred embodiment, said amphiphile is a phosphate salt comprising a phosphate moiety and at least one linear or branched, preferably linear C6 to C30 chain of fatty acid, fatty acid ester, alkyl or alkenyl. In a further preferred embodiment, said amphiphile is a phosphate salt comprising a phosphate moiety and at least one C8 to C20 chain of a fatty acid, alkyl or alkenyl. In a further preferred embodiment, said amphiphile is a phosphate salt comprising a phosphate moiety and at least one C10 to C18 chain of fatty acid, alkyl or alkenyl. In further preferred embodiment, said amphiphile is a phosphate salt comprising a phosphate moiety and at least one C12 to C18 chain of fatty acid, alkyl or alkenyl. In further preferred embodiment, said amphiphile is a phosphate salt comprising a phosphate moiety and at least one C12 to C18 chain of fatty acid, alkyl or alkenyl. In further preferred embodiment, said amphiphile is a phosphate salt comprising a phosphate moiety and at least one C12, C14, C16 or C18 chain or a mixture thereof of fatty acid, alkyl or alkenyl. Preferably, said alkyl or alkenyl is linear. More preferably said alkyl or alkenyl is linear and said fatty acid is unsaturated.

In a preferred embodiment, at least one of said one or more amphiphiles included in the surfactant composition is a phosphate salt selected from the group consisting of mono-alkyl phosphate ester salts, di-alkyl phosphate ester salts, mono-alkenyl phosphate ester salts, dialkenyl phosphate ester salts and phospholipids.

In a preferred embodiment, at least one of said one or more amphiphiles included in the surfactant composition is a phospholipid. Preferably, said phospholipid consists of at least two hydrophobic fatty acid moieties, preferably exactly two fatty acid moieties, and a hydrophilic phosphate moiety. The moieties are preferably covalently linked by a glycerol moiety. The phosphate moiety is preferably modified with simple organic molecules such as choline, ethanolamine or serine.

In a preferred embodiment, at least one of said one or more amphiphiles included in the surfactant composition is a phosphate salt selected from the group consisting of mono-alkyl phosphate ester salts, di-alkyl phosphate ester salts, mono-alkenyl phosphate ester salts, dialkenyl phosphate ester salts and phospholipids, wherein said alkyl or alkenyl of the phosphate ester salts or the fatty acids of the phospholipids is linear C6 to C30, preferably C8 to C20, more preferably C10 to 18, even more preferably C12 to C18, most preferably C12, C14, C16 or C18 or a mixture thereof.

In a preferred embodiment, at least one of said one or more amphiphiles included in the surfactant composition is a salt selected from the group consisting of primary or secondary alkylsulfate, primary or secondary alkylsulfonate, alkylphosphonate, alkylboronate, alkylsarcosinate, alkylcarboxylate, mono-alkyl phosphate ester, or di-alkyl phosphate ester, wherein the alkyl residue of said alkyl sulfate, alkyl sulfonate, alkylphosphonate, alkylboronate, alkylsarcosinate or alkylcarboxylate or phosphate ester is linear C6 to C30; or a salt of a sulfate of formula H₃C-(CH₂)ₙ-CH₂-(O-CH₂CH₂)ₘ-O-SO₃⁻, wherein m is a value from 1 to 4 and n is a value from 4 to 20. More preferably, said amphiphile is a salt selected from the group consisting of primary or secondary alkylsulfate, primary or secondary alkylsulfonate, alkylphosphonate, alkylboronate, alkylsarcosinate or alkylcarboxylate, mono-alkyl phosphate ester, or di-alkyl phosphate ester, wherein the alkyl residue of said alkyl sulfate, alkyl sulfonate, alkylphosphonate, alkylboronate, alkylsarcosinate or alkylcarboxylate or phosphate ester is linear C8 to C30; or a salt of a sulfate of formula H₃C-(CH₂)ₙ-CH₂-(O-CH₂CH₂)ₘ-O-SO₃⁻, wherein m is 2 or 3 and n is a value from 6 to 18. More preferably, said amphiphile is a salt selected from the group consisting of primary or secondary alkylsulfate, primary or secondary alkylsulfonate, alkylphosphonate, alkylboronate, alkylsarcosinate or alkylcarboxylate, mono-alkyl phosphate ester, or di-alkyl phosphate ester, wherein the alkyl residue of said alkylsulfate, alkyl sulfonate, alkylphosphonate, alkylboronate, alkylsarcosinate or alkylcarboxylate or phosphate ester is linear C10 to C18; or a salt of a sulfate of formula H₃C-(CH₂)ₙ-CH₂-(O-CH₂CH₂)ₘ-O-SO₃⁻, wherein m is 2 or 3 and n is a value from 8 to 16. More preferably, said amphiphile is a salt selected from the group consisting of primary or secondary alkylsulfate, primary or secondary alkylsulfonate, alkylphosphonate, alkylboronate, alkylsarcosinate or alkylcarboxylate, mono-alkyl phosphate ester, or di-alkyl phosphate ester, wherein the alkyl residue of said primary or secondary alkylsulfate, alkyl sulfonate, alkylphosphonate, alkylboronate, alkylsarcosinate or alkylcarboxylate or phosphate ester is linear C12 to C18; or a salt of a sulfate of formula H₃C-(CH₂)ₙ-CH₂-(O-CH₂CH₂)ₘ-O-SO₃⁻, wherein m is 2 or 3 and n is a value from 10 to 16. More preferably, said amphiphile is a salt selected from the group consisting of primary or secondary alkylsulfate, primary or secondary alkylsulfonate, alkylphosphonate, alkylboronate, alkylsarcosinate or alkylcarboxylate, mono-alkyl phosphate ester, or di-alkyl phosphate ester, wherein the alkyl residue of said primary or secondary alkyl sulfate, alkyl sulfonate, alkylphosphonate, alkylboronate, alkylsarcosinate or alkylcarboxylate or phosphate ester is linear C12 to C16; or a salt of a sulfate of formula H₃C-(CH₂)ₙ-CH₂-(O-CH₂CH₂)ₘ-O-SO₃⁻, wherein m is 2 or 3 and n is a value from 10 to 14.

In a preferred embodiment the molar ratio of total surfactant molecules (i.e. anionic amphiphile and optional anionic steroid) to protein cages encapsulated into the assembled protein cage is up to about 1000: 1. In a preferred embodiment, the molar ratio of total surfactant molecules to protein cages is about 800:1.

In another preferred embodiment said anionic amphiphile is a salt of dodecylsulfate, preferably sodium dodecyl sulfate, and the molar ratio of dodecylsulfate molecules encapsulated into the assembled protein cage to protein cages is up to about 1000: 1. In a preferred embodiment said anionic amphiphile is a salt of dodecylsulfate, preferably sodium dodecyl sulfate (SDS), and the molar ratio of dodecylsulfate molecules encapsulated into the assembled protein cage to protein cages is up to about 800:1. This is based on the data that indicate that no change to the quaternary structure of the protein cage of the invention occurs at a concentration of about 800 equivalent of SDS, while more than 1000 equivalents gave rise to bands of increased mobility, potentially due to external association of the SDS molecules with the protein cage.

In a preferred embodiment, said hydrocarbon moiety is selected from the group consisting of a fatty acid, fatty acid ester, steroid, sterol, steroid ester.

In another preferred embodiment, said one or more amphiphile included in the surfactant composition comprises an amphiphilic steroid. In another preferred embodiment, said one or more amphiphile included in the surfactant composition comprises an anionic amphiphilic steroid. In another preferred embodiment, said one or more amphiphile included in the surfactant composition comprises an anionic amphiphile and an anionic steroid. In another preferred embodiment, said one or more amphiphile included in the surfactant composition comprises an anionic amphiphile and an anionic steroid, wherein the hydrophilic group of said anionic amphiphile and anionic steroid is selected from the group consisting of sulfonate, sulfate, carboxylate, phosphonate, boronate, and phosphate moiety. More preferably, in another preferred embodiment, said one or more amphiphile included in the surfactant composition comprises an anionic amphiphile and an anionic steroid, wherein the hydrophilic group of said anionic amphiphile and anionic steroid is a sulfate moiety.

The term "steroid" as used herein preferably comprises steroids, steroid esters and sterols, more preferably anionic comprises steroids, steroid esters and sterols.

Preferably, said anionic steroid is selected from the group consisting of a steroid comprising a sulfonate, sulfate, carboxylate, phosphonate, boronate, phosphate, phosphate ester or hydrophilic amino acids. More preferably, said anionic steroid is selected from the group consisting of steroid sulfonate, steroid sulfate and steroid phosphate ester. More preferably said anionic steroid comprises an anionic moiety selected from the group consisting of sulfonate, sulfate, carboxylate, phosphonate, boronate, phosphate; and a steroid moiety selected from the group consisting of estradiol, estriol, diethylstilbestrol, dehydroepiandrosterone, cholesterol, pregnenolone, DHEA, androstenediol, androsterone, estrone, testosterone. In another preferred embodiment, said anionic steroid is selected from the group consisting of estriol sulfate, estradiol sulfate, estradiol disulfate (EDS), diethylstilbestrol disulfate, dehydroepiandrosterone sulfate, cholesterol sulfate, pregnenolone sulfate, DHEA sulfate, androstenediol sulfate, androsterone sulfate, estrone sulfate, estradiol sulfate, testosterone sulfate. Most preferably, said anionic steroid is cholesterol sulfate (CS).

In a preferred embodiment, said anionic amphiphile of the surfactant composition of the invention is sodium dodecyl sulfate (SDS) and said anionic steroid is cholesterol sulfate (CS).

In a preferred embodiment, said a surfactant composition comprises an anionic amphiphile and an anionic steroid encapsulated into the assembled protein cage, wherein the molar ratio of the anionic amphiphile and anionic steroid within the surfactant composition is from 50% anionic amphiphile/50% anionic steroid to 100% anionic amphiphile/0% anionic steroid, more preferably from 75% anionic amphiphile/25% anionic steroid to 100% anionic amphiphile/0% anionic steroid. In a preferred embodiment, said anionic steroid is used encapsulation of large planar cargo molecules, wherein preferably the surfactant composition comprises 75% of anionic amphiphile and 25% anionic steroid, indicated as molar ratio.

In a preferred embodiment, said anionic amphiphile is SDS and said anionic steroid is CS, wherein SDS is included in the surfactant composition from 100% to 50%, preferably from 100% to 75% in relation to CS, indicated as molar ratio.

In a preferred embodiment, said surfactant composition comprises SDS and CS. In a preferred embodiment, said surfactant composition consists of SDS and CS. In a preferred embodiment, said surfactant composition comprises SDS and CS, and wherein said molar ratio of said SDS to CS is equal or between 4 to 1 and 2 to 1. In a preferred embodiment, said surfactant composition consists of SDS and CS, and wherein said molar ratio of said SDS to CS is equal or between 4 to 1 and 2 to 1. In a preferred embodiment, said surfactant composition comprises SDS and CS, and wherein said molar ratio of said SDS to CS is 3 to 1. In a preferred embodiment, said surfactant composition consists of SDS and CS, and wherein said molar ratio of said SDS to CS is 3 to 1.

Said a protein cage comprising at least one polypeptide comprising an amino acid sequence I consisting of: 
MX13QAIGILELX1SIAAGMELGDAMLKSAX14VX15LLVSKTISX2GKFLLMLGGDIX8AI
X9X12AIX10TGTX11QAGX3LLVDSLVLAX16IHPSVLPAIX17GX18NX19VX20X7X21QAVGI
VETX4SVAACISAADX22AVX23GSX24VTLVRVHMAX5GIGGKCYMVVAGDVSDVALA
VTVASSSAGAYGX6LVYASLIPX25PHX26AMWX27QMVX28GX29E (SEQ ID NO: 1),
 wherein any of X₁ to X₂₉ are independently of each other an amino acid, provided that at least 3 of X₁ to X₆ are independently of each other a positively charged amino acid, and wherein optionally up to 5 amino acids in positions other than denoted by X₁ to X₂₉ in SEQ ID NO: 1 are exchanged by any amino acid;

The term "polypeptide" as used herein refers to any peptide-bond-linked polymer of amino acids, regardless of size, length, secondary and tertiary structure, number of subunits or post-translational modification. Thus, the term "polypeptide" is to be understood as covering the terms "peptide", "protein", "amino acid chain", "amino acid sequence". Polypeptides in accordance with the invention can be an open linear peptide chain or cyclic peptides; alternatively or additionally, peptides of the invention may include at least one chemical modification, such as lipidation, glycosylation and phosphorylation. Peptides, as understood herein, especially peptides of the invention, are isolated or, preferably can be produced by chemical synthesis, RNA translation and/or recombinant processes.

The term "amino acid", as used herein, refers to organic compounds containing the functional groups amine (-NH2) and carboxylic acid (-COOH) and its zwitterions, typically and preferably, along with a side chain specific to each amino acid. The term "amino acid" typically and preferably includes amino acids that occur naturally, such as proteinogenic amino acids (produced by RNA-translation), non-proteinogenic amino acids (produced by other metabolic mechanisms, e.g. posttranslational modification), standard or canonical amino acids (that are directly encoded by the codons of the genetic code) and non-standard or non-canonical amino acids (not directly encoded by the genetic code). Naturally occurring amino acids include proteinogenic and non-proteinogenic amino acids. The term "amino acid", as used herein, also includes unnatural amino acids that are chemically synthesized. Moreover, the term covers alpha- (α-), beta- (β-), gamma- (γ-) and delta- (δ-) etc. amino acids as well as mixtures thereof in any ratio, and, if applicable, any isomeric form of an amino acid, i.e. its D- and L-stereoisomers (alternatively addressed by the (*R*) and (*S*) nomenclature) as well as mixtures thereof in any ratio, preferably in a racemic ratio of 1:1. Amino acids in this invention are typically and preferably in L-configuration. The term "D-stereoisomer", "L-stereoisomer", "D-amino acid" or "L-amino acid" refers to the chiral alpha carbon of the amino acids. Amino acid can include modifications and/or attached compounds and residues, for example residues used for peptide synthesis, such as Boc, Fmoc or both.

The terms "Xₙ to Xₘ" and "Xₙ₋ₘ" are used interchangeably herein for denoting certain amino acid positions in SEQ ID NO: 1.

Optionally up to five amino acids in positions other than denoted by X₁₋₂₉ in SEQ ID NO: 1 can be exchanged by any amino acid. The term "amino acid exchange" or "exchanged by any amino acid" used interchangeably herein includes or preferably refers to an exchange by deletion of one amino acid or substitution of a single amino acid by one or more amino acids (addition), more preferably by one, two or three amino acids. Most preferably, the term "amino acid exchange" refers to deletion of a single amino acid or substitution of a single amino acid by one, two or three amino acids. In a preferred embodiment, said amino acid exchange is a substitution. In another preferred embodiment, said amino acid exchange is a conservative amino acid substitution.

The term "conservative substitution" is an amino acid substitution that changes a given amino acid to a different amino acid with similar biochemical properties. Conservative substitutions include and preferably refer to isosteric substitutions and substitutions where the charged, polar, aromatic, aliphatic or hydrophobic nature of the amino acid is maintained. Conservative substitutions refer to substitutions that maintain the capability of the polypeptide of the invention to self-assemble into the protein cage of the invention.

The term "positively charged" as used herein includes and preferably refers to a molecule that has a positively charged group. More preferably, said positively charged molecule has a positively charged group at neutral or physiological pH.

In preferred embodiments of the present invention, optionally up to 4 amino acids, more preferably up to 3 amino acids, again more preferably up to 2 amino acids, most preferably 1 amino acid in positions other than denoted by X₁₋₂₉ in SEQ ID NO: 1 is/are exchanged by any amino acid.

In a preferred embodiment, said polypeptide has a length of about 500 amino acids or less, preferably about 400 amino acids or less, more preferably 300 amino acids or less, again more preferably 250 amino acids or less, again more preferably 200 amino acids or less, again more preferably from 188 to 230 amino acids, most preferably 192 amino acids.

In another preferred embodiment, said polypeptide is an isolated polypeptide.

In another preferred embodiment, said polypeptide consists of an amino acid sequence consisting of SEQ ID NO: 1. In another preferred embodiment, said polypeptide consists of an amino acid sequence consisting of SEQ ID NO: 1, wherein optionally up to 5 amino acids in positions other than denoted by X₁₋₂₉ in SEQ ID NO: 1 are exchanged by any amino acid.

In another preferred embodiment, said positively charged amino acids of said at least 3 of X₁ to X₆ are independently of each other arginine or a conservative substitution of arginine. In another preferred embodiment, said positively charged amino acids of said at least 3 of said X₁ to X₆ are independently of each other selected from the group consisting of arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine, thialysine and histidine.

In another preferred embodiment, said positively charged amino acids of said at least 3 of X₁ to X₆ are independently of each other selected from the group consisting of arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine and thialysine.

In another more preferred embodiment, said positively charged amino acid of said at least 3 of X₁ to X₆ is independently of each other histidine, arginine or lysine. In another more preferred embodiment, said positively charged amino acid of said at least 3 of X₁ to X₆ is independently of each other arginine or lysine. In another more preferred embodiment, said at least 3 positively charged amino acid of said X₁ to X₆ are independently of each arginine. In one embodiment, each of said positively charged amino acids X₁ to X₆ is arginine. In one embodiment, said positively charged amino acids X₁ to X₆ is lysine.

X₇ is cysteine, a conservative substitution thereof or a positively charged amino acid.

In a preferred embodiment, said conservative substitution of cysteine is selected from the group consisting of methionine, homocysteine, selenocysteine, serine, hydroxynorvaline, 2-amino-5-hydroxypentanoic acid , allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. In a preferred embodiment, said conservative substitution of cysteine is selected from the group consisting of homocysteine, selenocysteine, serine, hydroxynorvaline, 2-amino-5-hydroxypentanoic acid , allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. More preferably, said conservative substitution of cysteine is selected from the group consisting of homocysteine, selenocysteine, and serine. Again more preferably, said conservative substitution of cysteine is homocysteine or selenocysteine.

In another preferred embodiment, X₇ is selected from the group consisting of homocysteine, selenocysteine, cysteine, arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine and thialysine.

In another preferred embodiment, X₇ is selected from the group consisting of homocysteine, selenocysteine, serine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine , 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine, arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine and thialysine. In another preferred embodiment, said X₇ is selected from arginine, lysine, serine, homocysteine, or cysteine. In another further preferred embodiment, said X₇ is selected from arginine, lysine, homocysteine, or cysteine.

If X₇ is cysteine or a conservative substitution of cysteine it could be used to append positively charged groups via disulfide formation (e.g. cysteamine, 1-(3-mercaptopropyl)guanidine etc.) to the polypeptide of the invention.

In one embodiment, said at least 3 of said X₁ to X₆ are independently of each other lysine or arginine, and said X₇ is selected from arginine, lysine, serine, homocysteine or cysteine.

In amino acid sequence I consisting of SEQ ID NO: 1, at least 3 of said amino acids X₁ to X₆ are independently of each other positively charged amino acids. In a preferred embodiment, at least 4, more preferably at least 5, again more preferably 6, i.e. each of said amino acids X₁ to X₆ is/are independently of each other a positively charged amino acid.

In a preferred embodiment, at least 4, preferably at least 5, more preferably 6, i.e. each of said amino acids X₁ to X₆ is/are independently of each other a positively charged amino acid, wherein said positively charged amino acid is arginine or lysine. In a preferred embodiment, at least 4, preferably at least 5, more preferably each of said amino acids of X₁ to X₆ is/are independently of each other a positively charged amino acids wherein said positively charged amino acids are arginine. In a preferred embodiment, at least 4, preferably at least 5, more preferably 6 of said amino acids X₁ to X₆ are independently of each other positively charged amino acids, wherein said positively charged amino acids are lysine.

In a preferred embodiment, said at least 3 of X₁ to X₆ being independently of each other a positively charged amino acid are X₁ to X₃. In another preferred embodiment, said at least 3 of X₁ to X₆ being independently of each other a positively charged amino acid are X₁, X₂, and X₄. In another preferred embodiment, said at least 3 of X₁ to X₆ being independently of each other a positively charged amino acid are X₁, X₂, and X₅. In another preferred embodiment, said at least 3 of X₁ to X₆ being independently of each other a positively charged amino acid are X₁, X₂, and X₆.

In another preferred embodiment, said at least 3 of X₁ to X₆ being independently of each other a positively charged amino acid are X₂, X₃, and X₄. In another preferred embodiment, said at least 3 of X₁ to X₆ being independently of each other a positively charged amino acid are X₂, X₃, and X₅. In another preferred embodiment, said at least 3 of X₁ to X₆ being independently of each other a positively charged amino acid are X₂, X₃, and X₆.

In another preferred embodiment, said at least 3 of X₁ to X₆ being independently of each other a positively charged amino acid are X₃, X₄, and X₅. In another preferred embodiment, said at least 3 of X₁ to X₆ being independently of each other a positively charged amino acid are X₃, X₄, and X₆. In another preferred embodiment, said at least 3 of X₁ to X₆ being independently of each other a positively charged amino acid are X₃, X₄, and X₁.

In another preferred embodiment, said at least 3 of X₁ to X₆ being independently of each other a positively charged amino acid are X₄, X₅, and X₆. In another preferred embodiment, said at least 3 of X₁ to X₆ being independently of each other a positively charged amino acid are X₄, X₅, and X₁. In another preferred embodiment, said at least 3 of X₁ to X₆ being independently of each other a positively charged amino acid are X₄, X₅, and X₂.

In another preferred embodiment, said at least 3 of X₁ to X₆ being independently of each other a positively charged amino acid are X₅, X₆, and X₁. In another preferred embodiment, said at least 3 of X₁ to X₆ being independently of each other a positively charged amino acid are X₅, X₆, and X₂. In another preferred embodiment, said at least 3 of X₁ to X₆ being independently of each other a positively charged amino acid are X₅, X₆, and X₃.

In another preferred embodiment, said at least 3 of X₁ to X₆ being independently of each other a positively charged amino acid are X₁, X₃, and X₅. In another preferred embodiment, said at least 3 of X₁ to X₆ being independently of each other a positively charged amino acid are X₁, X₃, and X₆. In another preferred embodiment, said at least 3 of X₁ to X₆ being independently of each other a positively charged amino acid are X₂, X₄, and X₆. In another preferred embodiment, said at least 3 of X₁ to X₆ being independently of each other a positively charged amino acid are X₁, X₄, and X₆.

In another preferred embodiment, said at least 3 of X₁ to X₆ being independently of each other a positively charged amino acid are X₁, X₂, X₃, and X₄. In another preferred embodiment, said at least 3 of X₁ to X₆ being independently of each other a positively charged amino acid are X₁, X₂, X₃, and X₅. In another preferred embodiment, said at least 3 of X₁ to X₆ being independently of each other a positively charged amino acid are X₁, X₂, X₃, and X₆. In another preferred embodiment, said at least 3 of X₁ to X₆ being independently of each other a positively charged amino acid are X₂, X₃, X₄, and X₅. In another preferred embodiment, said at least 3 of X₁ to X₆ being independently of each other a positively charged amino acid are X₂, X₃, X₄, and X₆. In another preferred embodiment, said at least 3 of X₁ to X₆ being independently of each other a positively charged amino acid are X₃ to X₆. In another preferred embodiment, said at least 3 of X₁ to X₆ being independently of each other a positively charged amino acid are X₂, X₄, X₅, and X₆. In another preferred embodiment, said at least 3 of X₁ to X₆ being independently of each other a positively charged amino acid are X₁, X₄, X₅, and X₆. In another preferred embodiment, said at least 3 of X₁ to X₆ being independently of each other a positively charged amino acid are X₁, X₃, X₅, and X₆. In another preferred embodiment, said at least 3 of X₁ to X₆ being independently of each other a positively charged amino acid are X₁, X₃, X₄, and X₆. In another preferred embodiment, said at least 3 of X₁ to X₆ being independently of each other a positively charged amino acid are X₂, X₃, X₄, and X₆.

In another preferred embodiment, said at least 3 of X₁ to X₆ being independently of each other a positively charged amino acid are X₁₋₅. In another preferred embodiment, said at least 3 of X₁ to X₆ being independently of each other a positively charged amino acid are X₁₋₄ and X₆. In another preferred embodiment, said at least 3 of X₁ to X₆ being independently of each other a positively charged amino acid are X₁₋₃, X₅ and X₆. In another preferred embodiment, said at least 3 of X₁ to X₆ being independently of each other a positively charged amino acid are X₁, X₂, and X4 to X₆. In another preferred embodiment, said at least 3 of X₁ to X₆ being independently of each other a positively charged amino acid are X₁, and X₃₋₆. In another preferred embodiment, said at least 3 of X₁ to X₆ being independently of each other a positively charged amino acid are X₂ to X₆.

In another preferred embodiment, said at least 3 of X₁ to X₆ being independently of each other a positively charged amino acid are X₁ to X₆ are independently of each other positively charged amino acids.

More preferably, said at least 3 of X₁ to X₆ being independently of each other a positively charged amino acid are X₁ to X₆, or X₄ to X₆, or X₁, X₂ and X₅, or X₁, X₂, X₄, and X₅, or X₁, X₃, X₄, and X₆, or X₁ and X₄ to X₆, or X₁ to X₃ and X₆, or X₁ to X₅. More preferably, said at least 3 of X₁ to X₆ being independently of each other a positively charged amino acid are X₁ to X₆, or X₄ to X₆, or X₁, X₂ and X₅, or X₁, X₂, X₄, and X₅, or X₁, X₃, X₄, and X₆, or X₁ and X₄ to X₆, or X₁ to X₃ and X₆, or X₁ to X₅.

In a preferred embodiment, X₄ to X₆ are independently of each other lysine or arginine. In another preferred embodiment, X₁, X₂, and X₄ are independently of each other lysine or arginine. In another preferred embodiment, X₁, X₂, X₄, and X₅ are independently of each other lysine or arginine. In another preferred embodiment, X₁, X₃, X₄, and X₆ are independently of each other lysine or arginine. In another preferred embodiment, X₁, and X₃ to X₆ are independently of each other independently of each other lysine or arginine. In another preferred embodiment, X₁₋₃, X₅ and X₆ are independently of each other lysine or arginine. In another preferred embodiment, X₁₋₅ are independently of each other lysine or arginine. In another preferred embodiment, X₁ to X₆ are independently of each other lysine or arginine.

In a preferred embodiment, each of X₁ to X₆ is independently arginine or lysine. In a preferred embodiment, each of X₁ to X₆ is arginine. In a preferred embodiment, each of X₁ to X₆ is lysine.

In another preferred embodiment, X₁ to X₆ are independently of each other lysine or arginine and X₇ is selected from arginine, lysine or cysteine.

The inventors found out that amino acids at positions X₈ to X₁₂ define pore size of the protein cage formed via self-assembly by the polypeptide of the invention. Larger amino acid at positions X₈ to X₁₂ reduced pore size, while smaller amino acid at these positions increased pore size.

Thus, in a preferred embodiment, said amino acids at positions X₈ to X₁₂ in the polypeptide of the invention are selected such that the surfactant composition can be loaded into and released from the protein cage and cargo into/from the lipoprotein cage, without disassembly. In another preferred embodiment, said amino acids at positions X₈ to X₁₂ in the polypeptide of the invention are selected such that cargo can be loaded extracellularly into the protein cage and released intracellularly, preferably, into the cytoplasm of a cell, without disassembly.

Preferably, said cargo is small cargo. Small cargo has preferably a size of 1000 Da or below. In a preferred embodiment, a size of 1000 Da or below means that said cargo has a size of 1000 Da or lower, preferably 800 Da or lower, more preferably 600 Da or lower, again more preferably 500 Da or lower, again more preferably 400 Da or lower, again more preferably 300 Da or lower, again more preferably 200 Da or lower, again more preferably 100 Da or lower.

Preferably, said cargo is hydrophobic cargo, more preferably non-polar cargo. Preferably, said cargo has a low solubility in aqueous media. Preferably, said small cargo having a size of 1000 Da or below has a low solubility in aqueous media. More preferably said cargo of low solubility is included in Class II or Class IV of the Biopharmaceutics Classification System (BCS). Again more preferably, said low soluble cargo has a lower solubility as a highly soluble cargo for which the highest strength dose is soluble in 250 mL or less of aqueous media over the pH range of 1.0-7.5, more preferably, 1.0-6.8, at 37 ± 1°C.

In a preferred embodiment, said X₈ is glycine or a conservative substitution thereof. Preferably, said conservative substitution of glycine is selected from the group consisting of, alanine, leucine, valine, tert-leucine, homoleucine, isoleucine, alloisoleucine 2-aminobutyric acid, diethylalanine, norleucine, and norvaline. More preferably, said conservative substitution of glycine is selected from the group consisting of, alanine, leucine, valine, tert-leucine, homoleucine, isoleucine, alloisoleucine 2-aminobutyric acid, diethylalanine, norleucine, and norvaline. In a further preferred embodiment, said X₈ is selected from the group consisting of glycine, alanine, leucine, and valine. In a preferred embodiment, said X₈ is glycine.

In another preferred embodiment, said X₉ and X₁₂ are independently of each other glutamine or a conservative substitution thereof. Preferably said conservative substitution of glutamine is selected from the group consisting of asparagine, beta-hydroxyasparagine, 3-methyl-L-glutamine, (2S,4S)-2,5-diamino-4-hydroxy-5-oxopentanoic acid, and n-methyl-asparagine. In another preferred embodiment, said X₉ and X₁₂ are independently of each other glutamine or asparagine.

In another preferred embodiment, said X₉ and X₁₂ are both glutamine. In another preferred embodiment, said X₉ and X₁₂ are both asparagine. In another preferred embodiment, said X₉ or X₁₂ is glutamine. In another preferred embodiment, said X₉ is glutamine. In another preferred embodiment, said X₁₂ is glutamine.

In another preferred embodiment, said X₁₀ is glutamate or a conservative substitution thereof. Preferably, said conservative substitution of glutamate is selected from the group consisting of glutamate, aspartate, (2S,4R)-4-methylglutamate, (3S)-3-methyl-L-glutamic acid, (3R)-3-methyl-L-glutamic acid, 5-O-methyl-glutamic acid, 4-hydroxy-glutamic acid, 6-carboxylysine, beta-hydroxyaspartic acid, 2-amino-propanedioic acid, 3,3-dimethyl aspartic acid, 2-aminoadipic acid and 3-methyl-aspartic acid. In a further preferred embodiment, said X₁₀ is glutamate or aspartate. In another further preferred embodiment, said X₁₀ is glutamate.

In another preferred embodiment, said X₁₁ is selected from the group consisting of serine or a conservative substitution thereof. Preferably said conservative substitution of serine is selected from the group consisting of cysteine, methionine, homocysteine, selenocysteine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. More preferably said conservative substitution of serine is selected from the group consisting of cysteine, homocysteine, selenocysteine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. Again more preferably said conservative substitution of serine is selected from the group consisting of hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. Again more preferably said conservative substitution of serine is selected from the group consisting of homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine.

In a further preferred embodiment, said X₁₁ is selected from the group consisting of serine, homoserine, and threonine. In another further preferred embodiment, said X₁₁ is serine.

In a preferred embodiment, said X₈ is selected from the group consisting of glycine, alanine, leucine, valine, tert-leucine, homoleucine, isoleucine, alloisoleucine 2-aminobutyric acid, diethylalanine, norleucine, and norvaline; X₉ and X₁₂ are independently of each other selected from the group consisting of glutamine, asparagine, beta-hydroxyasparagine, 3-methyl-L-glutamine, (2S,4S)-2,5-diamino-4-hydroxy-5-oxopentanoic acid, and n-methyl-asparagine; X₁₀ is selected from the group consisting of glutamate, aspartate (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid and 3-methyl-aspartic acid; and X₁₁ is selected from the group consisting of serine, homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine.

In a further preferred embodiment, said X₈ is selected from the group consisting of glycine, alanine, leucine, and valine; X₉ and X₁₂ are independently of each other glutamine or asparagine; X₁₀ is glutamate or aspartate; and X₁₁ is selected from the group consisting of serine, threonine, and homoserine.

In a further preferred embodiment, said X₈ is glycine, X₉ and X₁₂ are is glutamine, X₁₀ is glutamate and X₁₁ is serine.

In a preferred embodiment, each of X₈, X₉, X₁₀, X₁₁ and X₁₂ is independently selected from the group consisting of glycine, alanine, leucine, valine, tert-leucine, homoleucine, isoleucine, alloisoleucine 2-aminobutyric acid, diethylalanine, norleucine, norvaline; glutamine, asparagine, beta-hydroxyasparagine, 3-methyl-L-glutamine, (2S,4S)-2,5-diamino-4-hydroxy-5-oxopentanoic acid, n-methyl-asparagine; glutamate, aspartate (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid, 3-methyl-aspartic acid; serine, homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine.

In a further preferred embodiment, each of X₈, X₉, X₁₀, X₁₁ and X₁₂ is independently selected from the group consisting of glycine, alanine, leucine, valine; glutamate, aspartate; glutamine, asparagine; serine, threonine, and homoserine.

In a further preferred embodiment, each of X₈, X₉, X₁₀, X₁₁ and X₁₂ is independently selected from the group consisting of serine, glycine, glutamine, and glutamate.

In a preferred embodiment, in sequence I X₁₋₆ are lysine or arginine, X₇ is selected from arginine, lysine or cysteine and each of X₈, X₉, X₁₀, X₁₁ and X₁₂ is independently selected from the group consisting of glycine, alanine, leucine, valine, tert-leucine, homoleucine, isoleucine, alloisoleucine 2-aminobutyric acid, diethylalanine, norleucine, norvaline; glutamine, asparagine, beta-hydroxyasparagine, 3-methyl-L-glutamine, (2S,4S)-2,5-diamino-4-hydroxy-5-oxopentanoic acid, n-methyl-asparagine; glutamate, aspartate (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid, 3-methyl-aspartic acid; serine, homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine. In a preferred embodiment, preferably the C-terminus of the amino acid sequence I, and wherein in sequence I X₁₋₆ are lysine or arginine, X₇ is selected from arginine, lysine or cysteine and each of X₈, X₉, X₁₀, X₁₁ and X₁₂ is independently selected from the group consisting of glycine, alanine, leucine, valine, tert-leucine, homoleucine, isoleucine, alloisoleucine 2-aminobutyric acid, diethylalanine, norleucine, norvaline; glutamine, asparagine, beta-hydroxyasparagine, 3-methyl-L-glutamine, (2S,4S)-2,5-diamino-4-hydroxy-5-oxopentanoic acid, n-methyl-asparagine; glutamate, aspartate (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid, 3-methyl-aspartic acid; serine, homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine.

In another preferred embodiment, at least three of X₁₋₆ are independently of each other lysine or arginine, X₇ is selected from arginine, lysine or cysteine, X₈ is selected from the group consisting of glycine, alanine, leucine, and valine; X₉ and X₁₂ are independently of each other glutamine or asparagine; X₁₀ is glutamate or aspartate; and X₁₁ is selected from the group consisting of serine, threonine, and homoserine. In another preferred embodiment, at least three of X₁₋₆ are independently of each other lysine or arginine, X₈ is glycine, X₉ and X₁₂ are independently of each other glutamine, X₁₀ is glutamate and X₁₁ is serine. In another preferred embodiment, X₁₋₆ are lysine or arginine, X₇ is selected from arginine, lysine or cysteine and X₈ is glycine, X₉ and X₁₂ are independently of each other glutamine, X₁₀ is glutamate and X₁₁ is serine. In another preferred embodiment, X₁₋₆ are lysine or arginine, X₇ is selected from arginine, lysine or cysteine and X₈ is glycine, X₉ and X₁₂ are independently of each other is glutamine, X₁₀ is glutamate and X₁₁ is serine.

In one embodiment, at least 3 of said X₁₋₆ are independently of each other lysine or arginine, said X₇ is selected from arginine, lysine or cysteine, X₈ is glycine, X₉ and X₁₂ are independently of each other glutamine, X₁₀ is glutamate and X₁₁ is serine.

In another preferred embodiment, X₁ to X₆ are independently of each other lysine or arginine, X₇ is selected from arginine, lysine or cysteine, X₈ is glycine, X₉ and X₁₂ are independently of each other glutamine, X₁₀ is glutamate and X₁₁ is serine and said polypeptide.

In a preferred embodiment, in sequence I X₁₋₆ are lysine or arginine, X₇ is selected from arginine, lysine or cysteine and X₈ is glycine, X₉ and X₁₂ are independently of each other glutamine, X₁₀ is glutamate and X₁₁ is serine. In a preferred embodiment, in sequence I X1-6 are lysine or arginine, X7 is selected from arginine, lysine or cysteine and X8 is glycine, X9 and X12 are independently of each other glutamine, X10 is glutamate and X11 is serine; and said protein cage or said complex of the invention comprising said polypeptide of the invention is capable of endosomal escape.

When the polypeptide of the invention forms a protein cage by self-assembly, amino acids X₁₃ to X₂₉ are exposed to the external surface of the protein cage and are therefore most prone to tolerate mutations while still keeping ability to self-assemble into a cage-like protein cage.

Thus, in a preferred embodiment, amino acids X₁₃ to X₂₉ exposed to the external surface of the protein cage are any amino acid.

In a preferred embodiment, X₁₃, X₁₇ and X₁₉ are independently of each other serine or a conservative substitution thereof. Preferably said conservative substitution of serine is selected from the group consisting of cysteine, methionine, homocysteine, selenocysteine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. More preferably said conservative substitution of serine is selected from the group consisting of cysteine, homocysteine, selenocysteine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. Again more preferably said conservative substitution of serine is selected from the group consisting of hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, homoserine, 3-hydroxy-L-valine, 4,5-dihydroxy-isoleucine, 6-hydroxy-L-norleucine, S-(2-hydroxyethyl)-L-cysteine, phosphoserine, 4-hydroxy-L-threonine, threonine, and phosphothreonine. Again more preferably said conservative substitution of serine is selected from the group consisting of homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine.

In another preferred embodiment, X₁₃, X₁₇ and X₁₉, are independently of each other selected from the group consisting of serine, homoserine, and threonine. In another preferred embodiment, X₁₃, X₁₇ and X₁₉, are independently of each other serine.

In another preferred embodiment, X₁₄, X₁₆ and X₂₄ are independently of each other asparagine or a conservative substitution thereof. Preferably, said conservative substitution of asparagine is selected from the group consisting of glutamine, beta-hydroxyasparagine, 3-methyl-L-glutamine, (2S,4S)-2,5-diamino-4-hydroxy-5-oxopentanoic acid, and n-methyl-asparagine

In another preferred embodiment, X₁₄, X₁₆ and X₂₄ are independently of each other asparagine. In another preferred embodiment, X₁₄, X₁₆ and X₂₄ are independently of each other asparagine or glutamine.

In another preferred embodiment, X₁₅, X₂₀ X₂₆ and X₂₈ are independently of each other aspartate, glutamate or a conservative substitution thereof. Preferably said conservative substitution of aspartate or glutamate is selected from the group consisting of (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid and 3-methyl-aspartic acid.

In another preferred embodiment, X₁₅ and X₂₀ are independently of each other aspartate. In another preferred embodiment, X₂₆ and X₂₈ are independently of each other glutamate.

In another preferred embodiment, X₁₅, X₂₀ X₂₆ and X₂₈ are independently of each other aspartate or glutamate. In another preferred embodiment, X₁₅, X₂₀ X₂₆ and X₂₈ are independently of each other selected from the group consisting of glutamate, aspartate, 2S,4R-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid and 3-methyl-aspartic acid.

In another preferred embodiment, X₁₈ and X₂₉ are independently of each other leucine or a conservative substitution thereof. Preferably said conservative substitution of leucine is selected from the group consisting of glycine, alanine, valine, tert-leucine, homoleucine, isoleucine, alloisoleucine 2-aminobutyric acid, diethylalanine, norleucine, and norvaline.

In another preferred embodiment, X₁₈ and X₂₉ are independently of each other leucine. In a preferred embodiment, said X₁₈ and X₂₉ are independently of each other selected from the group consisting of glycine, alanine, leucine, and valine. In a preferred embodiment, said X₁₈, and X₂₉ are independently of each other selected from the group consisting of glycine, alanine, leucine, valine, tert-leucine, homoleucine, isoleucine, alloisoleucine 2-aminobutyric acid, diethylalanine, norleucine, and norvaline.

In another preferred embodiment, X₂₁, X₂₂, X₂₃, X₂₅ and X₂₇ are independently of each other a positively charged amino acid.

In another preferred embodiment, X₂₁, X₂₂, X₂₃, X₂₅ and X₂₇ are independently of each other arginine, lysine or a conservative substitution thereof. Preferably said conservative substitution of arginine or leucine is selected from the group consisting of histidine, 5-methyl-arginine, gamma-hydroxy arginine, 2-amino-4-guanidinobutryric acid, 2-amino-3-guanidinopropionic acid, canavanine, homoarginine, diaminobutyric acid, 2,3-diaminopropanoic acid, (2S)-2,8-diaminooctanoic acid, ornithine and thialysine. More preferably said conservative substitution of arginine or leucine is selected from the group consisting of 5-methyl-arginine, gamma-hydroxy arginine, 2-amino-4-guanidinobutryric acid, 2-amino-3-guanidinopropionic acid, canavanine, homoarginine, diaminobutyric acid, 2,3-diaminopropanoic acid, (2S)-2,8-diaminooctanoic acid, ornithine and thialysine.

In another preferred embodiment, X₂₁, X₂₂, X₂₃, X₂₅ and X₂₇ are independently of each other histidine, arginine or lysine. In another preferred embodiment, X₂₁, X₂₂, X₂₃, X₂₅ and X₂₇ are independently of each other arginine or lysine. In another preferred embodiment, X₂₁, X₂₂, X₂₃, X₂₅ and X₂₇ are independently of each other is arginine. In another preferred embodiment, X₂₁, X₂₂, X₂₃, X₂₅ and X₂₇ are independently of each other ₆ is lysine.

In another preferred embodiment, X₂₁, X₂₂, X₂₅ and X₂₇ are independently of each other arginine. In another preferred embodiment, X₂₃ is lysine. In another preferred embodiment, X₂₁, X₂₂, X₂₃, X₂₅ and X₂₇ are independently of each other selected from the group consisting of arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine and thialysine.

In another preferred embodiment, X₁₃, X₁₇ and X₁₉, are independently of each other serine; X₁₄, X₁₆ and X₂₄ are independently of each other asparagine; X₁₅ and X₂₀ are independently of each other aspartate; X₁₈ and X₂₉ are independently of each other leucine; X₂₆ and X₂₈ are independently of each other glutamate; X₂₁, X₂₂, X₂₅ and X₂₇ are independently of each other arginine; and X₂₃ is lysine.

In another preferred embodiment, X₁₃, X₁₇ and X₁₉, are independently of each other selected from the group consisting of serine, homoserine, and threonine; X₁₄, X₁₆ and X₂₄ are independently of each other asparagine or glutamine; X₁₅, X₂₀ X₂₆ and X₂₈ are independently of each other aspartate or glutamate; said X₁₈ and X₂₉ are independently of each other selected from the group consisting of glycine, alanine, leucine, and valine; X₂₁, X₂₂, X₂₃, X₂₅ and X₂₇ are independently of each other arginine or lysine.

In another preferred embodiment, X₁₃, X₁₇, and X₁₉, are independently of each other selected from the group consisting of serine, homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine;
X₁₄, X₁₆, and X₂₄ are independently of each other selected from the group consisting of glutamine, asparagine, beta-hydroxyasparagine, 3-methyl-L-glutamine, (2S,4S)-2,5-diamino-4-hydroxy-5-oxopentanoic acid, and n-methyl-asparagine;
X₁₅, X₂₀ X₂₆ and X₂₈ are independently of each other selected from the group consisting of glutamate, aspartate, (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid and 3-methyl-aspartic acid;
X₁₈, and X₂₉ are independently of each other selected from the group consisting of glycine, alanine, leucine, valine, tert-leucine, homoleucine, isoleucine, alloisoleucine 2-aminobutyric acid, diethylalanine, norleucine, and norvaline; and
X₂₁, X₂₂, X₂₃, X₂₅ and X₂₇ are independently of each other selected from the group consisting of arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine and thialysine.

In another preferred embodiment, X₈ is glycine or a conservative substitution thereof, wherein preferably said conservative substitution of glycine is selected from the group consisting of alanine, leucine, valine, tert-leucine, homoleucine, isoleucine, alloisoleucine 2-aminobutyric acid, diethylalanine, norleucine, and norvaline;
X₉ and X₁₂ are independently of each other glutamine or a conservative substitution thereof, wherein preferably said conservative substitution of glutamine is selected from the group consisting of asparagine, beta-hydroxyasparagine, 3-methyl-L-glutamine, (2S,4S)-2,5-diamino-4-hydroxy-5-oxopentanoic acid, and n-methyl-asparagine;
X₁₀ is glutamate or a conservative substitution thereof, wherein preferably said conservative substitution of glutamate is selected from the group consisting of aspartate (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid, and 3-methyl-aspartic acid; and
X₁₁ is serine or a conservative substitution thereof, wherein preferably said conservative substitution of serine is selected from the group consisting of homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine; more preferably X₈ is glycine, X₉ and X₁₂ are independently of each other glutamine or asparagine, X₁₀ is glutamate or aspartate, and X₁₁ is serine.

In another preferred embodiment, X₁₃, X₁₇, and X₁₉ are independently of each other serine or a conservative substitution thereof, wherein preferably said conservative substitution of serine is selected from the group consisting of homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine;
X₁₄, X₁₆, and X₂₄ are independently of each other asparagine, glutamine or a conservative substitution thereof, wherein preferably said conservative substitution of asparagine or glutamine is selected from the group consisting of beta-hydroxyasparagine, 3-methyl-L-glutamine, (2S,4S)-2,5-diamino-4-hydroxy-5-oxopentanoic acid, and n-methyl-asparagine;
X₁₅, X₂₀ X₂₆ and X₂₈ are independently of each other aspartate, glutamate or a conservative substitution thereof, wherein preferably said conservative substitution of aspartate or glutamate is selected from the group consisting of (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid and 3-methyl-aspartic acid;
X₁₈ and X₂₉ are independently of each other leucine or a conservative substitution thereof, wherein preferably said conservative substitution of leucine is selected from the group consisting of glycine, alanine, valine, tert-leucine, homoleucine, isoleucine, alloisoleucine 2-aminobutyric acid, diethylalanine, norleucine, and norvaline; and
X₂₁, X₂₂, X₂₃, X₂₅ and X₂₇ are independently of each other arginine, lysine or a conservative substitution thereof, wherein preferably said conservative substitution of arginine or leucine is selected from the group consisting of histidine, 5-methyl-arginine, gamma-hydroxy arginine, 2-amino-4-guanidinobutryric acid, 2-amino-3-guanidinopropionic acid, canavanine, homoarginine, diaminobutyric acid, 2,3-diaminopropanoic acid, (2S)-2,8-diaminooctanoic acid, ornithine and thialysine.

In a preferred embodiment, X8 is glycine or a conservative substitution thereof; X9 and X12 are independently of each other glutamine, asparagine or a conservative substitution thereof; X10 is glutamate, aspartate or a conservative substitution thereof; X11 is serine or a conservative substitution thereof; X13, X17, and X19 are independently of each other serine or a conservative substitution thereof; X14, X16, and X24 are independently of each other asparagine, glutamine or a conservative substitution thereof; X15, X20 X26 and X28 are independently of each other aspartate, glutamate or a conservative substitution thereof; X18 and X29 are independently of each other leucine or a conservative substitution thereof; and X21, X22, X23, X25 and X27 are independently of each other arginine, lysine or a conservative substitution thereof.

In another preferred embodiment, said positively charged amino acid of said at least 3 of X₁ to X₆ is independently of each other selected from the group consisting of arginine, 5-methyl-arginine, gamma-hydroxy arginine, 2-amino-4-guanidinobutyric acid, 2-amino-3-guanidinopropionic acid, canavanine, homoarginine, lysine, diaminobutyric acid, 2,3-diaminopropanoic acid, (2S)-2,8-diaminooctanoic acid, ornithine, and thialysine;
X₇ is cysteine, a conservative substitution thereof or a positively charged amino acid; preferably X₇ is selected from the group consisting of homocysteine, cysteine, selenocysteine, arginine, 5-methyl-arginine, gamma-hydroxy arginine, 2-amino-4-guanidinobutyric acid, 2-amino-3-guanidinopropionic acid, canavanine, homoarginine, lysine, diaminobutyric acid, 2,3-diaminopropanoic acid, (2S)-2,8-diaminooctanoic acid, ornithine, thialysine and serine;
X₈ is glycine or a conservative substitution thereof selected from the group consisting of, alanine, leucine, valine, tert-leucine, homoleucine, isoleucine, alloisoleucine 2-aminobutyric acid, diethylalanine, norleucine, and norvaline;
X₉ and X₁₂ are independently of each other glutamine or a conservative substitution thereof selected from the group consisting of asparagine, beta-hydroxyasparagine, 3-methyl-L-glutamine, (2S,4S)-2,5-diamino-4-hydroxy-5-oxopentanoic acid, and n-methyl-asparagine;
X₁₀ is glutamate or a conservative substitution thereof selected from the group consisting of aspartate (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid, and 3-methyl-aspartic acid; and
X₁₁ is serine or a conservative substitution thereof selected from the group consisting of homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine;
X₁₃, X₁₇, and X₁₉, are independently of each other selected from the group consisting of serine, homoserine, threonine, 4-hydroxy-L-threonine, 6-hydroxy-L-norleucine, 4,5-dihydroxy-isoleucine, 3-hydroxy-L-valine, hydroxynorvaline, 2-amino-5-hydroxypentanoic acid, allo-threonine, 3,3-dihydroxy-alanine, 4-hydroxy-L-isoleucine, (2S,3R)-2-amino-3-hydroxy-4-methylpentanoic acid, beta-hydroxyleucine, and allo-threonine;
X₁₄, X₁₆, and X₂₄ are independently of each other selected from the group consisting of glutamine, asparagine, beta-hydroxyasparagine, 3-methyl-L-glutamine, (2S,4S)-2,5-diamino-4-hydroxy-5-oxopentanoic acid, and n-methyl-asparagine;
X₁₅, X₂₀ X₂₆ and X₂₈ are independently of each other selected from the group consisting of glutamate, aspartate, (2S,4R)-4-methylglutamate, (3S)-3-methyl-L-glutamic acid, (3R)-3-methyl-L-glutamic acid, 5-O-methyl-glutamic acid, 4-hydroxy-glutamic-acid, 6-carboxylysine, beta-hydroxyaspartic acid, 2-amino-propanedioic acid, 3,3-dimethyl aspartic acid, 2-aminoadipic acid and 3-methyl-aspartic acid;
X₁₈, and X₂₉ are independently of each other selected from the group consisting of glycine, alanine, leucine, valine, tert-leucine, homoleucine, isoleucine, alloisoleucine 2-aminobutyric acid, diethylalanine, norleucine, and norvaline; and
X₂₁, X₂₂, X₂₃, X₂₅ and X₂₇ are independently of each other selected from the group consisting of arginine, 5-methyl-arginine, gamma-hydroxy arginine, 2-amino-4-guanidinobutryric acid, 2-amino-3-guanidinopropionic acid, canavanine, homoarginine, lysine, diaminobutyric acid, 2,3-diaminopropanoic acid, (2S)-2,8-diaminooctanoic acid, ornithine and thialysine.

In another preferred embodiment, said positively charged amino acid of said at least 3 of X₁ to X₆ is independently of each other arginine or lysine, X₇ is selected from the group consisting of homocysteine, cysteine, arginine, and lysine, X₈ is selected from the group consisting of glycine, alanine, leucine, and valine, X₉ and X₁₂ are independently of each other glutamine or asparagine, X₁₀ is glutamate or aspartate, X₁₁, X₁₃, X₁₇, and X₁₉ are independently of each other selected from the group consisting of serine, threonine, and homoserine, X₁₄, X₁₆, and X₂₄ are independently of each other asparagine or glutamine, X₁₅, X₂₀ X₂₆ and X₂₈ are independently of each other aspartate or glutamate, X₁₈ and X₂₉ are independently of each other selected from the group consisting of leucine, glycine, alanine, and valine, and X₂₁, X₂₂, X₂₃, X₂₅ and X₂₇ are independently of each other arginine or lysine.

In another preferred embodiment, said positively charged amino acid of said at least 3 of X₁ to X₆ is independently of each other arginine or lysine, X₇ is selected from the group consisting of homocysteine, cysteine, arginine, and lysine, X₈ is glycine, X₉ and X₁₂ are independently of each other glutamine or asparagine, X₁₀ is glutamate or aspartate, X₁₁ is serine, X₁₃, X₁₇, and X₁₉ are independently of each other serine, X₁₄, X₁₆, and X₂₄ are independently of each other asparagine, X₁₅, X₂₀ X₂₆ and X₂₈ are independently of each other aspartate or glutamate, X₁₈ and X₂₉ are independently of each other leucine, and X₂₁, X₂₂, X₂₃, X₂₅ and X₂₇ are independently of each other arginine or lysine.

In another preferred embodiment, at least three of X₁₋₆ are independently of each other lysine or arginine, X₇ is selected from arginine, lysine or cysteine, X₈ is selected from the group consisting of glycine, alanine, leucine, and valine; X₉ and X₁₂ are independently of each other glutamine or asparagine; X₁₀ is glutamate or aspartate; and X₁₁ is selected from the group consisting of serine, threonine, and homoserine; X₁₈ and X₂₉ are independently of each other leucine; X₁₅ and X₂₀ are independently of each other aspartate; X₂₆ and X₂₈ are independently of each other glutamate; X₁₄, X₁₆ and X₂₄ are independently of each other asparagine; X₁₃, X₁₇ and X₁₉, are independently of each other serine; X₂₁, X₂₂, X₂₅ and X₂₇ are independently of each other arginine; and X₂₃ is lysine.

In another preferred embodiment, at least three of X₁₋₆ are independently of each other lysine or arginine, X₇ is selected from arginine, lysine or cysteine, X₈ is selected from the group consisting of glycine, alanine, leucine, and valine; X₉ and X₁₂ are independently of each other glutamine or asparagine; X₁₀ is glutamate or aspartate; and X₁₁ is selected from the group consisting of serine, threonine, and homoserine; X₁₃, X₁₇ and X₁₉, are independently of each other selected from the group consisting of serine, homoserine, and threonine; X₁₄, X₁₆ and X₂₄ are independently of each other asparagine or glutamine; X₁₅, X₂₀ X₂₆ and X₂₈ are independently of each other aspartate or glutamate; said X₁₈ and X₂₉ are independently of each other selected from the group consisting of glycine, alanine, leucine, and valine; X₂₁, X₂₂, X₂₃, X₂₅ and X₂₇ are independently of each other arginine or lysine.

In another preferred embodiment, X₁₋₆ are independently of each other lysine or arginine, X₇ is selected from arginine, lysine or cysteine, X₈ is selected from the group consisting of glycine, alanine, leucine, and valine; X₉ and X₁₂ are independently of each other glutamine or asparagine; X₁₀ is glutamate or aspartate; and X₁₁ is selected from the group consisting of serine, threonine, and homoserine; X₁₃, X₁₇ and X₁₉, are independently of each other selected from the group consisting of serine, homoserine, and threonine; X₁₄, X₁₆ and X₂₄ are independently of each other asparagine or glutamine; X₁₅, X₂₀ X₂₆ and X₂₈ are independently of each other aspartate or glutamate; said X₁₈ and X₂₉ are independently of each other selected from the group consisting of glycine, alanine, leucine, and valine; X₂₁, X₂₂, X₂₃, X₂₅ and X₂₇ are independently of each other arginine or lysine.

In another preferred embodiment, X₁₋₆ are independently of each other lysine or arginine, X₇ is selected from arginine, lysine or cysteine, X₈ is selected from the group consisting of glycine, alanine, leucine, and valine; X₉ and X₁₂ are independently of each other glutamine or asparagine; X₁₀ is glutamate or aspartate; and X₁₁ is selected from the group consisting of serine, threonine, and homoserine; X₁₃, X₁₇ and X₁₉, are independently of each other serine; X₁₄, X₁₆ and X₂₄ are independently of each other asparagine; X₁₅ and X₂₀ are independently of each other aspartate; X₁₈ and X₂₉ are independently of each other leucine; X₂₆ and X₂₈ are independently of each other glutamate; X₂₁, X₂₂, X₂₅ and X₂₇ are independently of each other arginine; and X₂₃ is lysine.

In a preferred embodiment, said amino acid sequence I is a sequence selected from the group consisting of SEQ ID NO: 2 to 16 and 20 to 27. In a preferred embodiment, said polypeptide of the invention consists of a sequence selected from the group consisting of SEQ ID NO: 2 to 16 and 20 to 27.

In a preferred embodiment, said amino acid sequence I is a sequence selected from the group consisting of SEQ ID NO: 2 to 5 and SEQ ID NO: 10 to 16. In a preferred embodiment, said polypeptide of the invention consists of a sequence selected from the group consisting of SEQ ID NO: 2 to 5 and SEQ ID NO: 10 to 16.

In a preferred embodiment, said amino acid sequence I is a sequence selected from the group consisting of SEQ ID NO: 2 to 5 and SEQ ID NO: 10 to 16 and 22. In a preferred embodiment, said polypeptide of the invention consists of a sequence selected from the group consisting of SEQ ID NO: 2 to 5, SEQ ID NO: 10 to 16, and SEQ ID NO: 20 to 22.

In a preferred embodiment, said amino acid sequence I is a sequence selected from the group consisting of SEQ ID NO: 2 to 5 and SEQ ID NO: 10 to 16, 20 and 21. In a preferred embodiment, said polypeptide of the invention consists of a sequence selected from the group consisting of SEQ ID NO: 2 to 5 and SEQ ID NO: 10 to 16, 20 and 21. In a preferred embodiment, said amino acid sequence I is a sequence selected from the group consisting of SEQ ID NO: 2 to 5 and SEQ ID NO: 10 to 16 and 22. In a preferred embodiment, said polypeptide of the invention consists of a sequence selected from the group consisting of SEQ ID NO: 2 to 5 and SEQ ID NO: 10 to 16, 20, 21 and 22.

In a preferred embodiment, said amino acid sequence I is SEQ ID NO: 2. In a preferred embodiment, said amino acid sequence I is SEQ ID NO: 3. In another preferred embodiment, said amino acid sequence Iis SEQ ID NO: 4. In another preferred embodiment, said amino acid sequence I is SEQ ID NO: 5. In another preferred embodiment, said amino acid sequence I is SEQ ID NO: 10. In another preferred embodiment, said amino acid sequence I is SEQ ID NO: 11. In another preferred embodiment, said amino acid sequence I is SEQ ID NO: 12. In another preferred embodiment, said amino acid sequence I is SEQ ID NO: 13. In a preferred embodiment, said amino acid sequence I is SEQ ID NO: 14. In another preferred embodiment, said amino acid sequence I is SEQ ID NO: 15. In another preferred embodiment, said amino acid sequence Iis SEQ ID NO: 16. In another preferred embodiment, said amino acid sequence I is SEQ ID NO: 20. In another preferred embodiment, said amino acid sequence I is SEQ ID NO: 21. In another preferred embodiment, said amino acid sequence I is SEQ ID NO: 22.

In a preferred embodiment, said polypeptide of the invention consists of a sequence selected from the group consisting of SEQ ID NO: 2. In another preferred embodiment, said polypeptide of the invention consists of a sequence selected from the group consisting of SEQ ID NO: 3. In another preferred embodiment, said polypeptide of the invention consists of a sequence selected from the group consisting of SEQ ID NO: 4. In another preferred embodiment, said polypeptide of the invention consists of a sequence selected from the group consisting of SEQ ID NO: 5. In another preferred embodiment, said polypeptide of the invention consists of a sequence selected from the group consisting of SEQ ID NO: 10. In another preferred embodiment, said polypeptide of the invention consists of a sequence selected from the group consisting of SEQ ID NO: 11. In another preferred embodiment, said polypeptide of the invention consists of a sequence selected from the group consisting of SEQ ID NO: 12. In another preferred embodiment, said polypeptide of the invention consists of a sequence selected from the group consisting of SEQ ID NO: 13. In another preferred embodiment, said polypeptide of the invention consists of a sequence selected from the group consisting of SEQ ID NO: 14. In another preferred embodiment, said polypeptide of the invention consists of a sequence selected from the group consisting of SEQ ID NO: 15. In another preferred embodiment, said polypeptide of the invention consists of a sequence selected from the group consisting of SEQ ID NO: 16. In another preferred embodiment, said polypeptide of the invention consists of a sequence selected from the group consisting of SEQ ID NO: 20. In another preferred embodiment, said polypeptide of the invention consists of a sequence selected from the group consisting of SEQ ID NO: 21. In another preferred embodiment, said polypeptide of the invention consists of a sequence selected from the group consisting of SEQ ID NO: 22. In a preferred embodiment, said polypeptide of the invention consists of a sequence selected from the group consisting of SEQ ID NO: 3, wherein said polypeptide of the invention comprises a histidine tag (His tag) consisting of 3 or more consecutive histidines, wherein said His tag is attached to the C- or N-terminus, preferably the C-terminus of the amino acid sequence I.

In a preferred embodiment, said amino acid sequence I is a sequence selected from the group consisting of SEQ ID NO: 2 to 16 and 20 to 27, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1.

In a preferred embodiment, said amino acid sequence I is a sequence selected from the group consisting of SEQ ID NO: 2 to 5 and SEQ ID NO: 10 to 16, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1.

In a preferred embodiment, said amino acid sequence I is a sequence selected from the group consisting of SEQ ID NO: 2 to 5 and SEQ ID NO: 10 to 16 and 22, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1.

In a preferred embodiment, said amino acid sequence I is a sequence selected from the group consisting of SEQ ID NO: 2 to 5 and SEQ ID NO: 10 to 16, 20 and 21, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1.

In a preferred embodiment, said amino acid sequence I is a sequence selected from the group consisting of SEQ ID NO: 2 to 5 and SEQ ID NO: 10 to 16 and 22, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1.

In a preferred embodiment, said amino acid sequence I is SEQ ID NO: 2, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In a preferred embodiment, said amino acid sequence I is SEQ ID NO: 3, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 4, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 5, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 10, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 11, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 12, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 13, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In a preferred embodiment, said amino acid sequence I is SEQ ID NO: 14, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 15, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 16, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 20, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 21, and said surfactant composition comprises, preferably consists of SDS and CS, and wherein said molar ratio of said SDS to CS is equal or between 4 to 1 and 2 to 1, preferably wherein said molar ratio of said SDS to CS is 3 to 1. In another preferred embodiment, said amino acid sequence I is SEQ ID NO: 22, and said surfactant composition comprises, preferably consists of, SDS and CS, and wherein said molar ratio of said SDS to CS is equal or between 4 to 1 and 2 to 1, preferably wherein said molar ratio of said SDS to CS is 3 to 1.

In a preferred embodiment, said polypeptide of the invention comprises a tag, i.e. a peptide or non-peptide tag, preferably a peptide tag (i.e. a functional amino acid sequence). In a preferred embodiment, said tag is located at the C- or N-terminal end of the polypeptide of the invention. In a preferred embodiment, said tag is a non-peptide tag, preferably polyethylene glycol (PEG). Preferably, said PEG is coupled via the amino acid serine or cysteine to said amino acid sequence I. PEG coupled to the polypeptide of the invention will increase stability and reduce immunogenicity.

In a preferred embodiment, said polypeptide of the invention comprises a tag, preferably a peptide tag, wherein said tag is located at the C- or N-terminal end, preferably at the C-terminal end, of said polypeptide, preferably of said amino acid sequence I, and wherein said tag is preferably fused to said C- or N-terminal end of said polypeptide, preferably of said amino acid sequence I.

In a preferred embodiment, said amino acid sequence I is a sequence selected from the group consisting of SEQ ID NO: 2 to 16 and SEQ ID NO: 20 to 27, and wherein said polypeptide of the invention comprises a tag, preferably a peptide tag, wherein said tag is located at the C- or N-terminal end, preferably at the C-terminal end, of said polypeptide, preferably of said amino acid sequence I, and wherein said tag is preferably fused to said C- or N-terminal end of said polypeptide, preferably of said amino acid sequence I.

In a preferred embodiment, said amino acid sequence I is a sequence selected from the group consisting of SEQ ID NO: 2 to 5 and SEQ ID NO: 10 to 16, and wherein said polypeptide of the invention comprises a tag, preferably a peptide tag, wherein said tag is located at the Cor N-terminal end, preferably at the C-terminal end, of said polypeptide, preferably of said amino acid sequence I, and wherein said tag is preferably fused to said C- or N-terminal end of said polypeptide, preferably of said amino acid sequence I.

In a preferred embodiment, said amino acid sequence I is a sequence selected from the group consisting of SEQ ID NO: 2 to 5 and SEQ ID NO: 10 to 16 and SEQ ID NO: 20 to 22, and wherein said polypeptide of the invention comprises a tag, preferably a peptide tag, wherein said tag is located at the C- or N-terminal end, preferably at the C-terminal end, of said polypeptide, preferably of said amino acid sequence I, and wherein said tag is preferably fused to said C- or N-terminal end of said polypeptide, preferably of said amino acid sequence I.

In a preferred embodiment, said amino acid sequence I is a sequence selected from the group consisting of SEQ ID NO: 2 to 16 and SEQ ID NO: 20 to 27, , and said surfactant composition comprises, preferably consists of, SDS and CS, and wherein said molar ratio of said SDS to CS is equal or between 4 to 1 and 2 to 1, preferably wherein said molar ratio of said SDS to CS is 3 to 1, and wherein said polypeptide of the invention comprises a tag, preferably a peptide tag, wherein said tag is located at the C- or N-terminal end, preferably at the C-terminal end, of said polypeptide, preferably of said amino acid sequence I, and wherein said tag is preferably fused to said C- or N-terminal end of said polypeptide, preferably of said amino acid sequence I.

In a preferred embodiment, said amino acid sequence I is a sequence selected from the group consisting of SEQ ID NO: 2 to 5 and SEQ ID NO: 10 to 16, and said surfactant composition comprises, preferably consists of, SDS and CS, and wherein said molar ratio of said SDS to CS is equal or between 4 to 1 and 2 to 1, preferably wherein said molar ratio of said SDS to CS is 3 to 1, and wherein said polypeptide of the invention comprises a tag, preferably a peptide tag, wherein said tag is located at the C- or N-terminal end, preferably at the C-terminal end, of said polypeptide, preferably of said amino acid sequence I, and wherein said tag is preferably fused to said C- or N-terminal end of said polypeptide, preferably of said amino acid sequence I.

In a preferred embodiment, said amino acid sequence I is a sequence selected from the group consisting of SEQ ID NO: 2 to 5 and SEQ ID NO: 10 to 16 and SEQ ID NO: 20 to 22, and said surfactant composition comprises, preferably consists of, SDS and CS, and wherein said molar ratio of said SDS to CS is equal or between 4 to 1 and 2 to 1, preferably wherein said molar ratio of said SDS to CS is 3 to 1, and wherein said polypeptide of the invention comprises a tag, preferably a peptide tag, wherein said tag is located at the C- or N-terminal end, preferably at the C-terminal end, preferably at the C-terminal end, of said polypeptide, preferably of said amino acid sequence I, and wherein said tag is preferably fused to said C- or N-terminal end of said polypeptide, preferably of said amino acid sequence I.

In another preferred embodiment, said tag is selected from the group consisting of polyhistidine (His tag, i.e., an amino acid sequence consisting of two or more consecutively linked histidines), degradation tag, targeting tag, cell penetration tag, and endosomal escape tag. In a preferred embodiment, said additional tag included in the polypeptide of the invention is connected via a releasable linkage, such as a photo-cleavable linkage, or via reversible coupling.

Said targeting tag preferably binds to a cancer target, i.e. said targeting tag is a cancer targeting tag. Said cancer target includes receptors with an increased level of expression in/on certain tumor cells and tumor antigens.

In a preferred embodiment, said targeting tag is a peptide ligand, peptidomimetic, affibody, antibody binding domain or antibody. Said targeting tag is preferably folic acid.

In a preferred embodiment, said His tag, preferably said His6 tag comprises halogenated, preferably fluorinated histidines. In a preferred embodiment, said His tag comprises a fluorophore. In a preferred embodiment, said polypeptide of the invention comprises a fluorophore.

In a preferred embodiment, said polypeptide of the invention comprises a degradation tag. Preferably said degradation tag is functional in mammalian cells. In a preferred embodiment, said degradation tag is a C-terminal sequence of ornithine decarboxylase (cODC), preferably of SEQ ID NO: 17, or (poly)ubiquitin comprising or consisting of at least two consecutively linked ubiquitins. In another preferred embodiment, said degradation tag consists of cODC of SEQ ID NO: 17 (EFPPEVEEQDDGTLPMSCAQESGMDRHPAACASARINV).

In another preferred embodiment, said degradation tag comprises at least two consecutively linked ubiquitins. More preferably, said degradation tag consists of at least two consecutively linked ubiquitins.

In a preferred embodiment, said polypeptide of the invention comprises a polyhistidine (His tag). In a preferred embodiment, said polyhistidine is an amino acid sequence comprising two or more histidines. In another preferred embodiment, said polyhistidine is an amino acid sequence comprising two or more consecutively linked histidines. In another preferred embodiment, said polyhistidine is an amino acid sequence comprising 3 or more consecutively linked histidines. In another preferred embodiment, said polyhistidine is an amino acid sequence comprising 3 to 9 consecutively linked histidines. In a preferred embodiment, said His tag is attached to the C- or N-terminus, preferably the C-terminus, of the amino acid sequence I.

In a further embodiment, the polypeptide of the invention comprises an endosomal escape peptide or cell-penetrating peptide (CPP). The endosomal escape peptide is for example a dimerized disulfide-linked TAT or a thiol group. The term "cell-penetrating peptide" or CPP, as used herein, refers to a group of peptides with the ability to penetrate the plasma membrane for delivery of cargo into cells. Preferably, said CPP used in the polypeptide of the invention is a hydrophilic or cationic peptide. In another embodiment, said CPP is selected from an amphiphilic, anionic or hydrophobic peptide. A database of more than 1,600 CPPs is described by Agrawal et al. (Agrawal P, Bhalla S, Usmani SS, Singh S, CHaudhary K, Raghava GPS, et al. CPPsite 2.0: a repository of experimentally validated cell-penetrating peptides. Nucl Acids Res. 2016, 44:D1098-D103).

In a preferred embodiment, said additional tag included in the polypeptide of the invention is connected via a releasable linkage, such as a photo-cleavable linkage, or via reversible coupling.

In a preferred embodiment, said polypeptide of the invention comprises an amino acid sequence I selected from the group consisting of SEQ ID NO: 2 to 27, and wherein said lipoprotein cage is loadable and unloadable with cargo without disassembly of said lipoprotein cage.

In a preferred embodiment, said polypeptide of the invention comprises an amino acid sequence I selected from the group consisting of SEQ ID NO: 2 to 5, 10 to 16 and 18 to 22, and wherein said lipoprotein cage is loadable and unloadable with cargo without disassembly of said lipoprotein cage.

In a preferred embodiment, said polypeptide of the invention comprises an amino acid sequence I selected from the group consisting of SEQ ID NO: 2 to 5, 10 to 16, 18 or 19, and wherein said lipoprotein cage is loadable and unloadable with cargo without disassembly of said lipoprotein cage.

In a preferred embodiment, the polypeptide of the invention is modified genetically (for direct fusion of peptides) or chemically after production.

In a preferred embodiment, said lipoprotein cage of the invention is included in a composition, preferably in a pharmaceutical composition comprising a pharmaceutically acceptable carrier.

In a further aspect, the invention relates to a complex comprising the lipoprotein cage of the invention and one or more cargo molecules.

Preferably, said cargo is hydrophobic cargo, more preferably non-polar cargo. Preferably, said cargo is small cargo. Small cargo has preferably a size of 1000 Da or below.

In a preferred embodiment, a size of 1000 Da or below means that said cargo has a size of 1000 Da or lower, preferably 800 Da or lower, more preferably 600 Da or lower, again more preferably 500 Da or lower, again more preferably 400 Da or lower, again more preferably 300 Da or lower, again more preferably 200 Da or lower, again more preferably 100 Da or lower. In another embodiment, said cargo is small hydrophobic cargo having a size of 1000 Da or below, again more preferably small non-polar cargo having a size of 1000 Da or below. Preferably, said cargo has a low solubility in aqueous media. Preferably, said small cargo having a size of 1000 Da or below has a low solubility in aqueous media. More preferably said cargo of low solubility is included in Class II or Class IV of the Biopharmaceutics Classification System (BCS). Again more preferably, said low soluble cargo has a lower solubility than a highly soluble cargo for which the highest strength dose is soluble in 250 mL or less of aqueous media over the pH range of 1.0-7.5, more preferably, 1.0-6.8, at 37 ± 1°C. Preferred methods for determining solubility are the USP Dissolution Apparatus, shake-flask method or acid or base titration methods.

Preferably said cargo is an active agent, preferably a therapeutically and/or diagnostically active agent. In a preferred embodiment, said cargo is an imaging agent, such as a fluorescent agent. More preferably said cargo is selected from the group consisting of a chemotherapeutic agent, an antifungal agent, such as bifonazole or amphotericin B, an antiviral agent such as indinavir or ritonavir, and an antibiotic. Preferably said cargo is included in Class II or Class IV of the Biopharmaceutics Classification System (BCS). Preferably said chemotherapeutic agent is a small cargo molecule selected from the group consisting of doxorubicin, paclitaxel, dasatinib, imatinib, lapatinib, camptothecin, daunorubicin, buparlisib, amsacrine, bifonazole, glibenclamide, bicalutamide, celecoxib, fenofibrate, and danazol.

The treatment of cells with the complex of the invention leads to intracellular delivery of the complex and release of its cargo into the cytosol of treated cells, without disassembly of the lipoprotein cage. Thus, in a preferred embodiment, said complex of the invention comprises the lipoprotein cage of the invention and one or more cargo molecule, wherein said cargo molecule is encapsulated in the lipoprotein cage without disassembly of the lipoprotein cage. In a further preferred embodiment, said complex of the invention comprises a lipoprotein cage of the invention and one or more cargo molecule, wherein said cargo molecule is encapsulated into the lipoprotein cage extracellularly without disassembly of the lipoprotein cage, and said lipoprotein cage is capable of release said cargo intracellularly without disassembly of the lipoprotein cage. In a further preferred embodiment, said complex of the invention comprises a lipoprotein cage of the invention and one or more cargo molecule, wherein said cargo molecule is encapsulated into the lipoprotein cage extracellularly without disassembly of the lipoprotein cage, and said lipoprotein cage is capable of be taken-up by a cell and to release said encapsulated cargo molecule intracellularly, preferably into the cytosol of said cell, without disassembly of the lipoprotein cage.

Encapsulation of cargo into the lipoprotein cage of the invention is reversible in the presence of competing host molecules or environments, while the lipoprotein cage and complex of the invention are stable and not disassembled extracellularly. Thus, in a further preferred embodiment, the lipoprotein cage of the invention is capable of reversibly encapsulating and releasing cargo molecules, without disassembly of the lipoprotein cage. The release of cargo is triggered by a protein that can bind the cargo more tightly than the surfactant composition can bind the cargo, or a lipid bilayer into which the cargo preferentially partitions.

In a further aspect, the invention provides a method for manufacturing the lipoprotein cage of the invention comprising the steps of:
self-assembling of a protein cage from at least one polypeptide, preferably from 24 peptides, wherein said polypeptide comprises an amino acid sequence I consisting of: MX₁₃QAIGILELX₁SIAAGMELGDAMLKSAX₁₄VX₁₅LLVSKTISX₂GKFLLMLGG DIX₈AIX₉X₁₂AIX₁₀TGTX₁₁QAGX₃LLVDSLVLAX₁₆H-IPSVLPAIX₁₇GX₁₈NX₁₉VX₂₀ X₇X₂₁QAVGIVETX₄SVAACISAADX₂₂AVX₂₃GSX₂₄VTLVRVHMAX₅GIGGKCY MVVAGDVSDVALAVTVASSSAGAYGX₆LVYASLIPX₂₅PHX₂₆AMWX₂₇QMVX_{2 8}GX₂₉E (SEQ ID NO: 1), wherein any of X₁ to X₂₉ are independently of each other an amino acid, provided that at least 3 of X₁ to X₆ are independently of each other a positively charged amino acid, and wherein optionally up to 5 amino acids in positions other than denoted by X₁ to X₂₉ in SEQ ID NO: 1 are exchanged by any amino acid; and encapsulating the surfactant composition of the invention into the protein cage, without disassembly of the protein cage.

In a preferred embodiment, said amino acid sequence I is an amino acid sequence selected from the group consisting of SEQ ID NO: 2 to 5 and SEQ ID NO: 10 to 16, and 20 to 22. In a preferred embodiment, said amino acid sequence I is an amino acid sequence selected from the group consisting of SEQ ID NO: 2 to 5 and SEQ ID NO: 10 to 16, 20 and 21. In a preferred embodiment, said at least at least one polypeptide is defined as 24 polypeptides.

In a preferred embodiment, the method for manufacturing the lipoprotein cage of the invention comprises the further steps of producing the polypeptide of the invention, preferably by recombinant expression (e.g. in bacterial cells, preferably in *Escherichia coli* cells). Upon expression, the polypeptide of the invention self-assembles into a well-defined protein cage which can be isolated.

In a further aspect, the invention provides a method for manufacturing the complex of the invention comprising the step of mixing the lipoprotein cage of the invention with one or more cargo molecules, wherein said cargo is encapsulated into the lipoprotein cage of the invention without disassembly of the lipoprotein cage.

In a further aspect, the invention provides a method for treating cells with the complex of the invention comprising the step of contacting said cell with the complex of the invention. Preferably, said method for treating cells is an in vitro method. In a preferred embodiment, said cell is a eukaryotic cell. More preferably, said cell is an animal cell, again more preferably a mammalian cell.

**Table 1: Polypeptide sequences**

| **Identity** | **Sequence** |
|---|---|
| SEQ ID NO: 1 | MX13QAIGILELX1SIAAGMELG DAMLKSAX14VX15 LLVSKTISX2G KFLLMLGGDIX8AIX9X12AIX10TG TX11QAGX3LLVDSLVLAX16IHPS VLPAIX17GX18NX19 VX20X7X21QAVGIV ETX4SVAACISAADX22AV X23GSX24 VTLVRVHMAX5GIGGKCYMVVAGDVSDVALAVTVASSSAGAYGX6LVYASL IPX25PHX26AMWX27QMVX28GX29E |
| **OP** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHH |
| SEQ ID NO: 2 | |
| **OP-NoHis** (no His6) | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEGS |
| SEQ ID NO: 3 | |
| **OP-K93C** | MSQAIGILELRSIAAGMELGDAMLKSANVDLLVSKTISRGKFLLMLGGDIGAI QQAIETGTSQAGRLLVDSLVLANIHPSVLPAISGLNSVDCRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHH |
| SEQ ID NO: 4 | |
| **OP-cODC** | MSQAIGILELRSIAAGMELGDAMLKSANVDLLVSKTISRGKFLLMLGGDIGAI QQAIETGTSQAGRLLVDSLVLANIHPSVLPAISGLNSVDCRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHHEFPPEVEEQDDGTLP MSCAQESGMDRHPAACASARINV |
| SEQ ID NO: 5 | |
| **OTR101** | MSQAIGILELTSIAAGMELGDAMLKSANVDLLVSKTISRGKFLLMLGGDIGAI QQAIETGTSQAGELLVDSLVLANIHPSVLPAISGLNSVDKRQAVGIVETWSVA ACISAADRAVKGSNVTLVRVHMAFGIGGKCYMVVAGDVSDVALAVTVASS SAGAYGLLVYASLIPRPHEAMWRQMVEGLEHHHHHH |
| SEQ ID NO: 6 | |
| **OTR201** | MSQAIGILELTSIAAGMELGDAMLKSANVDLLVSKTISRGKFLLMLGGDIGAI QQAIETGTSQAGELLVDSLVLANIHPSVLPAISGLNSVDKRQAVGIVETWSVA ACISAADRAVKGSNVTLVRVHMARGIGGKCYMVVAGDVSDVALAVTVASS SAGAYGLLVYASLIPRPHEAMWRQMVEGLEHHHHHH |
| SEQ ID NO: 7 | |
| **OTR202** | MSQAIGILELRSIAAGMELGDAMLKSANVDLLVSKTISPGKFLLMLGGDIGAI QQAIETGTSQAGELLVDSLVLANIHPSVLPAISGLNSVDKRQAVGIVETRSVA ACISAADRAVKGSNVTLVRVHMAFGIGGKCYMVVAGDVSDVALAVTVASS SAGAYGLLVYASLIPRPHEAMWRQMVEGLEHHHHHH |
| SEQ ID NO: 8 | |
| **OTR203** | MSQAIGILELRSIAAGMELGDAMLKSANVDLLVSKTISRGKFLLMLGGDIGAI QQAIETGTSQAGELLVDSLVLANIHPSVLPAISGLNSVDKRQAVGIVETWSVA ACISAADRAVKGSNVTLVRVHMAFGIGGKCYMVVAGDVSDVALAVTVASS SAGAYGLLVYASLIPRPHEAMWRQMVEGLEHHHHHH |
| SEQ ID NO: 9 | |
| **OTR301** | MSQAIGILELTSIAAGMELGDAMLKSANVDLLVSKTISPGKFLLMLGGDIGAI QQAIETGTSQAGELLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHH |
| SEQ ID NO: 10 | |
| **OTR302** | MSQAIGILELRSIAAGMELGDAMLKSANVDLLVSKTISRGKFLLMLGGDIGAI QQAIETGTSQAGELLVDSLVLANIHPSVLPAISGLNSVDKRQAVGIVETWSVA ACISAADRAVKGSNVTLVRVHMARGIGGKCYMVVAGDVSDVALAVTVASS SAGAYGLLVYASLIPRPHEAMWRQMVEGLEHHHHHH |
| SEQ ID NO: 11 | |
| **OTR401** | MSQAIGILELRSIAAGMELGDAMLKSANVDLLVSKTISRGKFLLMLGGDIGAI QQAIETGTSQAGELLVDSLVLANIHPSVLPAISGLNSVDKRQAVGIVETRSVA ACISAADRAVKGSNVTLVRVHMARGIGGKCYMVVAGDVSDVALAVTVASS SAGAYGLLVYASLIPRPHEAMWRQMVEGLEHHHHHH |
| SEQ ID NO: 12 | |
| **OTR402** | MSQAIGILELRSIAAGMELGDAMLKSANVDLLVSKTISPGKFLLMLGGDIGAI QQAIETGTSQAGRLLVDSLVLANIHPSVLPAISGLNSVDKRQAVGIVETRSVA ACISAADRAVKGSNVTLVRVHMAFGIGGKCYMVVAGDVSDVALAVTVASS SAGAYGRLVYASLIPRPHEAMWRQMVEGLEHHHHHH |
| SEQ ID NO: 13 | |
| **OTR501** | MSQAIGILELRSIAAGMELGDAMLKSANVDLLVSKTISPGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHH |
| SEQ ID NO: 14 | |
| **OTR502** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETWSVA ACISAADRAVKGSNVTLVRVHMARGIGGKCYMVVAGDVSDVALAVTVASS SAGAYGRLVYASLIPRPHEAMWRQMVEGLEHHHHHH |
| SEQ ID NO: 15 | |
| **OTR503** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGLLVYASLIPRPHEAMWRQMVEGLEHHHHHH |
| SEQ ID NO: 16 | |
| **cODC** | EFPPEVEEQDDGTLPMSCAQESGMDRHPAACASARINV |
| SEQ ID NO: 17 | |
| **OP-His3** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHH |
| SEQ ID NO: 20 | |
| **OP-His9** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA |
| SEQ ID NO: 21 | ACISAADRAVKGSNVTLVRVHMARGIGGKCYMVVAGDVSDVALAVTVASS SAGAYGRLVYASLIPRPHEAMWRQMVEGLEHHHHHHHH |
| **OP-S38C** | MSQAIGILELRSIAAGMELGDAMLKSANVDLLVSKTICRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHH |
| SEQ ID NO: 22 | |
| **OP-93** | MGARASGSGSSQAIGILELRSIAAGMELGDAMLKSANVDLLVSKTISRGKFLL MLGGDIGAIQQAIETGTSQAGRLLVDSLVLANIHPSVLPAISGLNSVDKRQAIG ILETRSVAACISAADRAVKGSNVTLVRVHMARGIGGKCYMVVAGDVSDVAL AVTVASSSAGAYGRLVYASLIPRPHEAMWRQMVEGLEHHHHHH |
| SEQ ID NO: 23 | |
| **OP-96** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHHEFSGESSTAPGTSPSG ESSTAPGKLVQSPKGKA |
| SEQ ID NO: 24 | |
| **OP-ZHER2** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHHEFGSGSGGVDNKFN KEMRNAYWEIALLPNLNNQQKRAFIRSLYDDPSQSANLLAEAKKLNDAQAP K |
| SEQ ID NO: 25 | |
| **OP-ZEGFR** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHHEFGSGSGGVDNKFN KEMWAAWEEIRNLPNLNGWQMTAFIASLVDDPSQSANLLAEAKKLNDAQA PK |
| SEQ ID NO: 26 | |
| **OP-SP94** | MSQAIGILELRSIAAGMELGDAMLKSANVDLLVSKTISRGKFLLMLGGDIGAI QQAIETGTSQAGRLLVDSLVLANIHPSVLPAISGLNSVDKRQAVGIVETRSVA ACISAADRAVKGSNVTLVRVHMARGIGGKCYMVVAGDVSDVALAVTVASS SAGAYGRLVYASLIPRPHEAMWRQMVEGLEHHHHHHEFGSGSGGGGSGGG SSFSIIHTPILPL |
| SEQ ID NO: 27 | |

**Table 2: DNA sequences**

| | | |
|---|---|---|
| A488-DNA SEQ ID NO: 18 | A488* TTAATTAAAGACTTCAAGCGG | Used in assays where a fluorescent DNA probe is present |
| A488-DNA' SEQ ID NO: 19 | GCTTGAAGTCTTTAATTAATT | Complement for A488-DNA |

### EXAMPLES

### EXAMPLE 1 - Materials and methods

**Materials.** All chemicals were used as supplied without further purification. Isopropyl-beta-D-thiogalactopyranoside (IPTG) was purchased from Fluorochem (UK). Lysozyme was purchased from PanReac Axon Lab AG (Switzerland). For His-tagged protein isolation, Ni-NTA Agarose from Qiagen (Germany) was used. DNase I was from Roche (Switzerland) and RNase A was from Merck (Germany). GelRed was purchased from Biotium, Inc. (USA). Sodium dodecyl sulfate and cholesterol sulfate were purchased from Sigma-Aldrich (Merck, Germany).

**Instrumentation.** Protein quantification was carried out using a NanoDrop 2000c spectrophotometer from ThermoFisher Scientific Inc. (USA). All size-exclusion chromatography was carried out on an NGC^{™} Medium-Pressure Chromatography System from Bio-Rad Laboratories, Inc. (USA). Agarose gel electrophoresis (AGE) was performed on Mini-Sub^{®} cell GT from Bio-Rad Laboratories, Inc. (USA). Gel images were captured using an EOS 1100D from Canon (Japan). Transmission electron microscopy (TEM) images were obtained on a Morgagni 268 from FEI (USA). Fluorimetry was carried out on a QuantaMaster^{™} 50 fluorometer from Photon Technology International (USA). Confocal fluorescence microscopy images were obtained on an SP8-AOBS from Leica (Germany). Flow cytometry was carried out on an LSRFortessa from BD Biosciences (USA).

**Protein production.** Proteins were expressed in *E. coli* strain BL21-Gold(DE3). Cells were grown at 37 °C in LB medium containing kanamycin sulfate (86 µM) until OD₆₀₀ reached 0.6-0.8, and protein over-expression was induced with IPTG (0.1 mM). After culturing for ~18 h at 25 °C, cells were harvested by centrifugation (5,000 × *g*) at 4 °C for 15 min. Cell pellets were stored at -20 °C until purification. OP cages were isolated from *E. coli* cell pellets and purified by Ni-affinity and size-exclusion chromatography as previously reported (Edwardson et al., 2018, op. cit.).

**Preparation of OP:SDS and OP:SDS:CS complexes.** Protein cage-micelle complexes were formed directly from purified, empty OP cages and aqueous solutions of anionic surfactants. Unless otherwise specified, the molar ratio of total surfactant molecules to OP cages is 800:1 in all experiments. Buffers used were PBS (9.5 mM Na₂HPO₄, 1.4mM KH₂PO₄, 136 mM NaCl, 2.7 mM KCl, pH 7.4) and TSEC (25 mM Tris-HCl, 200mM NaCl, 5mM EDTA, pH 7.4). To form complexes, appropriate volumes of concentrated SDS solution (1-100 mM in PBS) or CS solution (8-16 mM, DMSO) were first diluted in PBS buffer to concentrations below 1 mM to avoid protein denaturation. Then, the necessary volume of OP solution (2-20 µM capsid, PBS or TSEC buffer) was added and the mixture was incubated for 1 hour at room temperature to allow complete complex formation. For small molecule encapsulation, concentrated solutions of fluorescent probe/drug in acetone or DMSO were added to the pre-formed OP:SDS:CS complexes and incubated for a further 15 minutes at room temperature. In each case, the total fraction of organic solvent was kept below 10 % v/v.

**Native agarose gel electrophoresis.** All native gel electrophoresis was carried out using 2% (w/v) agarose gels in Tris-acetate-EDTA buffer (40 mM Tris-HCl, 19 mM acetic acid, 1 mM EDTA, pH 8.3). After visualization of fluorescent molecules by UV transillumination, gels were stained with Coomassie Blue for protein visualization. In a typical experiment, ca. 100 pmol of capsid (with respect to monomer) was loaded per lane in 10 µL of buffer with an additional 2 µL of 70% (v/v) aqueous glycerol for loading.

**Size-exclusion chromatography.** Analytical SEC was carried out on a Superose 6 Increase 10/300 GL column (GE Healthcare, USA). Samples were 800 µL of 10-50 µM protein monomer and the mobile phase was 0.75xTSEC buffer. Peaks were detected by absorbance at 280 nm.

**Dynamic light scattering.** DLS measurements were carried out on Zetasizer Nano (Malvern Instruments, UK) at 25 °C using samples prepared from 0.22 µm filtered solutions of protein and surfactants. Sample concentrations were 30-100 µM of protein monomer.

**Transmission electron microscopy.** Negatively-stained transmission electron microscopy (TEM) was carried out as reported previously (Beck, T., Tetter, S., Künzle, M. & Hilvert, D. Construction of Matryoshka-Type Structures from Supercharged Protein Nanocages. Angew. Chem. Int. Ed. 54, 937-940 (2015)). For all TEM experiments, samples were between 2-4 µM of OP monomer in PBS buffer.

**Nile Red fluorescence.** For a typical fluorimetry experiment, 800 µL samples in PBS buffer were used. For the surfactant loading kinetics the ionic strength was adjusted by dissolving appropriate amounts of NaCl in PBS buffer. Stock solutions of Nile Red in acetone:water (1:1 v/v), or DMSO at concentrations of 50-500 µM were used. The excitation wavelength was set to 535 nm for all experiments.

**Effective concentration calculations.** The volume of the OP lumenal cavity (255,528 Å) was estimated as a sphere with a radius of 39.4 Å. This radius determined by averaging the distances between lumenally exposed residues from the reported crystal structure (Edwardson et al., 2018, op. cit.), using the UCSF Chimera software (Pettersen, E. F. et al. UCSF Chimera--a visualization system for exploratory research and analysis. J. Comput. Chem. 25, 1605-1612 (2004)). The effective concentration of SDS was simply estimated as the number of moles SDS per lumenal cavity volume. The expected number of SDS molecules was estimated by first determining the volume occupied by an individual SDS molecule in a micelle, from the reported average values of SDS micelle radius (17.5 Å) and aggregation number (n = 64). Division of the OP cavity volume by the average volume of a single SDS molecule packed into micellar aggregates gives an estimated value of 729 molecules per OP cage, which is within error of the volume estimations and experimental results, which approximate 800 molecules.

**Cell culture.** HeLa cells were maintained in Dulbecco's Modified Eagle Medium (high glucose) supplemented with 10% fetal bovine serum (FBS), 2 mM L-glutamine, 2 mM GlutaMAX and 1 µg/mL gentamicin. Cells were cultured in 5% CO2 at 37 °C and typically split in a 1:4 ratio every 3 days. Only passage numbers between 7-20 were used for all experiments.

**Flow cytometry.** HeLa cells were seeded at a density of 30,000 cells per well in a 24-well plate in 500 µL of culture medium and allowed to recover at 37 °C and 5% CO2 for 24 hours to reach 60-80% confluency. Both OP protein and surfactant solutions were sterilized by filtration through a 0.22 µm membrane, and stocks were prepared in sterile PBS. Nile Red solution (50 µM) in 1:1 EtOH:H₂O was used without sterilization. OP capsid-micelle complexes were prepared as described above. For each well 20 µL of sample in PBS was added to 200 µL of culture media to give the final concentration of 200 nM. Cells were incubated for 16-20 h in 5% CO2 at 37 °C before washing with PBS and trypsinization (0.05% Trypsin-EDTA (Thermo Fisher Scientific, USA), 4 minutes at 37 °C). Cells were collected in cold culture medium and washed twice with cold PBS before resuspension in flow cytometry buffer (PBS with 5% FBS). A representative cytometry analysis with all gating is shown in Figure 9.

**Fluorescent labelling of OP cages.** To provide a specific handle for fluorophore conjugation, a single serine to cysteine mutation was introduced at residue 38 of the OP protein through 'QuikChange' (Agilent) site-directed mutagenesis. This lumenally presented residue was chosen to avoid interfering with the exterior surface of the OP cage, which could disrupt the cellular uptake profile. Successful molecular cloning was confirmed by Sanger sequencing (Microsynth AG, Switzerland) of the pET29b(+)_OPS38C plasmid used for protein expression and the protein was produced as previously reported.¹⁹ Labelling of the OP cage with the Atto425-maleimide (Sigma-Aldrich) was carried out by simply mixing purified protein in TSEC buffer with dye solution (10 mM, DMSO) and incubating in the dark overnight at room temperature. To terminate the reaction, 2 equiv. (w.r.t. maleimide) of β-mercaptoethanol was added and after 30 minutes incubation, the protein was purified using a PD minitrap G-10 column (GE Healthcare, USA). The labelling efficiency was determined from UV-Vis absorbance measurement and the ε₂₈₀ and ε₄₃₉ values of Atto425 and the OP protein. For the experiments shown in Figure 4c, samples with an average labelling of 1.9 dyes per OP capsid were used.

**Confocal microscopy.** Cells were seeded at a density of 15-20,000 cells per well in a µ slide 8-well chambered coverslip with ibiTreat surface from ibidi GmbH (Germany). Cells were incubated in 200 µL of culture medium at 37 °C and 5% CO2 for 24 hours before sample addition. Sterile samples were prepared in PBS and for each well 10 µL of sample solution was added to 100 µL of culture media to give the desired final concentrations of protein and fluorophore. Cells were incubated with samples for 24 h in 5% CO₂ at 37 °C before washing with PBS and nuclear staining with 100 µL of Hoechst 33342 solution (5 µg/mL in PBS) at 37 °C for 10-15 mins. Cells were then washed twice with PBS and microscopy was carried out at 37 °C in PBS containing 10% FBS.

**Cell viability assay.** Cytotoxicity was assessed using the WST-8-based Cell Counting Kit-8 from Sigma according to the manufacturer's instructions. HeLa cells were seeded at a density of 5,000 cells per well in a 96-well plate in 100 µL of culture medium and allowed to recover at 37 °C and 5% CO2 for 24 hours. Protein, surfactant and drug samples were prepared by serial dilution in sterile PBS. A total volume of 25 µL sample was added to each well to provide the final concentrations shown in Figure 4e,f. As a negative control 10 µL of 10% Triton X-100 in PBS were used per well. The positive control was just PBS. Cells were incubated for 24 h in 5% CO2 at 37 °C before addition of 10 µL of CKK-8 reagent to each well. The plate was then incubated for 2-4 hours at 37 °C and 5% CO₂ before measuring absorbance at 450 nm. The absorbance of CKK-8 in culture media, without cells, was used for background subtraction and samples were normalized to untreated cells to provide the values shown in Figure 4e,f. Samples were measured in sextuplicate with biological replicates of different protein batches.

### EXAMPLE 2 - PREPARATION AND ANALYSIS OF AN ASSEMBLED LIPOPROTEIN CAGE

**Self-assembly of lipoprotein-mimetic cages.** For preparing an exemplarily lipoprotein cage according to the invention the artificial assembly OP was chosen as protein scaffold, which is a small porous capsid with a positively charged interior cavity (Edwardson et al., 2018, op. cit.). After expression in *Escherichia coli*, the OP protein is isolated as a complete octahedral assembly comprising 24 monomers with a ~3.5 nm pore on each of its six faces (Fig. 1b). The external diameter of the cage is ~13 nm and the diameter of the spherical internal cavity is ~8 nm. These dimensions were envisioned to be suitable for the scaffolding of small micellar aggregates within the lumen.

Starting from the positively charged OP cage, negatively charged amphiphiles were encapsulated, which in turn phase separate and create a hydrophobic core within the protein cage. The resulting protein-scaffolded lipid droplet then acts as a hydrophobic compartment that can sequester small molecules.

**Cage-templated micelle formation.** Sodium dodecyl sulfate (SDS) was chosen as the anionic surfactant (Fig. 2a), due to its aqueous solubility and the potential for favourable hydrogen bond formation between the sulfate headgroup and the many arginine residues on the interior surface of the OP capsid. As SDS is typically used as a protein denaturant, the inventors were curious if charge and shape complementarity of the OP cage could be leveraged to drive formation of a scaffolded micelle, rather than allowing hydrophobic interactions with the protein core to dominate, a pathway that leads to disruption of protein structure. As such, OP was subject to increasing molar equivalents of SDS, up to 2000 molecules per cage, and analyzed by native gel electrophoresis (Fig. 2b). The data indicate no change to OP quaternary structure up to approximately 800 equivalents of SDS per protein cage, while more than 1000 equivalents gave rise to bands of increased mobility, potentially due to disruption or denaturation of the cage structure.

In order to better understand the tolerance to SDS, OP was further interrogated in the presence of 800 equivalents of SDS per cage, through a combination of biophysical techniques (Figs. 5 and 6). As exemplified by size-exclusion chromatography and transmission electron microscopy (Figs. 2c, 2d, 2e), there was no discernible change to protein cage structure, even though the effective SDS concentration (~5.2 M, by lumenal cavity volume) was orders of magnitude above that typically used to denature proteins (mM range).

To determine whether SDS molecules were drawn within the interior cavity of the OP cage as envisioned, a fluorescently labelled oligonucleotide probe was used. It was expected that if the SDS molecules were localized in the lumen via the intended sulfate-guanidinium interactions, other potential negatively charged guests would be blocked from entry. This obstruction would be both due to negation of the high positive charge, which is the driving force for encapsulation, and occlusion of the entry pores. As oligonucleotides are internalized by OP cages rapidly with high affinity (Edwardson et al., 2018, op. cit.), they provided an ideal probe to test this hypothesis. Atto488-labelled 21 nt DNA was added to either empty OP cages or those pre-incubated with SDS, and the complexes were analyzed by native gel electrophoresis (Fig. 2f). While the empty OP cages encapsulate the probe quantitatively, the OP:SDS complexes are unable to encapsulate the DNA strands, consistent with the SDS molecules occupying the lumenal cavity.

Cryogenic electron microscopy offers a means to probe both protein cage structure and the presence of internalized guests directly. As such, the inventors analyzed both empty and surfactant (SDS)-filled OP cages (Figs. 2g-j, 7 and 8). Particles imaged with a 200 kV microscope were classified over multiple rounds in 2-D, and the best classes were ultimately used to refine a single class of a three-dimensional model. Firstly, it is of note that there is negligible change in the protein structure in the presence of SDS (Figs. 2g, 2h). Secondly, comparing OP with OP:SDS complexes shows an electron density difference corresponding to the internalized surfactants, revealing the formation of a well-defined protein-scaffolded micellar core (Fig. 2j). The structural fidelity of the OP:SDS complexes demonstrates the potential of well-defined protein structures to act as templates for the self-assembly of non-protein components, offering an alternative means to create hybrid assemblies.

**Hydrophobic core formation, cargo capacity and kinetics.** To gain a deeper understanding of the internal structure of the OP-templated micelles, the inventors used the solvatochromic dye Nile Red. This small molecule fluorophore is nearly non-emissive in aqueous media, but exhibits fluorescence in nonpolar environments (Greenspan, P., Mayer, E. P. & Fowler, S. D. Nile red: a selective fluorescent stain for intracellular lipid droplets. J. Cell Biol. 1985, vol. 100, pp. 965-973). To discern if the OP cage chaperoned SDS molecules into micelle-like aggregates with a hydrophobic core, Nile Red fluorescence was measured in the presence of each component of the system.

Below its critical micelle concentration (4-5 mM in PBS buffer)(De Paula, R., da Hora Machado, A. E. & de Miranda, J. A. 3-Benzoxazol-2-yl-7-(N,N-diethylamino)-chromen-2-one as a fluorescence probe for the investigation of micellar microenvironments. J. Photochem. Photobiol. A: Chem. 2004, vol. 165, pp. 109-114), SDS had negligible effect on the fluorescence of a 500 nM aqueous solution of Nile Red (Fig. 3a). Likewise, the OP cage did not enhance emission of the fluorescence probe. However, in the presence of both OP and SDS (800 equiv.) a large increase in Nile Red fluorescence was observed. This increase in signal was accompanied by a blue-shift in the emission maximum, indicating localization of the probe within a nonpolar environment (Greenspan et al., 1985, op. cit.).

With the suitability and utility of Nile Red established, the inventors carried out further experiments to determine the optimal number of SDS molecules per capsid and the number of Nile Red guests that could be encapsulated.

Fluorescence monitoring of two equivalents of Nile Red in the presence of OP:SDS complexes with increasing SDS content revealed a plateau at around 800 surfactant molecules per cage (Figs. 3b, 3c). This number fits with a value of ~730 calculated from the volumetric capacity of the OP cavity (~256 nm³) and the average volume occupied by an SDS molecule in a micelle (Methods section). This corroboration suggests that the molecules are arranged with some structural similarity to their typical oblate ellipsoid micellar form (Hammouda, B. Temperature Effect on the Nanostructure of SDS Micelles in Water. J Res Natl Inst Stand Technol 2013, vol. 118, pp. 151-167).

As cargo-carrying is an important characteristic of the protein-micelle complex, the inventors also used fluorescence titration to determine the number of Nile Red molecules that could be accommodated per cage. Single equivalents of Nile Red were added stepwise to a solution of OP:SDS complexes and the fluorescence spectra measured for each addition (Fig. 3d). Between 1-5 molecules of Nile Red per cage, a logarithmic increase in fluorescence emission is observed with each equivalent. From 5-20 molecules of Nile Red a steady decrease in fluorescence is observed, and above 20 a steady increase in fluorescence is observed. We attribute these three distinct stages to: i) Nile Red encapsulation, ii) self-quenching and iii) excess free dye. In the first stage, the addition of each Nile Red molecule gives a diminishing increase in emission due to self-quenching between the dyes, a process that begins to dominate in the second stage due the high effective molarity of the fluorophore (30-130 mM). In the third stage, the emission increase per equivalent is very close to that observed for Nile Red in bulk media and is associated with a redshift, indicating localization in an aqueous environment. From these data, it can be concluded that there is a well-defined loading capacity of the OP:SDS complex, which at around 20 molecules corresponds to an effective concentration (~130 mM) that is well above the solubility limit for Nile Red in organic solvents (<5 mM)

**Measurement of biological activity.** The OP assembly is capable of delivering short interfering RNA to the cytosol of mammalian cells, inducing efficient gene knockdown. As such, the inventors were interested if the micelle containing OP cages could improve the cellular uptake of poorly soluble compounds. Human cancer cells (HeLa) were treated with OP:SDS complexes carrying Nile Red or the free fluorophore itself. Analysis by flow cytometry (Fig. 4a) showed that OP:SDS complexes enhance the cellular uptake of the small molecular fluorophore. This finding was confirmed using confocal fluorescence microscopy (Fig. 4b), which also revealed that Nile Red was distributed throughout the cell. Based on the reported intracellular trafficking of OP cages (Edwardson et al., 2018, op. cit.), the majority of which localize in endosomes, this result suggests that the Nile Red cargo can escape from the cages after cellular uptake. To test this hypothesis, the same experiment was carried out using Atto425 labelled OP cages (Fig. 4c), which confirmed that while the majority of OP cage are trafficked through the endolysosomal system for degradation, the small molecule cargo is released and escapes to the cytosol.

To test the encapsulation, transport and intracellular release of a bioactive small molecule, the inventors chose the dual tyrosine kinase inhibitor, lapatinib (Moy, B. & Goss, P. E. Lapatinib: Current Status and Future Directions in Breast Cancer. The Oncologist 2006, vol. 11, pp. 1047-1057), as a model compound. Further preferred and suitable cargo molecules having a preferred size of below 600 Da are listed in the table 3 below. Lapatinib is used as a therapy for solid tumours, and has been shown to benefit from nanoparticle-mediated delivery due to its poor solubility and serum protein binding (Bonde, G. V. et al. Lapatinib nano-delivery systems: a promising future for breast cancer treatment. Expert Opin. Drug. Deliv. 2018, vol. 15, pp. 495-507). To reduce the thermodynamic cost of encapsulation and favour formation of a more stable complex the inventors modified the surfactant composition with the endogenous steroid, cholesterol sulfate (CS), to promote loading of large planar guest molecules. We found that a surfactant composition of 75% SDS and 25% CS was well-tolerated by the OP cage (Figs. 11 and 12), and enabled stable loading of lapatinib (Figs. 13a,b). Furthermore, flow cytometry of cells treated with Nile Red or Nile Red encapsulated in OP:SDS:CS complexes (Fig. 4d) revealed an even greater enhancement in intracellular delivery, from 2-fold to 5-fold, compared to OP:SDS complexes. This result is likely due to the increased stability of the encapsulation complex provided by the addition of CS, highlighting the inherent modularity of this strategy and the potential for tuning the surfactant composition to obtain desired physical properties for different cargo molecules.

**Table 3: Small therapeutic cargo molecules of a size lower than 600 Da**

| **Compound** | **Molecular Weight (Da)** | **Use** |
|---|---|---|
| dasatinib | 488 | Anti-cancer |
| imatinib | 494 | Anti-cancer |
| lapatinib | 581 | Anti-cancer |
| camptothecin | 348 | Anti-cancer |
| daunorubicin | 527 | Anti-cancer |
| buparlisib | 410 | Anti-cancer |
| amsacrine | 393 | Anti-cancer |
| bifonazole | 310 | Anti-fungal |
| glibenclamide | 494 | Type 2 diabetes |
| bicalutamide | 430 | Anti-cancer |
| celecoxib | 381 | Anti-inflammatory |
| fenofibrate | 360 | Hypercholesterolemia |
| danazol | 337 | Endometriosis |

To determine how stably the OP:SDS:CS complexes retained their lapatinib cargo, they were dialyzed against media containing 10% fetal bovine serum. After 72 hours, fluorescence spectroscopy showed the lapatinib content in the OP:SDS:CS sample, revealing signals in the range of control samples (Figure 13c). As lapatinib is known to be sequestered by serum albumin, this data confirms that OP:SDS:CS complexes exhibit a high affinity for this guest molecule.

Finally, the inventors assayed the ability of OP:SDS:CS complexes to increase the effective cytotoxicity of lapatinib in human cancer cells. Cells were treated with either free lapatinib or lapatinib packaged in OP:SDS:CS cages and cell viability was monitored after an 18 hour incubation (Fig. 4e). At a concentration of 2.5 µM, lapatinib had negligible effect on cell viability. However, packaging in OP cages had a marked effect on potency, killing 60% of the cells. Importantly, the surfactant containing OP cages themselves showed negligible toxicity, confirming that the increased efficacy was due to delivery of the lapatinib to its intracellular target. A dose-response comparison of free and encapsulated drug (Fig. 4f), reveals a 3-fold increase in potency provided by OP:SDS:CS complexes, without the addition of specific targeting or uptake enhancing ligands. Besides solubilizing the drug molecules, it is likely that encapsulation within OP-surfactant complexes prevents sequestration by serum proteins present in the media, which could also reduce cellular uptake.

### EXAMPLE 3 - Preparation and analysis of assembled lipoprotein cages with fluorophore-conjugated surface cysteine residues.

**Energy transfer between chemically conjugate fluorophores and Nile Red.** To demonstrate the compatibility of lipoprotein cage formation and small molecule cargo loading with cysteine containing proteins and their chemical conjugation, the inventors tested complex formation with two protein variants OP-K93C (SEQ ID NO: 4) and OP-S38C (SEQ ID NO: 22), which contain a single cysteine mutation per monomer, displayed on either the exterior or interior surface, respectively. These proteins form the same cage structure as OP (SEQ ID NO: 2), as shown by transmission electron microscopy (Fig. 14a,b). The purified proteins were labeled site-specifically with the cysteine reactive Atto495-maleimide fluorophore using the manufacturers recommended protocol. Lipoprotein cage formation with SDS was monitored by fluorimetry using Nile Red as a probe (Fig. 14c). Due to the overlap in the fluorescence emission of Atto495 (Emax = 527 nm) with the excitation wavelength of Nile Red (535 nm), Förster resonance energy transfer (FRET) is expected if the Nile Red is encapsulated in the lipoprotein cage complex. Indeed, this effect is observed for both OP-K93C and OP-S38C, consistent with the formation of the SDS-containing lipoprotein cages. Moreover, the marked difference in FRET efficiency is consistent with the location of the Atto495 on either the inside of the protein cage, close to the Nile Red, or on the exterior surface, further away from the micellar core. These data demonstrate that chemically-conjugated cysteine mutants of OP protein can be used to form lipoprotein cages that retain their function. Furthermore, the compatibility of OP-S38C with a different surfactant composition (75% SDS and 25% CS) and its efficient delivery into cells was also demonstrated (Figs. 4c, 10).

### EXAMPLE 4 - Preparation and analysis of assembled lipoprotein cages with C-terminal peptide tags.

**Formation of lipoprotein cages with C-terminal appendages.** The attachment of peptides, such as degradation tags, targeting tags, cell penetration tags, and endosomal escape tags, to the C-terminus of the protein allows to tune the functionality of this encapsulation system. To demonstrate the compatibility of lipoprotein cage formation with this type of protein modification, the inventors tested four protein variants with differing C-terminal peptide tags, which were created via genetic fusion. The four proteins tested were OP-96 (SEQ ID NO: 24), OP-ZHER2 (SEQ ID NO: 25), OP-ZEGFR (SEQ ID NO: 26), and OP-SP94 (SEQ ID NO: 27). Each of these four variants formed the protein cage structure, as demonstrated by size-exclusion chromatography (Fig. 15a) and transmission electron microscopy (Figs. 15b-e). Lipoprotein cage formation with SDS was investigated by fluorimetry, using Nile Red as a probe (Fig. 16f). The fluorescence enhancement and blue-shift of the emission wavelength in the presence of protein cage and SDS are consistent with lipoprotein cage formation and the encapsulation of the small molecule cargo, Nile Red.

### EXAMPLE 5 - Preparation and analysis of an assembled lipoprotein cages with N-terminal peptide tags.

**Formation of a lipoprotein cage with N-terminal appendages.** In addition to the C-terminus of the protein, the attachment of peptides, such as degradation tags, targeting tags, cell penetration tags, and endosomal escape tags, to the N-terminus of the protein allows also to tune the functionality of this encapsulation system. To demonstrate the compatibility of lipoprotein cage formation with this type of protein modification, the inventors tested a protein variant, OP-93 (SEQ ID NO: 23) with a N-terminal peptide tag, which was created via genetic fusion. This protein formed the protein cage structure as well, as demonstrated by size-exclusion chromatography (Fig. 16a). Lipoprotein cage formation with SDS was investigated by fluorimetry, using Nile Red as a probe (Fig. 16b). The fluorescence enhancement and blue-shift of the emission wavelength in the presence of protein cage and SDS are consistent with lipoprotein cage formation and the encapsulation of the small molecule cargo, Nile Red.

**EXAMPLE 6** - Preparation and analysis of an assembled lipoprotein cages with an alternative surfactant composition.

**Formation of lipoprotein cages with sodium dodecylbenzenesulfonate.** The protein cage is highly stable and acts as a template to form the lipidic/micellar core within its inner cavity, meaning a previous surfactant formulation step is not required. Therefore, the amphiphiles do not need to form a stable particle on their own before addition of the protein, allowing the use of various different amphiphiles, as long as the mixture possess sufficient negative charge. To further demonstrate the generality of the system to different surfactants, sodium dodecylbenzenesulfonate (SDBS) was used as a 1:1 mixture with SDS to form lipoprotein cages with the OP protein (SEQ ID NO: 2). Native agarose gel analysis revealed the formation of stable lipoprotein complexes, which can encapsulate three different types of small molecule cargo (Fig. 17a). Furthermore, flow cytometry analysis of HeLa cells treated with OP:SDS:SDBS:curcumin (1:400:400:8) complexes, showed an eightfold increase in cellular uptake compared to the free drug (Fig. 17b). These data highlight the modularity of the lipoprotein cage technology, as changing the lipid composition provides an easy means to tune the encapsulation properties for different cargo molecules.

### EXAMPLE 7 - Preparation and analysis of an assembled lipoprotein cages with alternative small molecule cargo.

**Formation of lipoprotein cages with alternative small molecule cargo.** To further demonstrate that the two-tier encapsulation concept can be generalized to alternative cargo, taking advantage of the hydrophobic effect, the inventors tested the encapsulation of five different small molecules: Nile Red, lapatinib, daunorubicin, curcumin and laurdan. These molecules have diverse molecular structures as well as unique biological and photophysical properties. Nevertheless, each of these molecules could be encapsulated and efficiently delivered to cells. Native agarose gel analysis of OP:SDS:SDBS (1:400:400) protein cages in the presence of curcumin, lapatinib and daunorubicin reveals encapsulation of these bioactive compounds within the lipoprotein cages (Fig. 17a). The encapsulation efficiency of small molecules could be improved by altering the surfactant composition, as seen with the use of OP:SDS:CS (1:600:200) for curcumin (Fig. 17c), Nile Red (Figs. 4,11) and lapatinib (Fig. 13). Importantly, the improved encapsulation efficiency also translated to increased cellular delivery of these compounds. For example, using OP:SDS:CS a 2.5-fold increase in cellular uptake of Nile Red (Fig. 4d) was observed compared to OP:SDS (1:800) cages. In the case of curcumin, OP:SDS:CS (1:600:200) lipoprotein cages provided a 2-fold increase in cellular delivery compared to their OP:SDS:SDBS (1:400:400) counterparts (Figs. 17a,17d). In the case of the fluorescent probe laurdan, packaging in OP:SDS (1:800) lipoprotein cages provided a marked increase in cellular uptake compared to the free molecule, as observed by confocal fluorescence microscopy (Fig. 17e).

## Claims

1. A lipoprotein cage for intracellular delivery of cargo, said lipoprotein cage comprises:
(i) a protein cage comprising at least one polypeptide comprising an amino acid sequence I consisting of: 
MX13QAIGILELX1SIAAGMELGDAMLKSAX14VX15LLVSKTISX2GKFLLML
GGDIX8AIX9X12AIX10TGTX11QAGX3LLVDSLVLAX16IHPSVLPAIX17GX18N
X19VX20X7X21QAVGIVETX4SVAACISAADX22AVX23GSX24VTLVRVHMAX5
GIGGKCYMVVAGDVSDVALAVTVASSSAGAYGX6LVYASLIPX25PHX26A
MWX27QMVX28GX29E (SEQ ID NO: 1),
wherein any of X₁ to X₂₉ are independently of each other an amino acid, provided that at least 3 of X₁ to X₆ are independently of each other a positively charged amino acid, and wherein optionally up to 5 amino acids in positions other than denoted by X₁ to X₂₉ in SEQ ID NO: 1 are exchanged by any amino acid;
wherein said protein cage possesses a positively charged interior; and
(ii) a surfactant composition comprising one or more amphiphiles, wherein the one or more amphiphiles are selected such that the net charge of the composition is negative; each of said amphiphiles comprises a hydrophilic group and a hydrophobic group, wherein the hydrophobic group comprises at least one hydrocarbon moiety selected from the group consisting of C4-C30 alkyl, C4-C30 alkenyl, C4-C30 alkynyl, C4-C30 alkoxy or C5-C30 cycloalkyl;
wherein said surfactant composition is encapsulated into the assembled protein cage.

2. The lipoprotein cage of claim 1, wherein said positively charged of X₁ to X₆ is independently of each other arginine or lysine.

3. The lipoprotein cage of claim 1 or 2, wherein at least 4 of X₁ to X₆ are a positively charged amino acid, preferably at least 5 of X₁ to X₆ are a positively charged amino acid, more preferably each of X₁ to X₆ is a positively charged amino acid.

4. The lipoprotein cage of any one of the preceding claims, wherein
X₈ is glycine or a conservative substitution thereof;
X₉ and X₁₂ are independently of each other glutamine, asparagine or a conservative substitution thereof;
X₁₀ is glutamate, aspartate or a conservative substitution thereof;
X₁₁ is serine or a conservative substitution thereof;
X₁₃, X₁₇, and X₁₉ are independently of each other serine or a conservative substitution thereof;
X₁₄, X₁₆, and X₂₄ are independently of each other asparagine, glutamine or a conservative substitution thereof;
X₁₅, X₂₀ X₂₆ and X₂₈ are independently of each other aspartate, glutamate or a conservative substitution thereof;
X₁₈ and X₂₉ are independently of each other leucine or a conservative substitution thereof; and X₂₁, X₂₂, X₂₃, X₂₅ and X₂₇ are independently of each other arginine, lysine or a conservative substitution thereof.

5. The lipoprotein cage of any one of the preceding claims, wherein said amino acid sequence I is an amino acid sequence selected from the group consisting of SEQ ID NO: 2 to 5, SEQ ID NO: 10 to 16, and SEQ ID NO: 20 to 22.

6. The lipoprotein cage of any one of the preceding claims, wherein 20 mol% of the compounds included in the surfactant composition have at least one single negative charge.

7. The lipoprotein cage of any one of the preceding claims, wherein said hydrophilic group is an anionic hydrophilic group.

8. The lipoprotein cage of claim 7, wherein said anionic hydrophilic group is selected from the group consisting of a carboxylate, sulfate, sulfonate, phosphonate, boronate, phosphate and amino acid moiety.

9. The lipoprotein cage of any one of the preceding claims, wherein said hydrocarbon moiety is selected from the group consisting of branched or linear alkyl, branched or linear alkenyl or cycloalkyl, each of C5-C30, preferably C8 to C20, more preferably C10 to 18, even more preferably C12 to C18, most preferably C12, C14, C16, C17 or C18.

10. The lipoprotein cage of claim 8 or 9, wherein said anionic hydrophilic group is a sulfate moiety and said hydrocarbon moiety is a linear alkyl, linear alkyl-ether or cycloalkyl residue, wherein the linear alkyl, linear alkyl-ether or cycloalkyl residue is C5-C30, preferably C8 to C20, more preferably C10 to 18, even more preferably C12 to C18, most preferably C12, C14, C16, C17 or C18.

11. The lipoprotein cage of any one of the preceding claims, wherein at least one of said one or more amphiphiles is an ammonium salt, alkaline salt or alkaline earth salt of dodecyl sulfate, preferably sodium dodecyl sulfate.

12. The lipoprotein cage of any one of the preceding claims, wherein at least one of said one or more amphiphiles is an anionic steroid; preferably said anionic steroid is cholesterol sulfate.

13. A complex comprising the lipoprotein cage of claims 1 to 12 and one or more cargo molecules, preferably said cargo molecules have a size of 1000 Da or below.

14. The complex of claim 13, wherein said cargo molecule is encapsulated in the lipoprotein cage without disassembly of the lipoprotein cage.

15. A method for manufacturing the lipoprotein cage of claims 1-12 comprising the steps of:
self-assembling of a protein cage from at least one polypeptide comprising the amino acid sequence I, preferably from 24 polypeptides each comprising the amino acid sequence I, and
encapsulating the surfactant composition of claim 1 into the protein cage, without disassembly of the protein cage.

## Patentansprüche

1. Lipoproteinkäfig zur intrazellulären Abgabe von Fracht, wobei der Lipoproteinkäfig umfasst:
(i) einen Proteinkäfig, der mindestens ein Polypeptid umfasst, das eine Aminosäuresequenz I umfasst, bestehend aus: 
MX13QAIGILELX1SIAAGMELGDAMLKSAX14VX15LLVSKTISX2GKFLLMLG
GDIX8AIX9X12AIX10TGTX11QAGX3LLVDSLVLAX16IHPSVLPAIX17GX18NX19VX20X7
X21QAVGIVETX4SVAACISAADX22AVX23GSX24VTLVRVHMAX5GIGGKCYMVVAG
DVSDVALAVTVASSSAGAYGX6LVYASLIPX25PHX26AMWX27QMVX28GX29E (SEQ
ID NO: 1),
wobei beliebige von X₁ bis X₂₉ unabhängig voneinander jeweils eine Aminosäure sind, vorausgesetzt, dass mindestens 3 von X₁ bis X₆ unabhängig voneinander eine positiv geladene Aminosäure sind, und wobei optional bis zu 5 Aminosäuren in anderen Positionen als mit X₁ bis X₂₉ bezeichnet, in SEQ ID NO: 1 durch eine beliebige Aminosäure ausgetauscht sind;
wobei der Proteinkäfig einen positiv geladenen Innenbereich aufweist; und
(ii) eine Tensidzusammensetzung, die ein oder mehrere Amphiphile umfasst, wobei das eine oder die mehreren Amphiphile so ausgewählt sind, dass die Nettoladung der Zusammensetzung negativ ist; jedes der Amphiphile eine hydrophile Gruppe und eine hydrophobe Gruppe umfasst, wobei die hydrophobe Gruppe mindestens eine Kohlenwasserstoffeinheit umfasst, die aus der Gruppe ausgewählt ist, bestehend aus C4-C30-Alkyl, C4-C30-Alkenyl, C4-C30-Alkinyl, C4-C30-Alkoxy oder C5-C30-Cycloalkyl;
wobei die Tensidzusammensetzung in dem zusammengesetzten Proteinkäfig eingekapselt ist.

2. Lipoproteinkäfig nach Anspruch 1, wobei das positiv geladene X₁ bis X₆ unabhängig voneinander Arginin oder Lysin ist.

3. Lipoproteinkäfig nach Anspruch 1 oder 2, wobei mindestens 4 von X₁ bis X₆ eine positiv geladene Aminosäure sind, vorzugsweise mindestens 5 von X₁ bis X₆ eine positiv geladene Aminosäure sind, mehr bevorzugt jedes von X₁ bis X₆ eine positiv geladene Aminosäure ist.

4. Lipoproteinkäfig nach einem der vorstehenden Ansprüche, wobei
X₈ Glycin oder eine konservative Substitution davon ist;
X₉ und X₁₂ unabhängig voneinander Glutamin, Asparagin oder eine konservative Substitution davon sind;
X₁₀ Glutamat, Aspartat oder eine konservative Substitution davon ist;
X₁₁ Serin oder eine konservative Substitution davon ist;
X₁₃, X₁₇ und X₁₉ unabhängig voneinander Serin oder eine konservative Substitution davon sind;
X₁₄, X₁₆ und X₂₄ unabhängig voneinander Asparagin, Glutamin oder eine konservative Substitution davon sind;
X₁₅, X₂₀, X₂₆ und X₂₈ unabhängig voneinander Aspartat, Glutamat oder eine konservative Substitution davon sind;
X₁₈ und X₂₉ unabhängig voneinander Leucin oder eine konservative Substitution davon sind; und X₂₁, X₂₂, X₂₃, X₂₅ und X₂₇ unabhängig voneinander Arginin, Lysin oder eine konservative Substitution davon sind.

5. Lipoproteinkäfig nach einem der vorstehenden Ansprüche, wobei die Aminosäuresequenz I eine Aminosäuresequenz ist, die ausgewählt ist aus der Gruppe bestehend aus SEQ ID NO: 2 bis 5, SEQ ID NO: 10 bis 16, und SEQ ID NO: 20 bis 22.

6. Lipoproteinkäfig nach einem der vorstehenden Ansprüche, wobei 20 Mol-% der in der Tensidzusammensetzung enthaltenen Verbindungen mindestens eine einzelne negative Ladung aufweisen.

7. Lipoproteinkäfig nach einem der vorstehenden Ansprüche, wobei die hydrophile Gruppe eine anionische hydrophile Gruppe ist.

8. Lipoproteinkäfig nach Anspruch 7, wobei die anionische hydrophile Gruppe aus der Gruppe ausgewählt ist, bestehend aus einer Carboxylat-, Sulfat-, Sulfonat-, Phosphonat-, Boronat-, Phosphat- und Aminosäureeinheit.

9. Lipoproteinkäfig nach einem der vorstehenden Ansprüche, wobei die Kohlenwasserstoffeinheit ausgewählt ist aus der Gruppe bestehend aus verzweigtem oder linearem Alkyl, verzweigtem oder linearem Alkenyl oder Cycloalkyl, jedem von C5-C30, vorzugsweise C8 bis C20, mehr bevorzugt C10 bis 18, noch mehr bevorzugt C12 bis C18, am meisten bevorzugt C12, C14, C16, C17 oder C18.

10. Lipoproteinkäfig nach Anspruch 8 oder 9, wobei die anionische hydrophile Gruppe eine Sulfateinheit ist und die Kohlenwasserstoffeinheit ein linearer Alkyl-, linearer Alkylether- oder Cycloalkylrest ist, wobei der lineare Alkyl-, lineare Alkylether- oder Cycloalkylrest C5-C30, vorzugsweise C8 bis C20, mehr bevorzugt C10 bis 18, noch mehr bevorzugt C12 bis C18, am meisten bevorzugt C12, C14, C16, C17 oder C18 ist.

11. Lipoproteinkäfig nach einem der vorstehenden Ansprüche, wobei mindestens eines des einen oder der mehreren Amphiphile ein Ammoniumsalz, ein Alkalisalz oder ein Erdalkalisalz von Dodecylsulfat, vorzugsweise Natriumdodecylsulfat ist.

12. Lipoproteinkäfig nach einem der vorstehenden Ansprüche, wobei mindestens eines des einen oder der mehreren Amphiphile ein anionisches Steroid ist; vorzugsweise das anionische Steroid Cholesterinsulfat ist.

13. Komplex, umfassend den Lipoproteinkäfig nach Anspruch 1 bis 12 und ein oder mehrere Frachtmoleküle, wobei die Frachtmoleküle vorzugsweise eine Größe von 1000 Da oder darunter aufweisen.

14. Komplex nach Anspruch 13, wobei das Frachtmolekül in dem Lipoproteinkäfig eingekapselt ist, ohne dass der Lipoproteinkäfig abgebaut wird.

15. Verfahren zum Herstellen des Lipoproteinkäfigs nach Anspruch 1 bis 12, umfassend die Schritte:
Selbstorganisieren eines Proteinkäfigs aus mindestens einem Polypeptid, das die Aminosäuresequenz I umfasst, vorzugsweise aus 24 Polypeptiden, die jeweils die Aminosäuresequenz I umfassen, und
Einkapseln der Tensidzusammensetzung nach Anspruch 1 in dem Proteinkäfig, ohne dass der Proteinkäfig abgebaut wird.

## Revendications

1. Cage de lipoprotéine pour une livraison intracellulaire de cargaison, ladite cage de lipoprotéine comprend :
(i) une cage de protéine comprenant au moins un polypeptide comprenant une séquence d'acides aminés I constituée de : 
MX13QAIGILELX1SIAAGMELGDAMLKSAX14VX15LLVSKTISX2GKFLLML
GGDIX8AIX9X12AIX10TGTX11QAGX3LLVDSLVLAX16IHPSVLPAIX17GX18NX19VX20
X7X21QAVGIVETX4SVAACISAADX22AVX23GSX24VTLVRVHMAX5GIGGKCYMVV
AGDVSDVALAVTVASSSAGAYGX6LVYASLIPX25PHX26AMWX27QMVX28GX29E
(SEQ ID NO: 1),
où l'un quelconque de X₁ à X₂₉ sont, indépendamment les uns des autres, un acide aminé, à condition qu'au moins 3 de X₁ à X₆ soient, indépendamment les uns des autres, un acide aminé chargé positivement, et où éventuellement jusqu'à 5 acides aminés dans des positions autres que celles indiquées par X₁ à X₂₉ dans SEQ ID NO: 1 sont échangés par n'importe quel acide aminé ;
dans laquelle ladite cage de protéine possède un intérieur chargé positivement ; et
(ii) une composition tensioactive comprenant une ou plusieurs molécules amphiphiles, dans laquelle la ou les molécules amphiphiles sont choisies de telle sorte que la charge nette de la composition soit négative ; chacune desdites molécules amphiphiles comprend un groupe hydrophile et un groupe hydrophobe, dans laquelle le groupe hydrophobe comprend au moins un fragment hydrocarboné choisi dans le groupe constitué d'alkyle en C4 à C30, alcényle en C4 à C30, alcynyle en C4 à C30, alcoxy en C4 à C30 ou cycloalkyle en C5 à C30 ;
dans laquelle ladite composition tensioactive est encapsulée dans la cage de protéine assemblée.

2. Cage de lipoprotéine selon la revendication 1, dans laquelle ladite charge positive de X₁ à X₆ est indépendamment l'une de l'autre arginine ou lysine.

3. Cage de lipoprotéine selon la revendication 1 ou 2, dans laquelle au moins 4 de X₁ à X₆ sont un acide aminé chargé positivement, de préférence au moins 5 de X₁ à X₆ sont un acide aminé chargé positivement, plus préférablement chacun de X₁ à X₆ est un acide aminé chargé positivement.

4. Cage de lipoprotéine selon l'une quelconque des revendications précédentes, dans laquelle
X₈ est glycine ou une substitution conservative de celle-ci ;
X₉ et X₁₂ sont indépendamment l'un de l'autre glutamine, asparagine ou une substitution conservative de celles-ci ;
X₁₀ est glutamate, aspartate ou une substitution conservative de ceux-ci ;
X₁₁ est sérine ou une substitution conservative de celle-ci ;
X₁₃, X₁₇, et X₁₉ sont indépendamment les uns des autres sérine ou une substitution conservative de celle-ci ;
X₁₄, X₁₆, et X₂₄ sont indépendamment les uns des autres asparagine, glutamine ou une substitution conservative de celles-ci ;
X₁₅, X₂₀, X₂₆ et X₂₈ sont indépendamment les uns des autres aspartate, glutamate ou une substitution conservatrice de ceux-ci ;
X₁₈ et X₂₉ sont indépendamment les uns des autres leucine ou une substitution conservatrice de celle-ci ; et X₂₁, X₂₂, X₂₃, X₂₅ et X₂₇ sont indépendamment les uns des autres arginine, lysine ou une substitution conservative de celles-ci.

5. Cage de lipoprotéine selon l'une quelconque des revendications précédentes, dans laquelle ladite séquence d'acides aminés I est une séquence d'acides aminés choisie dans le groupe constitué de SEQ ID NO: 2 à 5, SEQ ID NO: 10 à 16, et SEQ ID NO: 20 à 22.

6. Cage de lipoprotéine selon l'une quelconque des revendications précédentes, dans laquelle 20 % en moles des composés inclus dans la composition tensioactive ont au moins une charge négative unique.

7. Cage de lipoprotéine selon l'une quelconque des revendications précédentes, dans laquelle ledit groupe hydrophile est un groupe hydrophile anionique.

8. Cage de lipoprotéine selon la revendication 7, dans laquelle ledit groupe hydrophile anionique est choisi dans le groupe constitué d'un fragment de carboxylate, sulfate, sulfonate, phosphonate, boronate, phosphate et d'acide aminé.

9. Cage de lipoprotéine selon l'une quelconque des revendications précédentes, dans laquelle ledit fragment hydrocarboné est choisi dans le groupe constitué d'alkyle ramifié ou linéaire, alcényle ramifié ou linéaire ou cycloalkyle, chacun en C5 à C30, de préférence en C8 à C20, plus préférablement en C10 à 18, encore plus préférablement en C12 à C18, le plus préférablement en C12, C14, C16, C17 ou C18.

10. Cage de lipoprotéine selon la revendication 8 ou 9, dans laquelle ledit groupe hydrophile anionique est un fragment de sulfate et ledit fragment hydrocarboné est un résidu d'alkyle linéaire, d'éther d'alkyle linéaire ou de cycloalkyle, dans laquelle le résidu d'alkyle linéaire, d'éther d'alkyle linéaire ou de cycloalkyle est en C5 à C30, de préférence en C8 à C20, plus préférablement en C10 à 18, encore plus préférablement en C12 à C18, le plus préférablement en C12, C14, C16, C17 ou C18.

11. Cage de lipoprotéine selon l'une quelconque des revendications précédentes, dans laquelle au moins l'une desdites une ou plusieurs molécules amphiphiles est un sel d'ammonium, un sel alcalin ou un sel alcalino-terreux de dodécylsulfate, de préférence dodécylsulfate de sodium.

12. Cage de lipoprotéine selon l'une quelconque des revendications précédentes, dans laquelle au moins l'une desdites une ou plusieurs molécules amphiphiles est un stéroïde anionique ; de préférence ledit stéroïde anionique est sulfate de cholestérol.

13. Complexe comprenant la cage de lipoprotéine selon les revendications 1 à 12 et une ou plusieurs molécules cargo, de préférence lesdites molécules cargo ont une taille de 1000 Da ou moins.

14. Complexe selon la revendication 13, dans lequel ladite molécule cargo est encapsulée dans la cage de lipoprotéine sans démontage de la cage de lipoprotéine.

15. Procédé de fabrication de la cage de lipoprotéine selon les revendications 1 à 12, comprenant les étapes consistant à :
auto-assembler une cage de protéine à partir d'au moins un polypeptide comprenant la séquence d'acides aminés I, de préférence à partir de 24 polypeptides comprenant chacun la séquence d'acides aminés I, et
encapsuler la composition tensioactive selon la revendication 1 dans la cage de protéine, sans démontage de la cage de protéine.
